# EUROPEAN PATENT APPLICATION

(11) **EP 3 945 133 A1**
(43) Date of publication of application: **02.02.2022**
(21) Application number: 20188364.2
(22) Date of filing: 29.07.2020
(51) Int. Cl.: C12N 5/077, C12N 5/071

(54) **MASS PRODUCTION OF HUMAN PLURIPOTENT STEM CELL DERIVED CARDIAC STROMAL CELL**

(71) Applicant: Georg-August-Universität Göttingen, 37075 Göttingen (DE)
(72) Inventor: Zimmermann, Wolfram-Hubertus, 37073 Göttingen (DE); Tiburcy, Malte, 37083 Göttingen (DE)
(74) Representative: Wichmann, Hendrik

(57) **Abstract**

The application describes a method for producing a population of cardiac stromal cells from pluripotent stem cells. Specifically, the method relates to (i) inducing epithelial-mesenchymal transition of pluripotent stem cell derived epicardial cells and (ii) amplifying the number of cardiac stromal cells in serum-free conditions. These cardiac stromal cells can be mass produced according to the described method and said cells maintain the expression of CD90, CD73 and CD44 in at least 80% of the cardiac stromal cells. Furthermore, the application relates to a population of cardiac stromal cells, which are pluripotent stem cells derived and wherein at least 80% of the cardiac stromal cells express CD90, CD73 and CD44. Said cardiac stromal form the basis for several *in vitro* and *in vivo* applications such as the production of engineered organ tissue and the support of, for example, heart repair. Also, a serum-free culture medium for the amplification of cardiac stromal cells is provided herein.

## Description

### Background of the invention

The heart is composed of cardiomyocytes and non-myocytes. The non-myocyte fraction of the heart comprises primarily of stromal cells, endothelial cells, and leukocytes (Naito et al. 2006, Pinto et al. 2016, Zhou and Pu 2016). Cardiac stromal cells, *also known as* resident mesenchymal cells (Pinto et al. 2016), comprise for the most part fibroblasts or fibroblast-like cells. The developmental origin of the cardiac fibroblasts remains a matter of debate, but appears to be primarily from the epicardium (Ivey and Tallquist 2016). The epicardium is the outermost layer of the heart. It develops during the looping stage of the embryonic heart from the proepicardial organ (Witty et al. (2014)). Specifically, the proepicardial envelopes the heart to form an outer epithelial layer, the epicardium. The epicardium then undergoes an epithelial-to-mesenchymal transition (EMT) in response to various signals, including TGFβ1, Wnt, retinoic acid (RA), FGF and PDGF, to give rise to cardiac fibroblasts and coronary vascular smooth muscle cells that invade the myocardial layer and contribute to the structural and vascular populations of the developing heart. These cells are also known as cardiac stromal cells (or epicardial-derived cells) and constitute a substantial proportion of the non-cardiomyocyte population within the myocardial layer (Witty et al (2014)). The paracrine cross-talk between the epicardial-derived fibroblasts and the cardiomyocytes was found to be essential for proper heart development (Ieda et al. 2009). In line with these *in vivo* observations in mouse models, the inventors demonstrated previously the essential role of primary human fibroblasts in the formation of engineered human myocardium (EHM) from primarily human pluripotent stem cell (PSC)-derived cardiomyocytes (Tiburcy et al. 2017).

During differentiation of PSC into cardiac muscle cells, cardiac stromal cells have been a non-controlled by-product. The inventors previously demonstrated that controlling the non-myocyte stromal cell proportion either by directed differentiation (WO2015/040142) or by adding a homogenous stromal cell population (such as fibroblasts) in the engineering of heart muscle (Naito et al. 2006, Tiburcy et al. 2017), increases the function of the engineered heart muscle. Controlling the production of cardiomyocytes and stromal cells separately to provide largely pure (>90%) cellular starting material is of paramount importance for the engineering of heart muscle for from PSCs (e.g. induced PSCs, iPSCs) for therapeutic products (such as by tissue engineered product-advanced therapy medicinal products [TEP-ATMP]) and robust research (such as required in drug development) applications. Beyond the use in heart muscle engineering, there is an urgent need for high-quality (i.e., as to purity and function) and high-quantity stromal cell populations to model fibrosis and produce connective tissue (Dworatzek et al. 2019, Schlick et al. 2019, Zimmermann et al. 2006) for applications in the development of anti-fibrotic therapeutics (Santos et al. 2019).

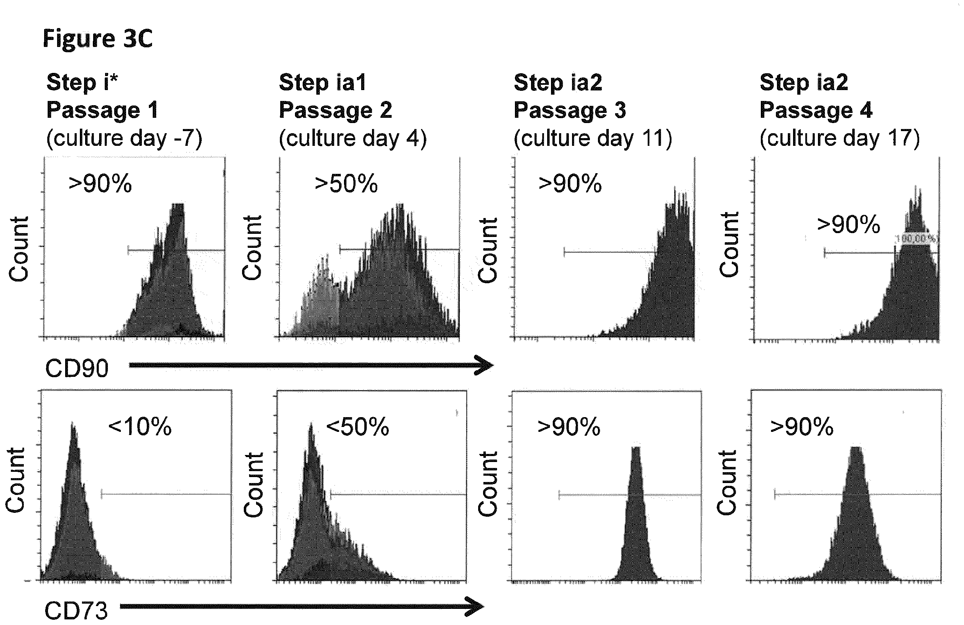

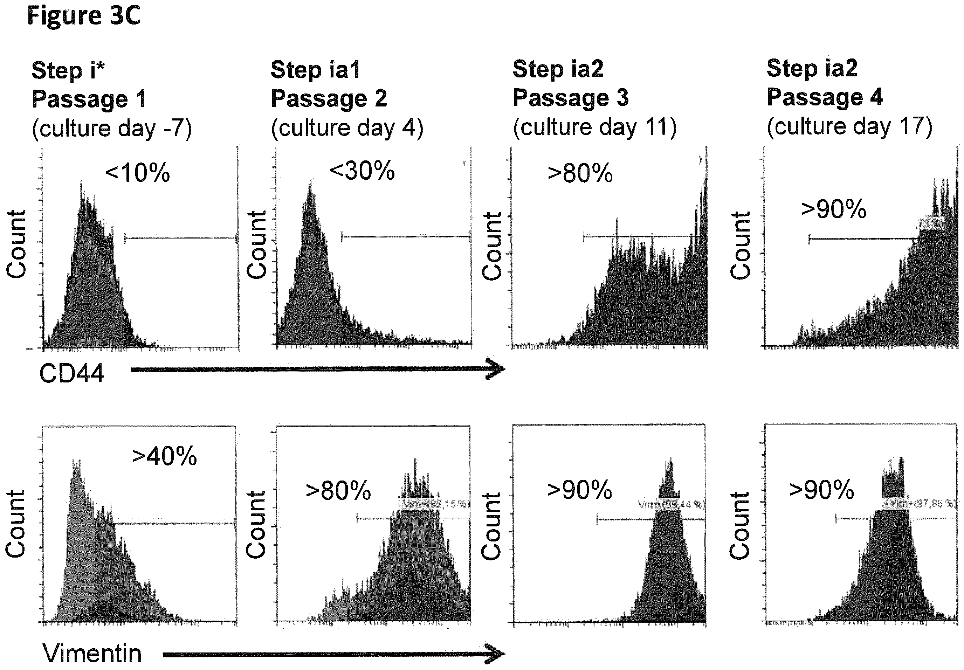

Hence, there is a need in the art for methods for producing cardiac stromal cells of a large quantity with a high degree of homogeneity in order to be able to generate engineered tissue such as human myocardium. The present invention is concerned with a method to derive a suitable cardiac stromal cell population from human iPSC-derived epicardial cells in a sufficient number in a scalable GMP-compatible process. As stromal cells from different organs can be quite distinct, cardiac stromal cells that have similar properties as primary cardiac fibroblast were sought for. During heart development *in vivo,* the majority of cardiac fibroblasts are derived from the epicardium and thus the inventors generated cardiac stromal cells by inducing epithelial-mesenchymal transition of epicardial cells. Previous studies have shown that epicardial cells can be differentiated from human pluripotent stem cells using staged differentiation protocols (Witty et al. 2014, Iyer et al. 2015, Bao et al. 2016, and Bao et al. 2017, Zhang et al. 2019). However, none of these disclosed protocols is suitable for a scalable production of a homogeneous cardiac stromal cell population, which can be used for large scale, including clinical scale, applications such as in tissue engineering. Furthermore, US 2018/0094245 A1 discloses methods for generating high-yield cardiac fibroblasts. However, there are clear differences between the cardiac fibroblasts as disclosed therein and the cardiac stromal cells as described herein. Most importantly, the cardiac fibroblasts of US 2018/0094245 A1 show a marked reduction in CD90 (also known as Thymocyte Differentiation Antigen 1 (Thy1)) already one day after (induction of differentiation; see Figure 2C) with a further loss of its expression with passaging (see Figure 3C). However, CD90 is a commonly used cell surface marker to sort resident fibroblasts from the heart and its loss is reported to be associated with a pathological phenotype (Li et al. 2020).
To the inventor's best knowledge, the prior art is lacking a defined method for the production of cardiac stromal cells stably expressing CD90 and Vimentin (VIM) from pluripotent stem cells, avoiding serum and other undefined substances such as matrigel.

### Summary of the invention

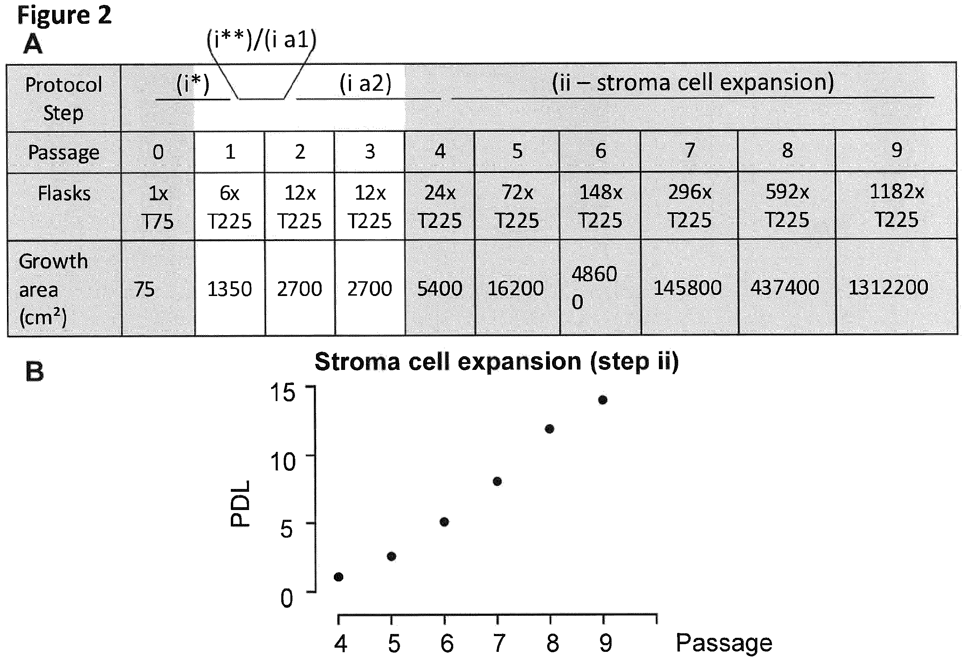

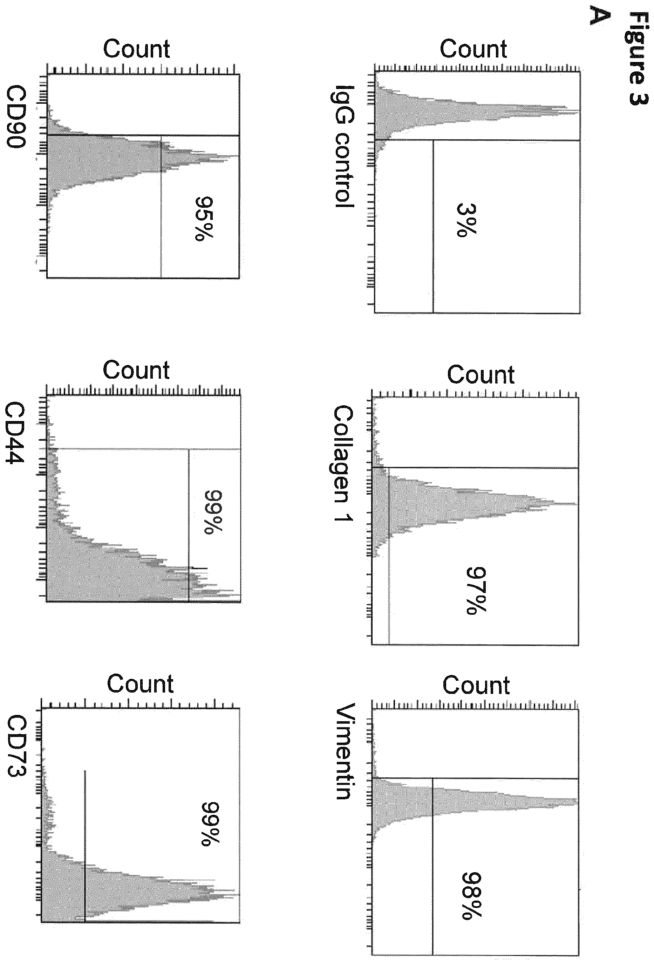

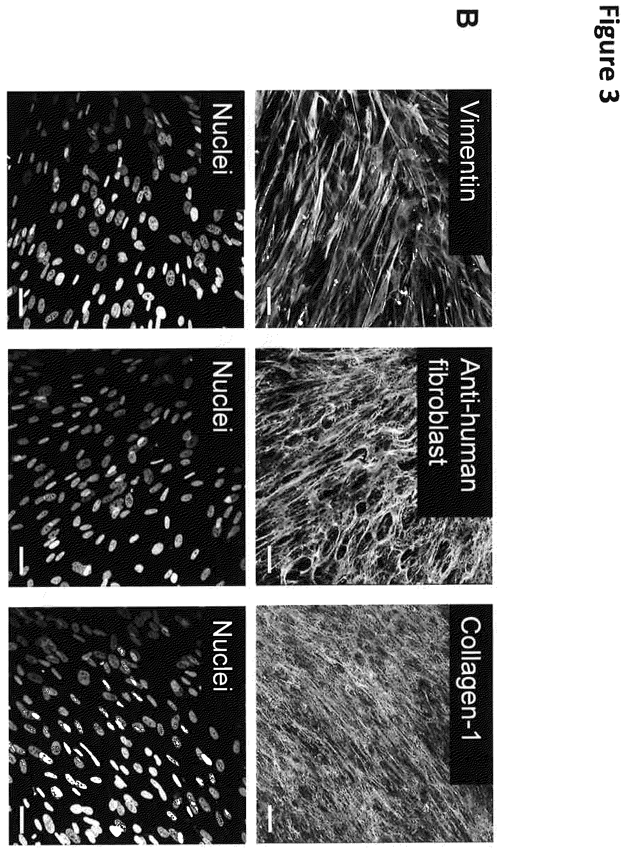

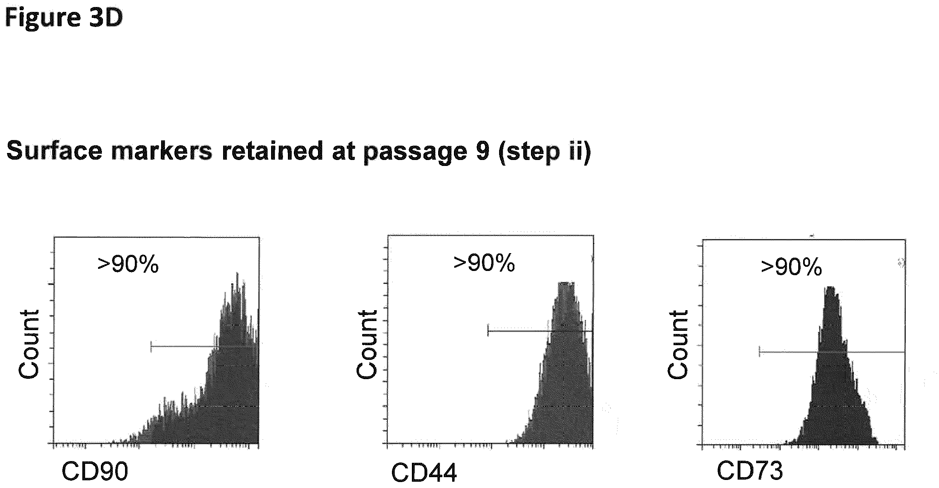

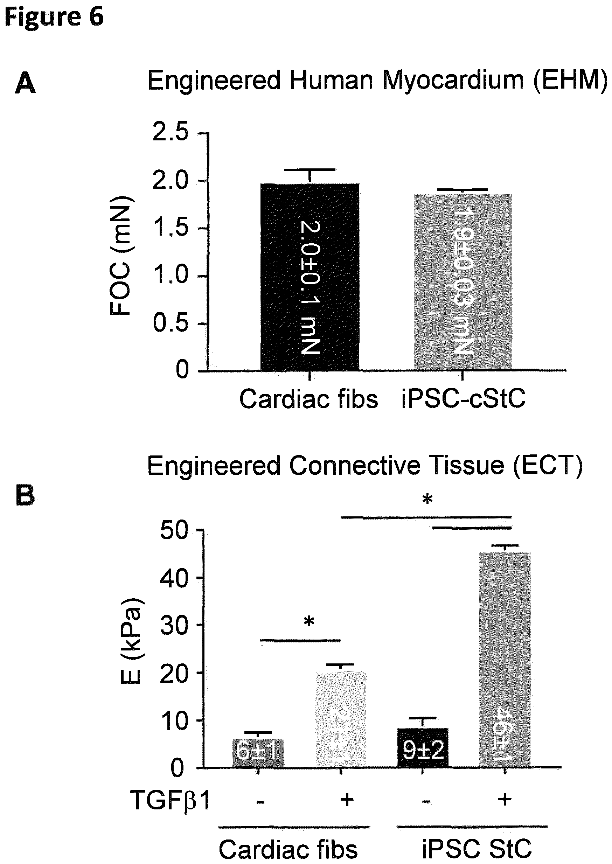

To overcome the shortcomings of the protocols disclosed in the prior art, the inventors developed a defined serum-free protocol, which allows for the generation and amplification of a population of cardiac stromal cells at (1) high quantity and (2) high quality, i.e. homogeneity (at least 80% CD90, CD73 and CD44), under serum-free conditions from (pluripotent stem cell derived) epicardial cells. The pluripotent stem cells are not produced by a process in which the genetic identity of the human being is altered in the germ line or in which a human embryo is used for industrial or commercial purposes.
The population of cardiac stromal cells as disclosed herein can be expanded over several passages, so that cardiac stromal cells can be mass produced. The amplification in a serum-free environment is essential and of prime importance for the production of large-scale engineered organ tissue, such as engineered human myocardium (EHM; Tiburcy et al. 2017) and engineered connective tissue (ECT; Dworatzek et al. 2019, Santos et al. 2019, Schlick et al. 2019), for clinical and research (e.g., drug development) use. Furthermore, it is of utmost importance to have a method, wherein a comparable and reproducible quality and quantity of cells can be produced, for large-scale production of engineered organ tissue for clinical use according to Good Manufacturing Practise (GMP) and for applications of stromal cells or engineered organ tissue in industry-scale drug development. The ability to amplify/expand the cardiac stromal cell number over at least six passages is also supported by experimental data **(Example 2 and 4;** **Figure 2** **and** **3D****).** The high quality (high homogeneity of cardiac stromal cells) is also supported by flow cytometry data, gene expression data, immunofluorescence data, and transcriptome data (see **Example 3-5;** **Figure 3****,** **4** and **5****).** The mass produced cardiac stromal cells, as produced herein, show a highly similar RNA expression profile as primary cardiac fibroblasts, but differ essentially from skin and gingiva fibroblasts. In addition, the generated cardiac stromal cells support force generation in engineered heart muscle **(Example 6;** **Figure 6A****)** and can be used to create engineered connective tissue with heart tissue-like viscoelastic properties **(Example 6;** **Figure 6B****).** It is demonstrated that the cardiac stromal cells as produced herein are not only high quality stromal cells in general, but are cardiac specific stromal cells with fibroblast properties.

In a first aspect, a method for producing a population of cardiac stromal cells from pluripotent stem cells is described, wherein the method comprising the steps of:
i. Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises a1) culturing said epicardial cells under suitable conditions in the presence of a first extracellular matrix protein in a serum-free basal medium;
   followed by a2) culturing the cells of step (i) a1) under suitable conditions in the presence of a second extracellular matrix protein in a serum-free basal medium; wherein at least about 80 % of the cells of the obtained population of cardiac stromal cells express CD90, CD73, and CD44; and
ii. Amplifying the number of said cardiac stromal cells by culturing said population of cardiac stromal cells of step (i) in the presence of at least one third extracellular matrix protein in a serum-free basal medium, wherein at least 80 % of the cells of the cardiac stromal cell population maintain the expression of CD90, CD73, and CD44, as defined in the claims.

Furthermore, an isolated population of cardiac stromal cells is described, wherein the cardiac stromal cells have been obtained by differentiation of pluripotent stem cells and wherein at least about 80 % of the cells of the population of cardiac stromal cells express CD90, CD73, and CD44, as defined in the claims.

In addition, an engineered organ tissue comprising a population of cardiac stromal cells as defined herein is defined, as defined in the claims.

Moreover, a use of the population of cardiac stromal cells as defined herein or as obtained by the method as disclosed herein, or use of the engineered organ tissue as defined herein in an *in vitro* model for drug screening is described, preferably *in vitro* model for drug efficacy screening or in an *in vitro* model for drug toxicity screening.

In another aspect, a use of the population of cardiac stromal cells as defined herein or as obtained by the method as defined herein in an *in vitro* production of an engineered organ tissue is described, preferably of a human engineered organ tissue, more preferably wherein the engineered human organ tissue is engineered human myocardium or engineered human connective tissue.

Furthermore, a population of cardiac stromal cells (cStC) as defined herein or as obtained by the method as defined herein for use in organ repair is disclosed, preferably heart repair or soft tissue repair.

Finally, a serum-free cell culture medium is defined, wherein the medium is suitable for amplification of cardiac stromal cells comprising (a) a serum-free basal medium, (b) 10-200 ng/ml FGF2, (c) 5-100 ng/ml VEGF, (d) 0.2-20 mM glutamine, and (e) a eukaryotic cell culture medium supplement comprising 6.6-165 µg/ml ascorbic acid, 2-50 µg/ml insulin, 1.1-27.5 µg/ml transferrin, 1660-41500 µg/ml albumin and 11-145 nM selenium.

### Detailed description of the invention

In a first aspect, a method for producing a population of cardiac stromal cells from pluripotent stem cells is described, wherein the method comprises the steps of:
(i) Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises a1) culturing said epicardial cells under suitable conditions in the presence of a first extracellular matrix protein in a serum-free basal medium;
   followed by a2) culturing the cells of step (i) a1) under suitable conditions in the presence of a second extracellular matrix protein in a serum-free basal medium; wherein at least about 80 % of the cells of the obtained population of cardiac stromal cells express CD90, CD73, and CD44; and
(ii) Amplifying the number of said cardiac stromal cells by culturing said population of cardiac stromal cells of step (i) in the presence of at least one third extracellular matrix protein in a serum-free basal medium, wherein at least 80 % of the cells of the cardiac stromal cell population maintain the expression of CD90, CD73, and CD44.

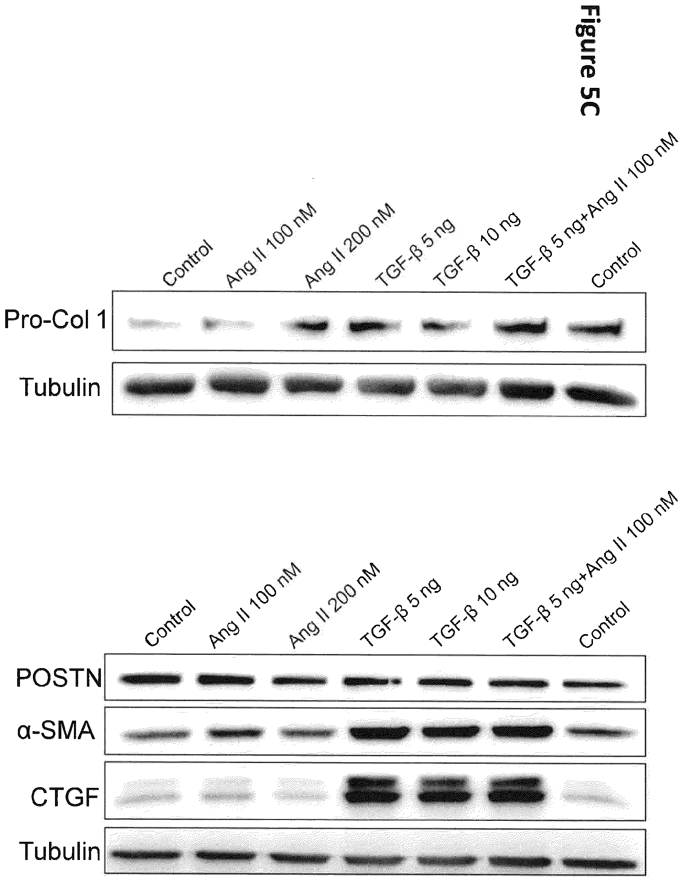

In general, a "population" of cells refers to a group of cells overall showing the same characteristics, as defined below. These characteristics can be detected by assays known in the art such as flow cytometry or fluorescent microscopy. "Cardiac stromal cells", as used herein, are collagen producing mesodermal cells. Specifically, cardiac stromal cells can be detected by flow cytometry. The methodology of "flow cytometry" is known to the person skilled in the art. In flow cytometry, physical and/or chemical properties of a cell population are detected. In the present case, the presence of differentiation specific markers or, in particular, cardiac stromal cell specific markers can be detected by flow cytometry through fluorescently labelling said expressed markers. Exemplary markers for cardiac stromal cells are CD90, CD44 and CD73. Thus, the expression of these markers is indicative for the presence of cardiac stromal cells, as produced herein. The cardiac stromal cells, as produced herein, have fibroblast-properties. This means that the cardiac stromal cells as produced herein may be capable of collagen secretion as well as the conversion to myofibroblasts in the context of wound healing processes/scar formation or induced by e.g. TGFb1 and Angiotenin II (see for example Figure 5C). A population of cardiac stromal cells, as defined herein, refers to a group of cardiac stromal cells, wherein at least about 80% of the cells express CD90, CD73 and CD44.

The skilled person is readily able to assess the expression of markers such as CD90, CD73 and/or CD44 by flow cytometry. For example, the population of cells expressing a marker, such as CD90, CD73 and/or CD44, can be distinguished from the cells not expressing the exemplary marker by comparing the cells to at least a negative control. For example, an isotype control can serve as a negative control. Isotype controls are primary antibodies that lack specificity to the target, but match the class and type of the primary antibody used in the application. With the help of an isotype control, the cells expressing the marker and cells not expressing the marker can be distinguished. One example of a negative control may be a polyclonal rabbit IgG antibody. A preferred method for flow cytometry is illustrated in Example 3.

CD90 (Cluster of Differentiation 90), also known as Thy-1, is a glycophosphatidylinositol (GPI) anchored cell surface protein. Furthermore, CD90 is a commonly used cell surface marker to sort resident fibroblasts in the heart (Li et al. 2020). Li et al. also showed that cells lacking CD90 are part of the pathogenic cardiac fibroblast fraction in cardiac fibrosis and suggest important roles of the expression of CD90 in pathophysiology of heart failure. In an embodiment, at least 80% of the population of cardiac stromal cells express CD90, preferably at least 81%, more preferably at least 82%, even more preferably at least 83%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD90, as determined by flow cytometry. Exemplary data for the expression of CD90 in the obtained cardiac stromal cells is provided in **Figure 3A****,** **3C** **and** **3D****.**

CD73, also known as 5'-nucleotidase (5'-NT) or ecto-5'-nucleotidase (cluster of differentiation 73), is an enzyme that in humans is encoded by the NT5E gene. CD73 was found to be highly enriched in the fibroblast population (Tiburcy et al. 2017). Expression of CD73 can be an indicator for the formation of cardiac stromal cells. In an embodiment, at least 80% of the population of cardiac stromal cells obtained by step (ii) express CD73, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD73, as determined by flow cytometry. Exemplary data for the expression of CD73 in the obtained cardiac stromal cells is provided in **Figure 3A****,** **3C** and **3D****.**

CD44 is a cell-surface, transmembrane glycoprotein that serves as a cell-surface receptor for a number of extracellular matrix proteins, especially the abundant matrix glycosaminoglycane hyaluronan. Hyaluronan via CD44 activates fibroblasts and regulates fibroblast function. CD44 is a mesenchymal cell marker which was found to be enriched in the fibroblast population (Tiburcy et al. 2017). Expression of CD44 can be an indicator for the formation of cardiac stromal cells. In an embodiment, at least 80% of the population of cardiac stromal cells obtained by step (ii) express CD44, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD44, as determined by flow cytometry. Exemplary data for the expression of CD44 in the obtained cardiac stromal cells is provided in **Figure 3A****,** **3C** and **3D****.**

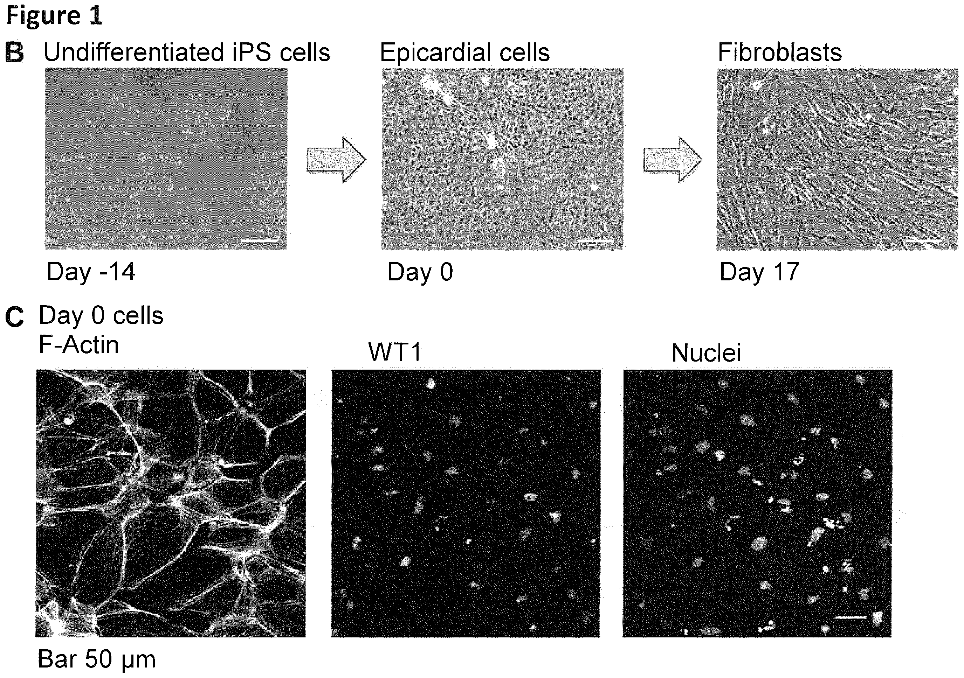

In order to achieve cell differentiation of pluripotent stem cells into cardiac stromal cells, specific chemical factors, additives and/or inhibitors are added to the culturing medium. Specifically, the differentiation of pluripotent stem cells into epicardial cells is well known in the art. Preferably, epicardial cells can be obtained as described in Schlick (2018) doctoral Thesis, November 2018, University of Gottingen, Witty et al Nat. Biotechnology 32, 1026-1035 (2014) or any other suitable method to obtain epicardial cells. Other protocols described in the art can be found in Iyer et al. 2015, Bao et al. 2016, and Bao et al. 2017, Zhang et al. 2019. More preferably, the skilled person can obtain epicardial cells from pluripotent stem cells as described in **Example 1** herein. At the molecular level, the developing epicardium can be distinguished from the myocardium and endocardium by expression of the transcription factors such as WT-1. The skilled person is aware of methods to detect WT-1 in epicardial cells. A preferred method to detect WT-1 in epicardial cells is fluorescent microscopy. The expression of WT-1 elicits a fluorescent signal, which can be detected under the microscope and the skilled person is aware of fluorescent microscopy. For example, **Figure 1C** provides experimental data and shows the expression of WT-1 of epicardial cells, which can either be obtained by any suitable method in the art or preferably by the method as disclosed herein. Other possible distinguishing factors are the expression of TBX18 and the aldehyde dehydrogenase enzyme retinaldehyde dehydrogenase 2 (ALDH1A2).

The expression of WT-1 can also be assessed by RT-PCR. The skilled person knows how to reliably detect WT-1 using qRT-PCR, as illustrated, e.g. by Witty et al. (2014) detecting WT-1 using qRT-PCR after induction of the epicardial differentiation. In an embodiment, the cells after step (ii) express Wilms tumor antigen 1 (WT-1) RNA at least 2-fold more than, for example, TBP (TATA-binding protein). TBP is considered a housekeeping gene, which is not expected to show a different expression during differentiation. Housekeeping genes are typically constitutive genes that are required for the maintenance of basic cellular function, and are expressed in all cells of an organism under normal and pathophysiological conditions. Thus, other housekeeping genes can also be used for the comparative expression of WT-1. In a preferred embodiment, the epicardial cells express Wilms tumor antigen 1 (WT-1) RNA at least 3-fold more than a housekeeping gene such as TBP, more preferably 4-fold, even more preferably at least 5-fold, most preferably at least 6-fold; and at most 15-fold, as determined by qRT-PCR.

"Pluripotent stem cells" (PSC) are able to differentiate into any cell type of the body. Therefore, pluripotent stem cells offer the remarkable possibility to obtain, for example, cardiac stromal cells. Currently, the most commonly used pluripotent cells are induced pluripotent stem cells (iPSC) or embryonic stem cells (ESC). Human ESC lines were first produced by Thomson et al. (1998). Today, human ESC research enables the development of a new technology for reprogramming body cells into an ES-like cell. This technology was pioneered in 2006 by Yamanaka et al. and is also applicable to human cells (Takahashi & Yamanaka (2006) and Takahashi et al. (2007)). The resulting induced pluripotent cells (iPSC) show a very similar behaviour to ESC and are also able to differentiate into any cell of the body. In addition, parthenogenetic stem cells can be used in a further embodiment. Parthenogenetic stem cells can be obtained in mammals, preferably in mice as well as in humans, from blastocysts that develop after in vitro activation of unfertilized egg cells. These cells exhibit the key characteristics of pluripotent stem cells, enabling them to differentiate into any cell type *in vitro* (Didie et al (2013)). Accordingly, pluripotent stem cells can be selected from induced pluripotent stem cells, embryonic stem cells and parthenogenetic stem cells. In a preferred embodiment, the pluripotent stem cells are not produced by a process in which the genetic identity of the human being is modified in the germ line or in which a human embryo is used for industrial or commercial purposes. In an embodiment, the pluripotent stem cells are pluripotent stem cells of primate origin, preferably human pluripotent stem cells. In a preferred embodiment, the pluripotent stem cells are selected from induced pluripotent stem cells and parthenogenetic stem cells, preferably wherein the pluripotent stem cells are induced pluripotent stem cells (iPSC). Induced pluripotent stem cells (iPSC) are selected in a particularly preferred embodiment.

During development, epicardial cells undergo epithelial-to-mesenchymal transition (EMT). In the method as disclosed herein, the EMT of epicardial cells is induced in order to differentiate the epicardial cells into cardiac stromal cells. Said differentiation step takes place under suitable conditions in order to obtain cardiac stromal cells so that at least about 80% of the obtained cells express CD90, CD73 and CD44, which can be determined by flow cytometry. In light of the present disclosure, the person of average skill in the art is readily able to determine suitable conditions for inducing EMT of epicardial cells. Exemplary experimental data for the cardiac stromal cells expressing CD90, CD73 and CD44 after the completion of step (i) of the method as disclosed herein can be found in **Figure 3A****.**

Specifically, the EMT is induced under suitable condition in the presence of a first and a second extracellular matrix protein. The term "extracellular matrix protein" refers to any extracellular matrix (ECM) protein known by the person of average skill (Hynes and Naba (2012)). In particular, extracellular matrix proteins often comprise the amino acid sequence RGD. Preferably, the first and/or second extracellular matrix protein comprises laminin, vitronectin, collagen, in particular gelatine, fibronectin, elastin. In an especially preferred embodiment, collagen is present in the form of gelatine. Gelatine is derived from collagen after it has undergone hydrolysis and denaturation. More preferred is that the first extracellular matrix protein comprises laminin and even more preferred is that the first extracellular matrix protein is laminin.

Laminin is generally a combination of an alpha-chain, a beta-chain, and a gamma-chain. In a preferred embodiment, the alpha-chain, the beta-chain, and the gamma-chain can be independently selected. For example, the alpha-chain can be selected from alpha-1, alpha-2, alpha-3, alpha-4, alpha-5; the beta-chain can be selected from beta-1, beta-2, and beta-3; and the gamma chain can be selected from gamma-1, gamma-2, and gamma-3. The combination of the alpha-5 chain, the beta-2 chain and the gamma-1 chain of laminin is especially preferred. Said laminin is also known as laminin-521. The skilled person is aware that laminin can be generated recombinantly and that it is commercially available. For example, Biolamina is a well-known supplier in the art. In another embodiment, the laminin is dissolved in Dulbecco's phosphate-buffered saline (DPBS) when it is used. As demonstrated in the Example section, even more preferred is the commercially available laminin-521.

In another embodiment, the second extracellular matrix protein (ECM) is the same or different from the first extracellular matrix protein. Preferably, the second extracellular matrix protein is different from the first extracellular matrix protein. It is also preferred that the second extracellular matrix protein comprises vitronectin, laminin, collagen, in particular gelatine, fibronectin, or elastin. It is especially preferred that the second ECM comprises vitronectin.

It is further contemplated that step (i) takes place in the presence of a "serum-free basal medium". A basal medium for culturing cells generally supplies the essential nutrients (e.g. amino acids, carbohydrates, vitamins, minerals), and gases (e.g. CO2, O2), and regulates the physio-chemical environment (e.g. pH buffer). Any suitable basal medium can be used for the method described herein. Basal media are commercially available or can be produced according to publicly available recipes, e.g. from ATCC catalogues. If deemed appropriate, the basal medium may be supplemented with e.g. amino acids. "Serum-free basal medium" does not comprise serum such as fetal bovine serum as a supplement. This has the advantage that serum-free basal medium is defined in that the ingredients do not vary, for example, depending on the serum batch. Further preferred embodiments of the serum-free basal media are described below.

After the completion of step (i), cardiac stromal cells are obtained so that at least 80% of the cell population express CD90, CD73 and CD44. In the following amplification step (ii), the number of cardiac stromal cells is amplified under defined conditions. In doing so, the cardiac stromal cells maintain the expression of CD90, CD73 and CD44, as can be determined by flow cytometry. In one embodiment, step (ii) stably amplifies the population cardiac stromal cells as determined by the maintained expression of at least 80% of CD90, CD73 and CD44 using flow cytometry. Amplifying the number of cardiac stromal cells takes place in the presence of at least one third extracellular matrix protein. Similarly to the second extracellular matrix protein, the "at least one third extracellular matrix protein" is preferably selected from the group consisting of vitronectin, laminin, collagen, in particular gelatine, fibronectin, elastin, Matrigel, a peptide containing the amino acid sequence RGD, a protein containing the amino acid sequence RGD and combinations thereof. More preferably the at least one third extracellular matrix protein is selected from the group consisting of vitronectin, laminin, collagen, in particular gelatine, fibronectin, and elastin. In an especially preferred embodiment, the at least one third extracellular matrix protein is vitronectin.

Step (ii) of the method also takes place in the presence of a serum-free basal media, as defined herein. It is further contemplated that the serum-free basal medium can be independently selected in step (i) a1), (i) a2), and (ii) and preferred embodiments of the serum-free basal medium for each of these steps are described below.

In an embodiment, the cultivation of the cells in step (i) a1), step (i) a2), and/or the cultivation of said population of said cardiac stromal cells in step (ii) is carried out in suspension culture. In suspension culture, single cells or small aggregates of cells differentiate or multiply, while suspended in agitated liquid medium. The skilled person is aware of cellular suspension culture in general for various cell types. For example, Chen et al. (2012) described a scalable suspension culture system for culturing human embryonic stem cells in spinner flasks. Said suspension culture system provides an approach for scale-up expansion of hESCs under defined and serum-free conditions for clinical and research applications. Three years later, Chen et al. (2015) described the use of a scalable suspension culture system to generate cardiomyocytes from human pluripotent stem cells. Thus, the skilled person would expect that the differentiation of epicardial cells into cardiac stromal cells can also take place in suspension culture. In a preferred embodiment, the first extracellular matrix protein in step (i) a1) is provided to the suspension culture in soluble form. In another preferred embodiment, the second extracellular matrix protein is provided to the suspension culture in step (i) a2) in soluble form. Even more preferred is that the first and the second extracellular matrix proteins are provided in steps (i) a1) and (i) a2) in soluble form. In another preferred embodiment, the at least one third extracellular matrix protein in step (ii) is provided to the suspension culture in soluble form.

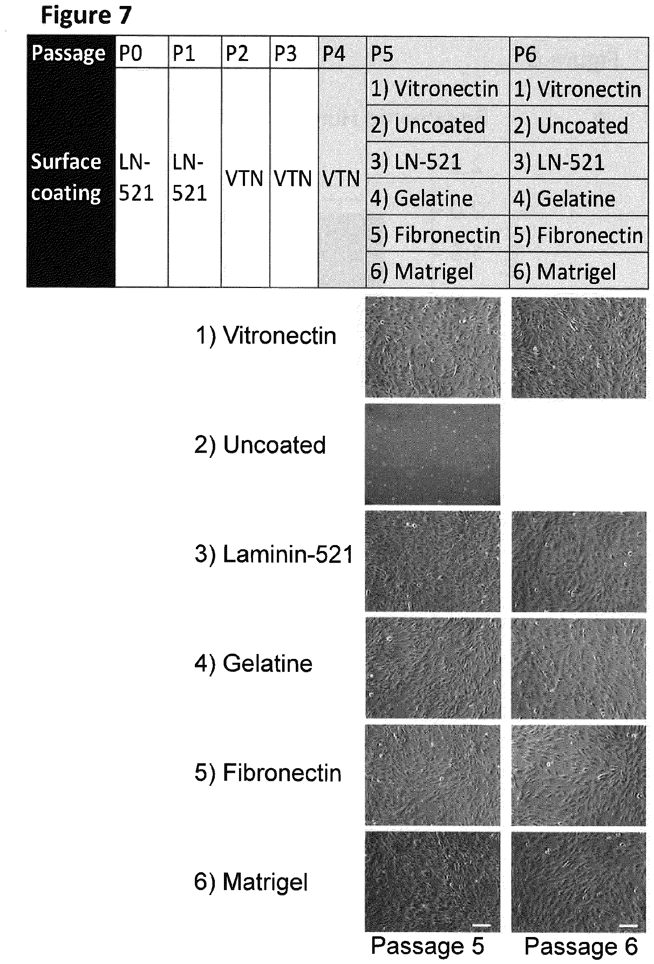

In another embodiment, the first, second, and at least one third extracellular matrix protein is provided in immobilised form on a substrate or to the suspension culture in soluble form. It is even more preferred that the extracellular matrix protein is provided in immobilized form on a substrate. A "substrate" as used herein is a surface on which the cells can differentiate or amplify. Furthermore, the skilled person is aware of suitable substrates for supporting the differentiation or amplification of cells. When the ECM protein is provided in immobilized form, the substrate may be covered with said ECM protein. Suitable concentrations for said covering/coating are known in the art and further described below. In a preferred embodiment, the cells are cultured on ECM coated plates, but the cells may also be cultured on beads and/or in bioreactors as aggregate cultures. For example, Frank et al. (2019) provides a hollow-fiber bioreactor for culturing human mesenchymal stem cells. Thus, it is contemplated that the method as disclosed herein can also be carried out in bioreactors. Preferred substrates for the method as disclosed herein are plates or beads. The most preferred substrate are plates. Exemplary data for the cultivation on plates can be found in the Example section, as well as **Figure 1C** and **Figure 7****.**

In the context of the present disclosure, it is preferred that the first ECM protein in step (i) a1) is provided in immobilized form on a substrate and or the second ECM protein in step (i) a2) is provided in immobilized form on a substrate. It is also preferred that the at least one third extracellular matrix protein in step ii) is provided in immobilized form on a substrate.

Step i) is subdivided in two steps: (i) a1) and (i) a2). For example, the skilled person can monitor the expression of CD90 during step (i) a1) and (i) a2). It is preferred that step (i) a1) is completed when at least 50% of the cells produced by step (i) a1) express of CD90, which can be determined by flow cytometry.

In a preferred embodiment, at least 50% of the cell population produced by step (i) a1) express CD90, less than 50% of the cell population produced by step (i) a1) express CD73, and less than 30% of the cell population produced by step (i) a1) express CD44, as determined by flow cytometry.

Furthermore, in a preferred embodiment, the culturing step (i) a1) is carried out for 2-8 days, preferably 2.5-7 days, more preferably 3-6 days, even more preferably 3.5-5 days and most preferably around 4 days. It is also preferred that the culturing step (i) a2) is carried out for 10-20 days, preferably 11-19 days, more preferably 12-18 days, more preferably 13-17 days, even more preferably 14-16 days and most preferably around 15 days.

It is also preferred that the step (i) (i a1 and i a2) is carried out for 12-28 days, preferably wherein step (i a1 and i a2) is carried out for 15-25 days, more preferably wherein step (i a1 and i a2) is carried out for 16-23 days, most preferably wherein step (i a1 and i a2) is carried out for 17-21 days.

In another preferred embodiment, the serum-free basal medium in step (i) a1) comprises effective amounts of (a) FGF2, (b) vascular endothelial growth factor (VEGF), (c) glutamine and (d) a GSK-3 inhibitor, wherein said amounts result in the expression of CD90 in at least 50% of the cells obtained by step (i) a1), the expression of CD73 in at most 50% of the cells obtained by step (i) a1), and the expression of CD44 in at most 30% of the cells obtained by step (i) a1). Exemplary data for said expression profile can be found in **Figure 3C** measured by flow cytometry.

It is also contemplated that the serum-free basal medium in step (i) a2) comprises effective amounts of (a) FGF2, (b) vascular endothelial growth factor (VEGF), and (c) glutamine, wherein said amounts result in the expression of CD90, CD73, and CD44 in at least 80 % of said population of cardiac stromal cells obtained by step (i) a2).

It is preferred that the serum-free basal medium in step ii) comprises effective amounts of (a) FGF2, (b) VEGF, and (c) glutamine, wherein said amounts in step (ii) are effective to amplify the cardiac stromal cell number.

In an especially preferred embodiment, the method for producing a population of cardiac stromal cells from pluripotent stem cells comprises the step of:
(i) Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises
   a1) culturing said epicardial cells under suitable conditions in the presence of an first extracellular matrix protein in a serum-free basal medium comprising effective amounts of (a) FGF2, (b) vascular endothelial growth factor (VEGF), (c) glutamine and (d) a GSK-3 inhibitor, wherein said amounts result in the expression of CD90 in at least 50% of the cells obtained by step (i) a1), the expression of CD73 in at most 50% of the cells obtained by step (i) a1), and the expression of CD44 in at most 30% of the cells obtained by step (i) a1); followed by
   a2) culturing the cells of step (i) a1) under suitable conditions in the presence of a second extracellular matrix protein in a serum-free basal medium comprising effective amounts of (a) FGF2, (b) VEGF, and (c) glutamine; wherein said amounts result in the expression of CD90, CD73, and CD44 in at least 80 % of said population of cardiac stromal cells obtained by step (i) a2); and
(ii) Amplifying the number of said cardiac stromal cells by culturing said population of cardiac stromal cells of step (i) in the presence of at least one third extracellular matrix protein in a serum-free basal medium comprising effective amounts of (a) FGF2, (b) VEGF, and (c) glutamine, wherein said amounts result in the maintained expression of CD90, CD73, and CD44 in at least 80 % of said cardiac stromal cell population.

As noted above, serum-free basal media are commercially available and recipes can be found in catalogues such as the ATCC catalogue. Preferably, the basal medium of step (i) a1), (i) a2), and/or step (ii) may be KO DMEM, DMEM, DMEM/F12, RPMI, IMDM, alphaMEM, medium 199, Hams F-10, or Hams F-12. Especially preferred is KO DMEM. The basal medium in step (i) a1), (i) a2), and/or step (ii) can be analogously or independently selected.

It is particularly preferred that the serum-free basal medium in step (i) a1), (i) a2), and/or (ii) comprises a final concentration of 10-200 ng/ml FGF2, preferably 15-100 ng/ml, more preferably 20-80 ng/ml, even more preferably 30-70 ng/ml, most preferably 40-60 ng/ml, and most preferably about 50 ng/ml. The concentration of FGF2 may be analogously or independently selected in the steps (i) a1), (i) a2), and/or (ii).

It is also envisaged that the serum-free basal medium in step (i) a1), (i) a2), and/or (ii) may comprise a final concentration of 5-100 ng/ml VEGF, preferably 7-50 ng/ml, more preferably 10-40 ng/ml, even more preferably 15-35 ng/ml, most preferably 20-30 ng/ml, and most preferably about 25 ng/ml VEGF. The concentration of VEGF may be analogously or independently selected in the steps (i) a1), (i) a2), and/or (ii). In mammals, the VEGF family comprises five members: VEGF-A, placenta growth factor (PGF), VEGF-B, VEGF-C and VEGF-D. VEGF-A was discovered first and is also commonly referred to as VEGF. As used herein, VEGF-A and VEGF are referred to synonymously. There are multiple isoforms of VEGF-A that result from alternative splicing of mRNA from a single, 8-exon VEGFA gene. These are classified into two groups, which are referred to according to their terminal exon (exon 8) splice site: the proximal splice site (denoted VEGFxxx) or distal splice site (VEGFxxxb). Furthermore, the alternate splicing of exon 6 and 7 alters their heparin-binding affinity and amino acid number. In humans, the following VEGF forms are known: VEGF121, VEGF121b, VEGF145, VEGF165, VEGF165b, VEGF189, VEGF206. In a particularly preferred embodiment, VEGF is VEGF165.

Furthermore, it is preferred that the serum-free basal medium in step (i) a1), (i) a2), and/or (ii) comprises a final concentration of 0.2-20 mM glutamine, preferably, 0.5-10 mM glutamine, more preferably 0.75-5 mM glutamine, even more preferably 1-3 mM glutamine, even more preferably 1.5-2.5 mM glutamine, and most preferably about 2 mM glutamine. The concentration of glutamine may be analogously or independently selected in the steps (i) a1), (i) a2), and/or (ii). In an especially preferred embodiment, glutamine is present as a L-alanyl-L-glutamine dipeptide. However, any suitable and bioavailable form a glutamine can be used. The skilled person is, for example, aware that L-alanyl-L-glutamine is more stable and water-soluble than glutamine by itself.

It is contemplated that the serum-free basal medium in step (i) a1) comprises a GSK-3 inhibitor, which can be selected from a group consisting of CHIR99021, CHIR98014, SB216763, TWS119, Tideglusib, SB415286, 6-bromoindurubin-3-oxime and a valproate salt, preferably wherein the GSK-3 inhibitor is CHIR99021. However, any GSK3-inhibitor suitable in the method described herein can be applied. In a preferred embodiment, the GSK3-inhibitor in the basal medium of step (i) a1) is CHIR99021. It will be understood by the skilled person that the concentration of an effective amount of a GSK3-inhibitor varies with the availability and inhibition constant of the inhibitor in question. As used herein, the term "effective amount" in the context of a GSK3-inhibitor is intended to mean an enzyme inactivating concentration. For example, in case of CHIR99021, the basal medium in step (i) comprises a final concentration of 0.1-10 µM CHIR99021, preferably 0.2-9 µM, more preferably 0.3-8 µM, even more preferably 0.4-7 µM, still more preferably 0.5-6 µM, more preferably 0.6-5 µM, more preferably 0.7-4 µM, more preferably 0.8-3 µM, most preferably 0.9-2 µM, and even most preferably about 1 µM CHIR99021.

In an embodiment, the serum-free basal medium in step (i) a2) and/or (ii) may further comprises (e) a serum-free "Eukaryotic Cell Culture Medium" (ECCM) supplement. The ingredients and concentrations of the ECCM can be analogously or independently selected in the steps (i) a1), (i) a2) and/or (ii). It is preferred that the ECCM comprises a suitable concentration of ascorbic acid, insulin, transferrin, albumin, and selenium. The skilled person knows that an effective concentration varies with the availability and biological activity of the respective substance. In a preferred embodiment, the ECCM supplement is formulated to provide a final concentration of 1.5-180 µg/ml ascorbic acid (preferably 5-120 µg/ml ascorbic acid, more preferably 15-70 µg/ml ascorbic acid, even more preferably 22-50 µg/ml ascorbic acid, even more preferably 27-40 µg/ml ascorbic acid, even more preferably 30-36 µg/ml ascorbic acid, most preferably about 33 µg/ml ascorbic acid);
2-50 µg/ml insulin; (preferably 4-40 µg/ml insulin, more preferably 5-30 µg/ml insulin, even more preferably 6-22 µg/ml insulin, even more preferably 7-15 µg/ml insulin, even more preferably 9-11 µg/ml insulin, most preferably about 10 µg/ml insulin);
1.1-27.5 µg/ml transferrin (preferably 2-20 µg/ml transferrin, more preferably 3-12.5 µg/ml transferrin, even more preferably 4-8 µg/ml transferrin, even more preferably 4.5-7 µg/ml transferrin, even more preferably 5-6 µg/ml transferrin, most preferably about 5.5 µg/ml transferrin);
1660-41500 µg/ml albumin (preferably 3200-30000 µg/ml albumin, more preferably 4800-20000 µg/ml albumin, even more preferably 6000-11000 µg/ml albumin, even more preferably 7500-9200 µg/ml albumin, even more preferably 8000-8600 µg/ml albumin, most preferably about 8300 µg/ml albumin); and
11-145 nM selenium (preferably 20-105 nM selenium, even more preferably 25-80 nM selenium, even more preferably 30-50 nM selenium, even more preferably 35-45 nM selenium, and most preferably about 40.5 nM selenium).

Ascorbic acid, as part of the ECCM, is defined herein so as to also include a suitable derivative thereof such as ascorbate-2-phosphate. In other words, the ECCM may be formulated to provide a final concentration in the medium of 10-1000 µM ascorbic acid, preferably 20-500 µM, more preferably 100-300 µM, even more preferably 150-250 µM, and most preferably about 195 µM of ascorbic acid or derivative thereof. Especially preferred is that derivative of ascorbic acid is ascorbate-2-phosphate.

Selenium, as part of the ECCM, can be available in the medium as a bioavailable salt. Preferably, the selenium is provided as sodium selenite. In case the selenium is provided as sodium selenite, the ECCM supplement is formulated to provide a final concentration in the basal medium of 0.002-0.025 µg/ml sodium selenite, preferably 0.004-0.01 µg/ml sodium selenite, more preferably 0.006-0.008 µg/ml, even more preferably wherein the ECCM supplement is formulated to provide a final concentration in the basal medium of about 0.007 µg/ml sodium selenite.

In an especially preferred embodiment, the ECCM further comprises a suitable amount of glutathione, and trace elements. In an even more preferred embodiment the ECCM supplement comprises albumin, thiamine, transferrin, insulin, one or more antioxidants selected from the group consisting of reduced glutathione, ascorbic acid and ascorbic acid-2-phosphate, 10 or more amino acids selected from the group consisting of L-histidine, L-isoleucine, L-methionine, L-Phenylalanine, L-Proline, L-Hydroxyproline, L-Serine, L-Threonine, L-Tryptophan, L-Tyrosine, L-Valine, and 15 or more trace elements selected from the group consisting of Ag⁺, Al³⁺, Ba²⁺, Cd²⁺, Co²⁺, Cr³⁺, Ge⁴⁺, Se⁴⁺, Br⁻, I⁻, Mn²⁺, F⁻, Si⁺, V⁵⁺, Mo⁶⁺, Ni²⁺, Rb⁺, Sn²⁺ and Zr⁴⁺. In an even more preferred form, the ECCM comprises an effective concentration of the substances listed in **Table 2.** In an even more preferred embodiment, the ECCM supplement is provided in the basal medium by 5-20% (v/v) as specified in **Table 2,** preferably 6-17.5% (v/v), more preferably 7-15% (v/v), more preferably 8%-12% (v/v), more preferably 9%-11% (v/v), and most preferably about 10% (v/v) ECCM supplement. The person of average skill is aware of the ECCM supplement and the ECCM supplement is also referred to as Knockout serum replacement^{™} (KSR^{™}) commercially. The ECCM can be produced as described in the art, e.g. as described according to the patent application WO 98/30679. Alternatively, ECCM is for example commercially available under the name KSR^{™}, e.g. from Gibco.

In an especially preferred embodiment, the serum-free basal medium in step (i) a1) comprises about 50 ng/ml FGF, about 25 ng/ml VEGF, about 2 mM glutamine and about 1 µM of the GSK-inhibitor, wherein the GSK-inhibitor is preferably selected from the group consisting of CHIR99021, CHIR98014, SB216763, TWS119, Tideglusib, SB415286, 6-bromoindurubin-3-oxime and a valproate salt. It is even more preferred that the serum-free basal medium used in step (i) a1) is Knockout DMEM medium comprising about 2 mM glutamine, about 10% KSR^{™} supplement, about 50 ng/ml FGF, about 25 ng/ml VEGF and about 1 µM CHIR.

In another especially preferred embodiment, the serum-free basal medium in step (i) a2) comprises about 50 ng/ml FGF, about 25 ng/ml VEGF, and about 2 mM glutamine. It is even more preferred that the serum-free basal medium used in step (i) a2) is Knockout DMEM medium comprising about 2 mM glutamine, about 10% KSR^{™} supplement, about 50 ng/ml FGF and about 25 ng/ml VEGF.

In a further especially preferred embodiment, the serum-free basal medium in step (ii) comprises about 50 ng/ml FGF, about 25 ng/ml VEGF, and about 2 mM glutamine. It is even more preferred that the serum-free basal medium used in step ii) is Knockout DMEM medium comprising about 2 mM glutamine, about 10% KSR^{™} supplement, about 50 ng/ml FGF and about 25 ng/ml VEGF.

In a particularly preferred embodiment, the method comprises the steps of:
(i) Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises
   a1) culturing said epicardial cells under suitable conditions in the presence of an first extracellular matrix protein in a serum-free basal medium comprising (a) 10-200 ng/ml FGF2, (b) 5-100 ng/ml vascular endothelial growth factor (VEGF), (c) 0.2-20 mM glutamine, (d) 0.1-10 µM GSK-3 inhibitor, and (e) the ECCM supplement, wherein the ECCM supplement is formulated to provide a final concentration in the basal medium of 6.6-165 µg/ml ascorbic acid, 2-50 µg/ml insulin, 1.1-27.5 µg/ml transferrin, 1660-41500 µg/ml albumin, and 11-145 nM selenium, wherein at least 50% of said cells express CD90, at most 50% of said cells express CD73, and at most 30% of said cells express CD44; followed by
   a2) culturing the cells of step (i) a1) under suitable conditions in the presence of a second extracellular matrix protein in a serum-free basal medium comprising (a) 10-200 ng/ml FGF2, (b) 5-100 ng/ml VEGF, and (c) 0.2-20 mM glutamine, and (d) the ECCM supplement as in step (i) a1); wherein at least about 80 % of the cells of the obtained population of cardiac stromal cells express CD90, CD73, and CD44; and
(ii) Amplifying the number of said cardiac stromal cells by culturing said population of cardiac stromal cells of step (i) in the presence of at least one third extracellular matrix protein in a serum-free basal medium comprising (a) 10-200 ng/ml FGF2, (b) 5-100 ng/ml VEGF, (c) 0.2-20 mM glutamine, and (d) the ECCM supplement as in step (i) a1), wherein at least 80 % of said cardiac stromal cell population maintain the expression of CD90, CD73, and CD44.

In a preferred embodiment, the at least one third extracellular matrix protein is provided in immobilised form on a substrate. It is further contemplated that the substrate is coated in step (ii) with the at least one third extracellular matrix protein selected from the group consisting of vitronectin, laminin, collagen, in particular gelatine, Matrigel, and fibronectin. During step (ii) the cardiac stromal cells can be passaged several times in order to amplify the number of cardiac stromal cells. It is preferred that the at least one third extracellular matrix protein coated substrate in step (ii) is coated with vitronectin in one or more passage(s) and with vitronectin, laminin, collagen, in particular gelatine, Matrigel, or fibronectin in one or more subsequent passage(s). It is even more preferred that the at least one third extracellular matrix protein coated substrate in step (ii) is coated for one passage with vitronectin and for the subsequent passage or passages with vitronectin, laminin, collagen, in particular gelatine, Matrigel, or fibronectin. Exemplary data for such passaging of the cardiac stromal is shown in **Figure 7****.** It is also contemplated that the at least one third extracellular matrix protein is selected from vitronectin, laminin, collagen, in particular gelatine, Matrigel, and fibronectin, preferably wherein the extracellular matrix protein is vitronectin.

The skilled person is aware of suitable concentrations for coating an immobilised substrate and is able to determine suitable concentrations for coating such substrates. For example, the at least one third extracellular matrix protein may be vitronectin, which may then provided in immobilised form on the substrate with a final concentration of 0.1125 µg/cm² - 7.2 µg/cm² vitronectin, preferably 0.225 µg/cm² - 3.6 µg/cm² vitronectin, more preferably 0.45 µg/cm² - 1.8 µg/cm² vitronectin, even more preferably 0.6 µg/cm² - 1.2 µg/cm² vitronectin, even more preferably 0.7 µg/cm² - 1.1 µg/cm² vitronectin, even more preferably 0.8 µg/cm² - 1 µg/cm² vitronectin, most preferably about 0.9 µg/cm² vitronectin. In another embodiment, the at least one third extracellular matrix protein is laminin and is provided in immobilised form on the substrate with a final concentration of 0.1125 µg/cm² - 7.2 µg/cm² laminin, preferably 0.225 µg/cm² - 3.6 µg/cm² laminin, more preferably 0.45 µg/cm² - 1.8 µg/cm² laminin, even more preferably 0.6 µg/cm² - 1.2 µg/cm² laminin, even more preferably 0.7 µg/cm² - 1.1 µg/cm² laminin, even more preferably 0.8 µg/cm² - 1 µg/cm² laminin, most preferably about 0.9 µg/cm² laminin. Furthermore, the at least one third extracellular matrix protein may be collagen and may be provided in immobilised form on the substrate with a final concentration of 8 µg/cm² - 800 µg/cm² collagen, preferably 16 µg/cm² - 400 µg/cm² collagen, more preferably 35 µg/cm² - 250 µg/cm² collagen, even more preferably 50 µg/cm² - 180 µg/cm² collagen, even more preferably 60 µg/cm² - 100 µg/cm² collagen, even more preferably 70 µg/cm² 90 µg/cm² collagen, even more preferably about 80 µg/cm², and most preferably wherein collagen is provided in the form of gelatine. In another embodiment, the at least one third extracellular matrix protein is fibronectin and is provided in immobilised form on a substrate with a final concentration of 0.125 µg/cm² - 8 µg/cm² fibronectin, preferably 0.25 µg/cm² - 4 µg/cm² fibronectin, more preferably 0.5 µg/cm² - 2 µg/cm² fibronectin, even more preferably 0.7 µg/cm² - 1.3 µg/cm² fibronectin, even more preferably 0.8 µg/cm² - 1.2 µg/cm² fibronectin, even more preferably 0.9 µg/cm² - 1.1 µg/cm² fibronectin, most preferably about 1 µg/cm² fibronectin. Exemplary data for the coating of immobilised substrate with ECM proteins is provided in **Example 1** herein.

As discussed above, the cells obtained by step (i)a2) are cardiac stromal cells, wherein at least about 80% of the obtained population of cardiac stromal cells express CD90, CD73 and CD44. In addition to these three marker, cardiac stromal cells may also express vimentin.

The skilled person knows how to reliably detect vimentin in accordance with standard procedures in the art. For example, the expression of vimentin can be detected by flow cytometry, as illustrated by **Example 3** herein. Vimentin is a type III intermediate filament (IF) protein that is expressed in mesenchymal cells. Thus, vimentin is a canonical marker for mesenchymal cells. In a preferred embodiment, at least 80% of the population of cardiac stromal cells obtained by step (i) a2) express vimentin, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, even more preferably at least 90%, even more preferably at least 91%, even more preferably at least 92%, even more preferably at least 93%, even more preferably at least 94%, even more preferably at least 95%, even more preferably at least 96%, even more preferably at least 97%, even more preferably at least 98% vimentin, as determined by flow cytometry. Exemplary data for the expression of vimentin in cardiac stromal cells can also be found in **Figure 3A** as disclosed herein. As step (ii) amplifies the number of cardiac stromal cells, while retaining the phenotype of the cardiac stromal cells, it is also preferred that the amplification step (ii) provides a cell population, wherein at least about 90 % of the cells of said population of cardiac stromal cells express vimentin.

It is further contemplated, that the cardiac stromal cells obtained by step (i) a2) also express type I collagen (collagen-1). The skilled person knows how to reliably detect collagen-1 in accordance with standard procedures in the art. For example, the expression of collagen-1 can be detected by flow cytometry, as illustrated by **Example 3** herein. Type I collagen (collagen 1) is the most abundant collagen of the human body. It forms large, eosinophilic fibers known as collagen fibers. Collagen 1 is formed by a triple-stranded, rope-like procollagen molecules, which are processed by enzymes outside the cell. Once these molecules are processed, they arrange themselves into long, thin fibrils that cross-link to one another in the spaces around cells. The cross-links result in the formation of very strong mature type I collagen fibers (collagen 1), which is all known to the skilled person. In a preferred embodiment, at least 80% of the population of cardiac stromal cells obtained by step (i) a2) express collagen 1, preferably at least 82%, more preferably at least 82%, even more preferably at least 84%, even more preferably at least 86%, even more preferably at least 88%, even more preferably at least 90%, even more preferably at least 91%, even more preferably at least 92%, even more preferably at least 93%, even more preferably at least 94%, even more preferably at least 95%, even more preferably at least 96%, and most preferably at least 97% collagen 1, as determined by flow cytometry. Exemplary data for the expression of collagen-1 can be found in **Figure 3A****.** As step (ii) amplifies the number of cardiac stromal cells, while retaining the phenotype of the cardiac stromal cells, it is also preferred that the amplification step (ii) provides a cell population, wherein at least about 90 % of the cells of said population of cardiac stromal cells express collagen 1. As shown in **Figure 3A****,** cardiac stromal cells can be preferably obtained by the method according to step (i) of the disclosed method, wherein at least about 90 % of the cells produced by step (i) a2) of the population of cardiac stromal cells express vimentin, CD90, collagen-1, and CD73, and at least about 80 % of the cells of the population of cardiac stromal cells express CD44.

It is particularly preferred that the amplification step (ii) provides a cell population, wherein at least about 90 % of the cells of the population of cardiac stromal cells express CD90, CD73 and CD44, preferably wherein the at least 90% of the cells in addition express vimentin and collagen 1.

As described above, epicardial cells can be obtained according to many protocols described in the art such as Witty et al. (2014) or Schlick (2018) Doctoral Thesis, November 2018, University of Gottingen. It is even more preferred that the epicardial cells are obtained from pluripotent stem cells according to the steps (i*) and (i**) as further described below and in Example 1. In particular, the epicardial cells may be obtained by differentiation of pluripotent stem cells by inducing in a step (i*) mesodermal differentiation of the pluripotent stem cells, followed by inducing in a step (i**) epicardial differentiation.

"Mesodermal differentiation" is induced by specific factors/additives in step (i*). The mesoderm is one of the three primary germ layers in the very early embryo. The other two layers are the ectoderm (outside layer) and endoderm (inside layer), with the mesoderm as the middle layer between them. In a preferred embodiment, the mesodermal differentiation in step (i*) comprises culturing said pluripotent stem cells under suitable conditions on a laminin coated immobilised substrate in a serum-free basal medium. It is further preferred that the serum-free basal medium used in step (i*) comprises effective amounts of (a) bone morphogenetic protein 4 (BMP4), (b) Activin A (ActA), (c) a GSK-3 inhibitor, (d) basic fibroblast growth factor (FGF2), (e) glutamine, and (f) a serum-free supplement comprising albumin, transferrin, ethanol amine, selenium or a bioavailable salt thereof, L-carnitine, fatty acid supplement, and triodo-L-thyronine (T3), wherein the amounts are effective to induce mesodermal differentiation of the pluripotent stem cells.

The laminin coated immobilised substrate may be coated as described for step (i) a1).

The length of step (i*) and the concentration of factors such as of (a) bone morphogenetic protein 4 (BMP4), (b) Activin A (ActA), (c) a GSK-3 inhibitor, (d) basic fibroblast growth factor (FGF2), (e) glutamine, and (f) a serum-free supplement may be adjusted by monitoring the efficiency of induction of mesoderm differentiation. This can be achieved by monitoring the expression of cell surface marker CD309 and/or CD140a. Assessing the expression of these cell surface markers can for example be assessed by flow cytometry. The skilled person knows how to reliably detect CD309 and/or CD140a by flow cytometry using standard procedures known in the art. For example, the authors of Kattman et al. (2011) detected CD309 and CD140a by flow cytometry after induction of the mesodermal differentiation.

In an embodiment, at least 50% of the obtained cells after step (i*) express CD309 as determined by flow cytometry, preferably wherein at least 55% of the cells after step (i*) express CD309, more preferably wherein at least 60% of the cells after step (i*) express CD309, more preferably wherein at least 65% of the cells after step (i*) express CD309, more preferably wherein at least 70% of the cells after step (i*) express CD309, more preferably wherein at least 75% of the cells after step (i*) express CD309, even more preferably wherein at least 80 % of the cells after step (i*) express CD309. In another embodiment, at least 50% of the obtained cells after step (i*) express CD140a as determined by flow cytometry, preferably wherein at least 55% of the cells after step (i*) express CD140a, more preferably wherein at least 60% of the cells after step (i*) express CD140a, more preferably wherein at least 65% of the cells after step (i*) express CD140a, more preferably wherein at least 70% the cells after step (i*) express CD140a, more preferably wherein at least 75% of the cells after step (i*) express CD140a, even more preferably wherein at least 80% of the cells after step (i*) express CD140a. In an even more preferred embodiment, at least 80% of the cells after step (i*) express CD309 and CD140a.

According to the expression of CD309 and/or CD140a, the skilled person is able to determine when the mesodermal differentiation step is completed. In a preferred embodiment, step (i*) of the method is carried out for 3-9 days, more preferably wherein step (i*) is carried out for 4-8 days, even more preferably wherein step (i*) is carried out for 5-7 days, most preferably wherein step (i*) is carried out for around 6 days.

The basal medium used in step (i*) can be selected from RPMI, DMEM, αMEM, DMEM/F12, StemPro, and Iscove's medium. Preferably, the basal medium is RPMI. However, any suitable basal medium can be used for step (i*). Basal media are commercially available or can be produced according to publicly available recipes, e.g. from ATCC catalogues. If deemed appropriate, the basal medium may be supplemented with amino acids.

Furthermore, the basal medium of step (i*) may additionally comprise ascorbic acid or a derivative thereof. For example, when ascorbic acid is comprised in the basal medium of step (i*) a concentration range of 10-1000 µM, preferably 50-400 µM, more preferably 100-300 µM, even more preferably 150-250 µM, and most preferably about 200 µM is used. Even more preferred is ascorbic acid in the form of ascorbate-2-phosphate.

In addition, the basal medium of step (i*) can be RPMI, which is further comprises pyruvate. An especially preferred concentration range of pyruvate in RPMI is 0.1-10 mM pyruvate, more preferably 0.2-5 mM pyruvate, even more preferably 0.4-2.5 mM pyruvate, even more preferably 0.8-1.5 mM pyruvate, even more preferably 0.9-1.2 mM pyruvate, most preferably about 1 mM pyruvate.

In a preferred embodiment, the basal medium in step (i*) comprises a final concentration in the basal medium of 1-100 ng/ml BMP4, preferably 2-50 ng/ml, more preferably 4-25 ng/ml, even more preferably 7-15 ng/ml, even more preferably 8-12.5 ng/ml, even more preferably 9-11 ng/ml, and most preferably about 10 ng/ml;
0.3-30 ng/ml Activin A, preferably 0.5-15 ng/ml, more preferably 0.8-7 ng/ml, even more preferably 1-5 ng/ml, still more preferably 2-4 ng/ml, most preferably 2.5-3.5 ng/ml, and even most preferably about 3 ng/ml;
0.1-10 ng/ml FGF2, preferably 1-9 ng/ml, more preferably 2-8 ng/ml, even more preferably 3-7 ng/ml, most preferably 4-6 ng/ml, and even most preferably about 5 ng/ml; and or
0.2-20 mM glutamine (preferably 0.4-10 mM glutamine, more preferably 0.5-5 mM glutamine, more preferably 1-3 mM glutamine, more preferably 1.8-2.2 mM glutamine, even preferably about 2 mM glutamine).

In an especially preferred embodiment, the glutamine is provided in the form of L-alanyl-L-glutamine dipeptide.

The GSK-3 inhibitor of step (i*) can be selected from CHIR99021, CHIR98014, SB216763, TWS119, Tideglusib, SB415286, 6-bromoindurubin-3-oxime and a valproate salt. However, any GSK3-inhibitor suitable in the method described herein can be applied. In a preferred embodiment, the GSK3-inhibitor in the basal medium of step (i*) is CHIR99021. It will be understood by the skilled person that the concentration of an effective amount of a GSK3-inhibitor varies with the availability and inhibition constant of the inhibitor in question. The term "effective amount" as used herein in the context of a GSK3-inhibitor is intended to mean an enzyme inactivating concentration. For example, in case of CHIR99021, the basal medium in step (i*) comprises a final concentration of 0.1-10 µM CHIR99021, preferably 0.2-9 µM, more preferably 0.3-8 µM, even more preferably 0.4-7 µM, more preferably 0.5-6 µM, more preferably 0.6-5 µM, more preferably 0.7-4 µM, more preferably 0.8-3 µM, most preferably 0.9-2 µM, and even most preferably about 1 µM CHIR99021.

The medium also comprises serum-free supplement. The serum-free supplement of step (i*) is formulated to provide a final concentration in the basal medium of the following components: 0.25-25 mg/ml albumin (preferably 0.5-20 mg/ml, more preferably 1-15 mg/ml, more preferably 1.5-10 mg/ml, more preferably 2-5 mg/ml and most preferably 2.25-3.75 mg/ml, such as approximately 2.5 mg/ml);
0.5-50 µg/ml transferrin, (preferably 1-45 µg/ml, more preferably 1.5-40 µg/ml, more preferably 2-35 µg/ml, more preferably 2.5-30 µg/ml, more preferably 3-25 µg/ml, more preferably 3.5-20 µg/ml, more preferably 4-15 µg/ml, more preferably 4.5-10 µg/ml, such as about 5 µg/ml);
0.05-5 µg/ml ethanolamine, (preferably 0.1-4.5 µg/ml, more preferably 0.15-4 µg/ml, even more preferably 0.2-3.5 µg/ml, even more preferably 0.25-3 µg/ml, more preferably 0.3-2.5 µg/ml, more preferably 0.35-2 µg/ml, more preferably 0.4-1.5 µg/ml, most preferably 0.5-1.25 µg/ml, such as about 1 µg/ml);
7.2-723 nM selenium or a bioavailable salt thereof, (preferably 20-350 nM, more preferably 35-150 nM, even more preferably 65-80 nM, most preferably about 72.3 nM);
0.2-20 µg/ml L-carnitine HCI (preferably 0.25-15 µg/ml, more preferably 0.5-10 µg/ml, even more preferably 1-5 µg/ml, more preferably 1.5-2.5 µg/ml, and most preferably about 2 µg/ml);
0.5-50 µg/ml fatty acid supplement, (preferably 0.7-40 µg/ml, more preferably 0.9-20 µg/ml, even more preferably 1-12 µg/ml, more preferably 1.2-4 µg/ml, and most preferably 1.6-3 µg/ml, such as about 2 µg/ml); and
0.0002-0.02 µg/ml triodo-L-thyronine (T3) (preferred 0.0005-0.01 µg/ml, more preferred 0.0008-0.005 µg/ml, even more preferred 0.001-0.003 µg/ml, most preferred about 0.002 µg/ml).
The fatty acid supplement may, for example, include linoleic acid and/or linolenic acid.
For example, a bioavailable salt of selenium is sodium selenite, so that a final concentration of sodium selenite in the basal medium of 0.00125-0.125 µg/ml (preferably 0.0025-0.08 µg/ml, more preferably 0.005-0.05 µg/ml, even more preferably 0.01-0.025 µg/ml, and most preferably 0.0125 µg/ml) is provided, when the serum-free supplement is provided in the medium.
In another embodiment, the serum-free supplement may also comprise one or more of D-galactose, progesterone and putrescine. These components are beneficial for cell viability. Suitable concentrations of the respective components are known to the skilled person or can be easily determined by routine experimentation.
An example of a serum-free supplement may be prepared according to published protocols (see also Brewer et al. 1993) or may be commercially purchased. For example, B27 minus insulin (**Table 1a**) can be used. B27 and 'B27 minus insulin' either comprise insulin or do not comprise insulin, respectively. In a preferred embodiment, the serum-free supplement in step (i*) is provided by 0.1-10 % (v/v) B27 minus insulin in the basal medium, preferably 0.5-8 % (v/v), more preferably 1-6 % (v/v), more preferably 1.5-4 % (v/v), even more preferably 1.5-4 % (v/v), even more preferably 1.7-2.5 % (v/v) B27 minus insulin, and most preferably about 2 % (v/v) B27 minus insulin in the basal medium, as commercially purchased or as preferably prepared according to **Table 1a.**

In order to obtain epicardial cells, the step (i*) mesodermal differentiation of the pluripotent stem cells is then followed by inducing in the step (i**) epicardial differentiation. The "epicardial differentiation" is induced by specific factors/additives in step (i**). Specifically, the epicardial differentiation can be induced by culturing the cells of step (i*) under suitable conditions on laminin coated plates in a serum-free basal medium comprising an effective amount of (a) BMP4, (b) retinoic acid (RA), (c) a GSK-3 inhibitor, (d) insulin, and (e) glutamine and (f) the serum-free supplement as in (i*); whereby the obtained cells express Wilms tumor antigen 1 (WT-1), as determined by qRT-PCR. The laminin coated immobilised substrate may be coated as described for step (i) a1). As described in detail above, the developing epicardium can, for example, be distinguished from the myocardium and endocardium by expression of the transcription factors such as WT-1. The length of step (i**) and the concentration of factors such as (a) BMP4, (b) retinoic acid (RA), (c) a GSK-3 inhibitor, (d) insulin, and (e) glutamine and (f) the serum-free supplement may be adjusted by monitoring the efficiency of induction of epicardial differentiation.
Furthermore, the serum-free basal medium used in step (i**) may comprise effective amounts of (a) BMP4, (b) retinoic acid (RA), (c) a GSK-3 inhibitor, (d) insulin, (e) glutamine and (f) the serum-free supplement as in step (i*), wherein the amounts are effective to induce epicardial differentiation of the cells obtained by step (i*). This can be achieved by monitoring the expression of transcription factor WT-1. Assessing the expression of said marker can for example be assessed by qRT-PCR and the skilled person knows how to reliably detect WT-1 using qRT-PCR, as described above. In a preferred embodiment, the cells after step (i**) express Wilms tumor antigen 1 (WT-1) RNA at least 3-fold more than a housekeeping gene such as TBP, more preferably 4-fold, even more preferably at least 5-fold, most preferably at least 6-fold; and at most 15-fold, as determined by qRT-PCR.
In addition to the expression of WT-1 RNA, the expression can also be assessed by immunofluorescence staining of WT-1. The expression of WT-1 elicits a fluorescent signal, which can be detected under the microscope and the skilled person is aware of fluorescent microscopy. For example, **Figure 1C** provides experimental data and shows the expression of WT-1 after the completion of step (i**) of the method as disclosed herein.

According to the increased expression of WT-1 compared to a housekeeping gene such as TBP, the skilled person is able to determine when the epicardial differentiation step is completed. In a preferred embodiment, step (i**) of the method is carried for 5-9 days, preferably wherein step (i**) is carried out for 6-8 days, more preferably wherein step (i**) is carried out for 6.5-7.5 days, most preferably wherein step (i**) is carried out for around 7 days.

In a preferred embodiment, the basal medium of step (i**) further comprises 10-1000 µM, preferably 50-400 µM, more preferably 100-300 µM, even more preferably 150-250 µM, and most preferably about 200 µM of ascorbic acid or a derivative thereof. Especially preferred is that the derivative of ascorbic acid is ascorbate-2-phosphate.

The basal medium of step (i**) may be for example selected from RPMI, DMEM, αMEM, DMEM/F12, StemPro, and Iscove's medium. In an especially preferred embodiment, the basal medium is RPMI. However, any suitable basal medium can be used for the method. Basal media are commercially available or can be produced according to publicly available recipes, e.g. from ATCC catalogues. If deemed appropriate, the basal medium may be supplemented with amino acids. Even more preferred is that the medium of step (i**) is RPMI comprising pyruvate. If the basal medium is comprising pyruvate, exemplary concentrations in the basal medium are 0.1-10 mM pyruvate (preferably 0.2-5 mM pyruvate, more preferably 0.4-2.5 mM pyruvate, more preferably 0.8-1.5 mM pyruvate, more preferably 0.9-1.2 mM pyruvate, most preferably about 1 mM pyruvate).

As noted above, the basal medium of step (i**) can comprise an effective amount of BMP4, RA, a GSK-3 inhibitor, and insulin. The skilled person knows that an effective concentration or amount of a receptor/enzyme agonist or inhibitor varies with the availability and biological activity of the respective substance.

For example, such a basal medium in step (i**) may comprise a final concentration of 5-500 ng/ml BMP4, preferably 10-250 ng/ml BMP4, more preferably 20-125 ng/ml BMP4, even more preferably 35-70 ng/ml BMP4, even more preferably 40-60 ng/ml BMP4, even more preferably 45-55 ng/ml BMP4, and most preferably about 50 ng/ml BMP4;
0.4-40 µM retinoic acid (RA), preferably 0.8-20 µM RA, more preferably 1.6-10 µM RA, even more preferably 2-8 µM RA, still more preferably 2.5-7 µM RA, even more preferably 2.8-6 µM RA, even more preferably 3-5 µM RA, most preferably 3.5-4.5 µM RA, and even most preferably about 4 µM RA;
0.3-30 µg/ml insulin, preferably 0.5-20 µg/ml, more preferably 1-15 µg/ml, even more preferably 1.5-10 µg/ml, even more preferred 2-5 µg/ml, even more preferably 2.5-3.5 µg/ml, most preferably about 3 µg/ml insulin; and/or
0.2-20 mM glutamine (preferably 0.4-10 mM glutamine, more preferably 0.5-5 mM glutamine, more preferably 1-3 mM glutamine, more preferably 1.8-2.2 mM glutamine, even preferably about 2 mM glutamine).

In an especially preferred embodiment, the glutamine is provided in the form of L-alanyl-L-glutamine dipeptide.

Exemplary and preferred embodiments for possible GSK-3 inhibitors of step (i**) and preferred concentration thereof can be selected analogously and independently from the exemplary and preferred GSK-3 inhibitors in step (i*).

Furthermore, the serum-free supplement referred to in step (i**) can comprise the same ingredients and preferred concentrations as the serum-free supplement as defined for step (i*) above. The ingredients may be selected analogously or independently from step (i*).

In an especially preferred embodiment, the serum-free supplement may comprise insulin, in order to provide insulin in the serum-free basal medium. For example, B27 is a serum-free supplement, which comprises insulin. The difference between B27 and B27 minus insulin is the presence of insulin in B27. **Table 1b** provides the ingredients and concentrations of B27. B27 is also known by the skilled person and has been published for example in Brewer et al. (1993) and B27 is also commercially available. Thus, in an especially preferred embodiment, the serum-free supplement and the insulin in step (i**) are provided by B27 in the basal medium, preferably 0.1-10% (v/v) of B27, preferably 0.5-8 % (v/v), more preferably 1-6 % (v/v), even more preferably 1.5-4% (v/v), even more preferably about 2% (v/v) B27, as commercially obtained or preferably provided by **Table 1b.**

In an especially preferred embodiment, the method comprises the steps of:
i*. Inducing mesodermal differentiation by culturing said pluripotent stem cells under suitable conditions on laminin coated substrate in a serum-free basal medium, wherein at least 90% of the obtained cells express CD90, at most 10% of the obtained cells express CD73 and at most 10% of the obtained cells express CD44, as determined by flow cytometry;
i**. Inducing epicardial differentiation by culturing the cells of step (i*) under suitable conditions on laminin coated substrate in a serum-free basal medium, wherein the obtained cells express Wilms tumor antigen 1 (WT-1), as determined by fluorescent microscopy,
   (i) Inducing epithelial-mesenchymal transition by (i) a1) culturing the cells of step (i**) under suitable conditions in the presence of a first extracellular matrix protein in a serum-free basal medium, wherein at least 50% of said cells express CD90 at most 50% of said cells express CD73 and at most 30% of said cells express CD44; followed by a2) culturing the cells of step (i) a1) under suitable conditions in the presence of a second extracellular matrix protein substrate in a serum-free basal medium; wherein at least about 80 % of the cells of the obtained population of cardiac stromal cells express CD90, CD73, and CD44; and
   (ii) Amplifying the number of said cardiac stromal cells by culturing said population of cardiac stromal cells of step (i) in the presence of at least one third extracellular matrix protein substrate in a serum-free basal medium, wherein at least 80 % of said cardiac stromal cell population maintain the expression of CD90, CD73, and CD44.

In an even more preferred embodiment, the method comprises the steps of:
i*. Inducing mesodermal differentiation by culturing said pluripotent stem cells under suitable conditions on laminin coated substrate in a serum-free basal medium comprising effective amounts of (a) bone morphogenetic protein 4 (BMP4), (b) Activin A (ActA), (c) a GSK-3 inhibitor, (d) basic fibroblast growth factor (FGF2), (e) glutamine, and (f) a serum-free supplement comprising albumin, transferrin, ethanol amine, selenium or a bioavailable salt thereof, L-carnitine, fatty acid supplement, and triodo-L-thyronine (T3), wherein said amounts result in the expression of CD90 in at least 90% of cells obtained by step (i*), the expression of CD73 in at most 10% of cells obtained by step (i*), and the expression of CD44 in at most 10% of the cells obtained by step (i*) determined by flow cytometry;
i**. Inducing epicardial differentiation by culturing the cells of step (i*) under suitable conditions on laminin coated substrates in a serum-free basal medium comprising effective amounts of (a) BMP4, (b) retinoic acid (RA), (c) a GSK-3 inhibitor, (d) insulin, (e) glutamine and (f) the serum-free supplement as in (i); wherein said amounts result in the expression of Wilms tumor antigen 1 (WT-1), as determined by fluorescent microscopy;
   (i) Inducing epithelial-mesenchymal transition by culturing the cells of step (i**) under suitable conditions in the presence of an first extracellular matrix protein in a serum-free basal medium comprising effective amounts of (a) FGF2, (b) vascular endothelial growth factor (VEGF), (c) glutamine and (d) a GSK-3 inhibitor, wherein said amounts result in the expression of CD90 in at least of the cells obtained by step (i) a1), the expression of CD73 in at most 50% of the cells obtained by step (i) a1), and the expression of CD44 in at most 30% of the cells obtained by step (i) a1); followed by a2) culturing the cells under suitable conditions in the presence of a second extracellular matrix protein in a serum-free basal medium comprising effective amounts of (a) FGF2, (b) VEGF, and (c) glutamine; wherein said amounts result in the expression of CD90, CD73, and CD44 in at least 80 % of the obtained population of cardiac stromal cells; and
   (ii) Amplifying the number of said cardiac stromal cells by culturing said population of cardiac stromal cells of step (i) in the presence of at least one third extracellular matrix protein in a serum-free basal medium comprising effective amounts of (a) FGF2, (b) VEGF, and (c) glutamine, wherein said amounts result in the maintained expression of CD90, CD73, and CD44 in at least 80 % of said cardiac stromal cell population.

It is contemplated that the amplification step (ii) can be carry out as long as the cardiac stromal cells maintain the expression of CD90, CD73 and CD44 in at least 80% of the cells. In principle, the amplification may be performed indefinitely. For example, the cardiac stromal cells maintained the expression of CD90, CD73 and CD44 over at least 5 passages as demonstrated in **Example 4,** **Figure 3D****.** In a preferred embodiment, when carrying out the amplification step (ii), a population doubling level of at least 2-fold, preferably at least 3-fold, more preferably at least 4-fold, more preferably at least 5-fold, more preferably at least 6-fold, even more preferably at least 7-fold, even more preferably at least 8-fold, even more preferably at least 9-fold, even more preferably at least 10-fold, even more preferably at least 11-fold, even more preferably at least 12-fold, even more preferably at least 13-fold, even more preferably at least 14-fold, even more preferably at least 15-fold, even more preferably at least 20-fold and at most 200-fold may be achieved. In another preferred embodiment, the amplification step (ii), a population doubling level of at least 2-fold, preferably at least 3-fold, more preferably at least 4-fold, more preferably at least 5-fold; and at most 200-fold, preferably at most 150-fold, preferably at most 100-fold, preferably at most 90-fold, preferably at most 80-fold, more preferably at most 70-fold, more preferably at most 60-fold, more preferably at most 50-fold, even more preferably at most 40-fold, even more preferably at most 30-fold, even more 20-fold may be achieved.

As also demonstrated by experimental evidence in **Figure 2** **(Example 2),** the amplification step (ii) may be performed over several passages and the expression of CD90, CD73 and CD44 are maintained **(****Figure 3D****, Example 4).** In a preferred embodiment, the amplification step (ii) may take place over at least 1 passage, preferably 2 passages, more preferably 3 passages, more preferably 4 passages, more preferably 5 passages, even more preferably 6 passages, even more preferably 7 passages, even more preferably 10 passages; and at most 50 passages. In another preferred embodiment, the amplification step (ii) may take place over at least 1 passage; and at most 50 passages, preferably at most 40 passages, more preferably at most 30 passages, more preferably at most 20 passages, more preferably at most 15 passages, even more preferably at most 14 passages, even more preferably at most 13 passages, even more preferably at most 12 passages, even more preferably at most 11 passages, even more preferably at most 10 passages, even more preferably at most 9 passages, even more preferably at most 8 passages, even more preferably at most 7 passages, and even more preferably at most 6 passages. In light of the present disclosure, the skilled person would readily consider combining any of the disclosed range end-points.

In order to achieve suitable cell densities, pluripotent stem cells may be seeded before start of the differentiation. Thus, in a preferred embodiment, the methods comprises a seeding step prior to step (i*), wherein said pluripotent stem cells are seeded onto laminin coated plates. Furthermore, it may be considered helpful to supplement the medium used in the seeding step with a ROCK-inhibitor. The ROCK-inhibitor may be any ROCK-inhibitor, which can be suitably applied in the method as described herein. In a further preferred embodiment, the ROCK inhibitor is selected from Y27632, H-1152P, Thiazovivin, Fasudil, Hydroxyfasudil, GSK429286A, and RKI-1447, preferably selected from Y27632, H-1152P, Thiazovivin, Fasudil, Hydroxyfasudil, and more preferably the ROCK inhibitor is Y27632 or H-1152P. As demonstrated in the examples below, one particularly useful ROCK-inhibitor is Y27632. It will be understood by the skilled person that the concentration of an effective amount of a ROCK-inhibitor varies with the availability and inhibition constant of the inhibitor in question. For example, in case of Y27632, the medium used in the seeding step may comprise 1-50 µM, preferably 2.5-40 µM, more preferably 5-30 µM, even more preferably 7.5-20 µM, most preferably 8-12 µM, and most preferably about 10 µM Y27632. It will be understood that an effective concentration of any receptor/enzyme agonist or inhibitor varies with the availability and biological activity of the respective compound. The skilled person is able to determine an effective concentration. In a preferred embodiment, the seeding step is carried out 72-120 hours, more preferably 84-108 hours, most preferably about 96 hours prior to step (i*).

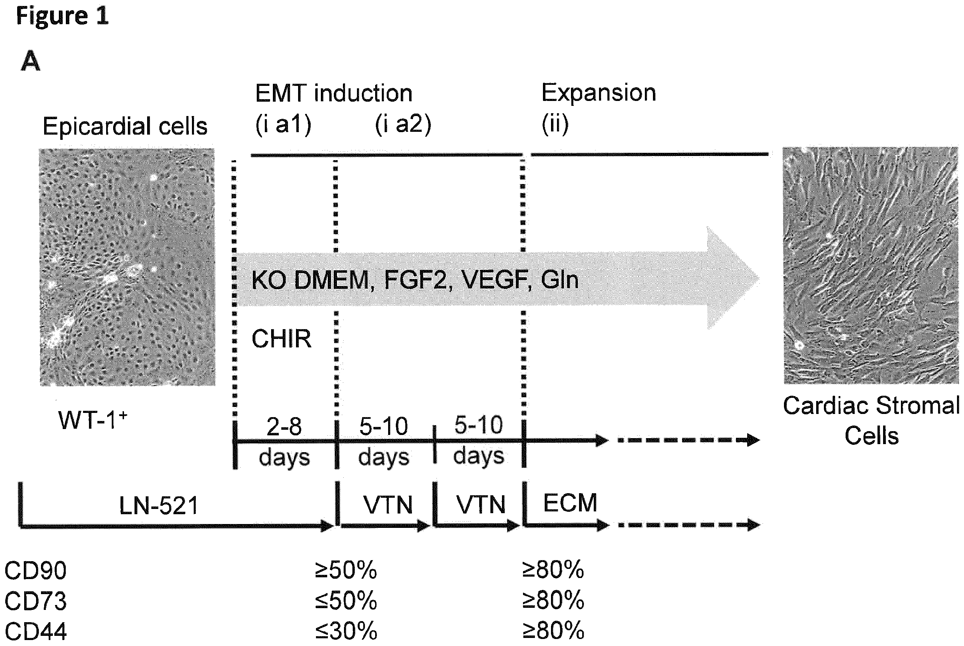

Previous studies have shown that epicardial cells can be differentiated from human pluripotent stem cells using staged differentiation protocols (Witty et al. 2014, Iyer et al. 2015, Bao et al. 2016, and Bao et al. 2017). However, none of the disclosed protocols comprise an amplification step for cardiac stromal cells and do not seem suitable for a scalable production of a homogeneous cardiac stromal cell population, which can be used for tissue engineering purposes. Furthermore, US 2018/0094245 A1 discloses methods for generating high-yield cardiac fibroblasts. The protocol described therein allegedly generates functional cardiac fibroblasts from human pluripotent stem cells under chemically-defined conditions and for long-term maintenance of such human PSC-derived cardiac fibroblasts. However, the experiments disclosed therein differ in at least three features: human PSC-derived cardiac fibroblasts are plated onto uncoated plastic plates in the presence of 2% FBS. Thus, the protocol is (1) not serum-free and (2) the cardiac fibroblasts are cultured on uncoated plates (paragraph [0081]). (3) US '245 discloses that the cardiac fibroblasts already start to lose the expression of CD90 after three days of cultivation (Figure 1a) and fully lose the expression of CD90 during the course of the differentiation protocol (Figure 3c). However, the expression of CD90 is a commonly used cell surface marker to sort resident fibroblasts in the heart (Li et al. 2020). Furthermore, Li et al. showed that cells lacking CD90 are part of the pathogenic cardiac fibroblast fraction in cardiac fibrosis and suggest important roles of the expression CD90 in pathophysiology of heart failure. Thus, cardiac stromal cells as defined and obtained herein, stably expressing CD90, CD97 and CD44 in at least 80% of the cells, are considered to be in a physiological state and able to support the formation of engineered human myocardium (see for example **Figure 6A****)** and engineered connective tissue (see for example **Figure 6B****)** with functional properties of non-diseased myocardium and connective tissue, respectively. This makes the developed population of cardiac stromal cells an attractive source for application in drug development (e.g., anti-fibrotic drugs) and tissue engineered organ repair. Therefore, the expression of CD90, CD73, and CD44 in the production of cardiac stromal cells is an advantageous feature of the cells as produced herein and as defined herein.

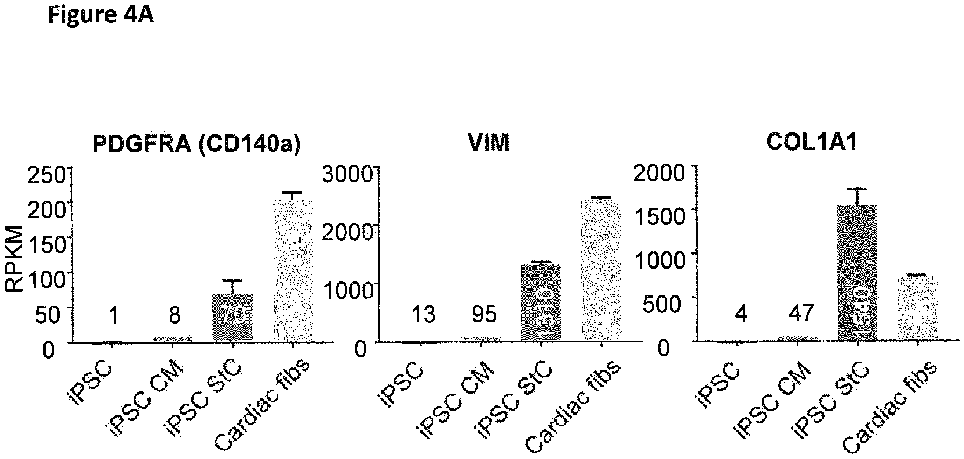

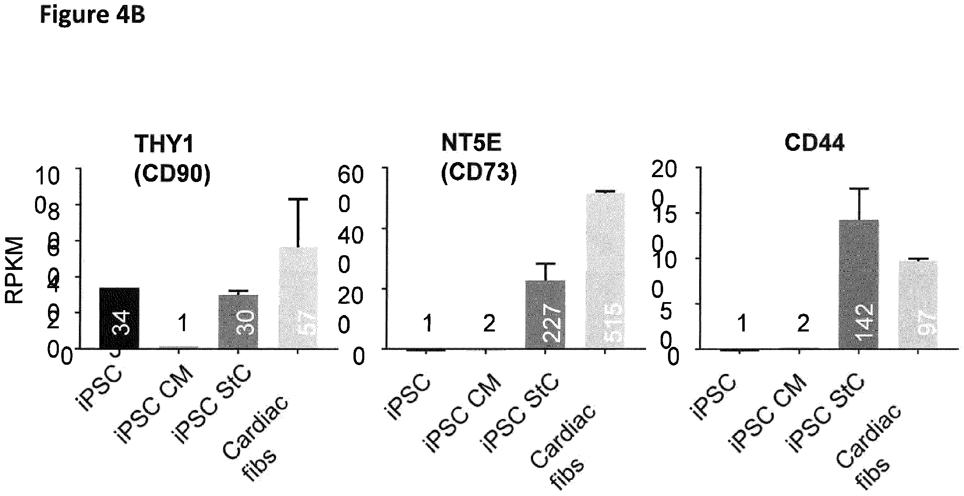

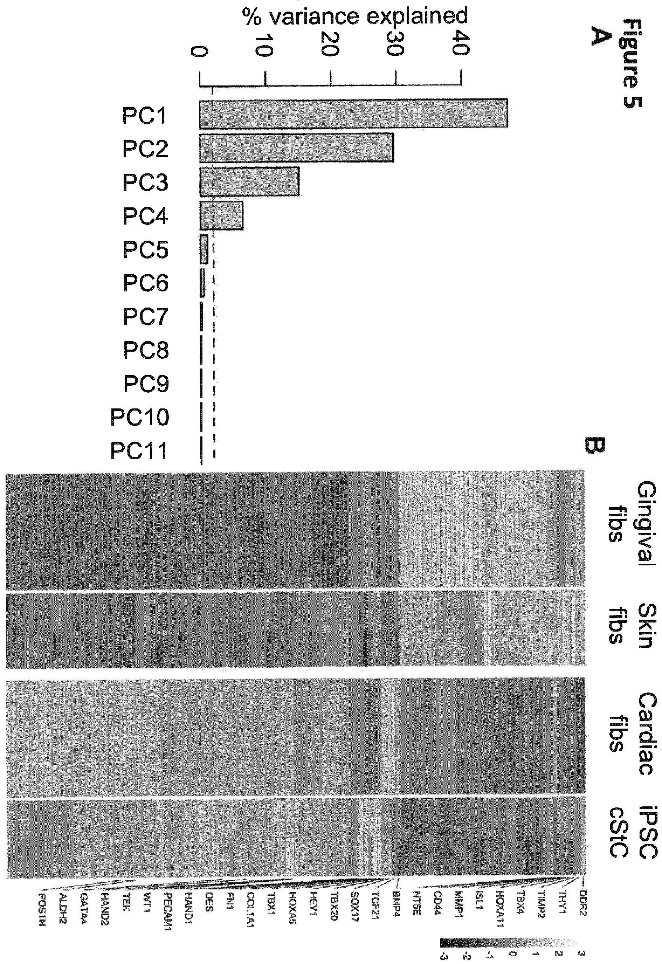

In contrast to many methods disclosed in the prior art, the method as described herein is serum-free and fully defined. The cardiac stromal cells as disclosed herein can be amplified, so that cardiac stromal cells can be mass produced. The production of millions of cells in a serum-free environment is essential and of prime importance for the production of large-scale engineered heart muscles for potential clinical use and standardized research for example in drug development, in particular in anti-fibrotic drug development. It is particularly important that the produced cardiac stromal cells are not only produced in high quantity but also in high quality, i.e. homogeneity. Furthermore, for such a large-scale production of an engineered heart muscle, it is of utmost importance to have a method, wherein a comparable and reproducible quality and quantity of cells can be produced. The ability to expand the cardiac stromal cell over at least six passages is also supported by experimental data **(Example 2** and **Figure 2****).** The high quality (high homogeneity of cardiac stromal cells) is also supported by flow cytometry data in **Example 3, 4** and **Figure 3A****, C** and **D.** The produced cells as disclosed herein express CD90, CD73 and CD44 as determined by flow cytometry, preferably at least 90% of cells **(****Figure 3****).** In addition, the produced cardiac stromal cells can also express Collagen 1 and/or vimentin, which are further indicators of cardiac stromal cells **(****Figure 3A****).** The presence of intracellular pro-collagen-1 **(****Figure 5C****)** is a clear sign, that the produced cells have the ability to produce Collagen-1. The production of pro-collagen-1 can be further stimulated by application of TGFb1 and/or Angiotensin II (ATII) confirming a normal pathophysiological response by the iPSC induced cardiac stromal cells as produced herein. Cardiac stromal cells as produced herein also show a characteristic morphology **(Example 3** shown in **Figure 3B****).** Cardiac stromal cells express vimentin and Collagen-1 by immunofluorescence and show the characteristic pattern of stromal cells. Furthermore, the cardiac stromal cells show a similar gene expression profile of the markers CD140a, vimentin, collagen-1, CD90, CD73 an CD44 when compared to primary cardiac fibroblasts and show a very different expression profile compared to cardiomyocytes **(****Figure 4****).** Moreover, the produced cardiac stromal cells shows an overwhelming overlap of the RNA expression profile with primary cardiac fibroblasts as exemplarily determined by RNA-sequencing **(Example 6,** **Figure 5A and B****).** As the comparison of **Figure 5A** and **B** also includes other types of primary fibroblasts, i.e. skin fibroblasts and gingival fibroblasts, it is demonstrated that the cardiac stromal cells as produced herein are not only high quality stromal cells in general, but are cardiac specific stromal cells with fibroblast properties. Thus, the method as described herein, is highly specific and targeted to obtain cardiac stromal cells.

Furthermore, the cardiac stromal cells as produced herein show fibroblast properties, which is exemplified by the capability of producing pro-collagen **(****Figure 5C****).** In addition, the cardiac stromal cells as produced herein, are also functional in engineered heart muscle and engineered connective tissue **(****Figure 6A** and **6B****).** For example, **Figure 6A** compares the cardiac stromal cells are produced herein with primary cardiac fibroblasts. The engineered heart muscles show a comparable force generation and thus, the cardiac stromal cells as produced herein support the generation of an engineered heart muscle equally well as primary cardiac fibroblasts. Of course cardiac stromal cells, as produced herein, are superior over primary cardiac fibroblasts as they can be artificially produced. Furthermore, the methods as described herein can mass produce these cardiac stromal cells, as pluripotent stem cells can be differentiated and then expanded with the disclosed method with a stable phenotype and sustained CD90 expression, which is a property of non-diseased fibroblasts (Li et al. 2020).

A particular advantage of the method described herein is that a serum-free defined protocol also provides a GMP conform generation of cardiac stromal cells. Good manufacturing practices (GMP) are the practices required in order to conform to the guidelines recommended by agencies that control the authorization and licensing of the manufacture and sale of pharmaceutical products and medical devices. These guidelines provide minimum requirements that a manufacturer must meet to assure that their products are consistently high in quality, from batch to batch, for their intended use. The method described herein is not dependent on a serum or an undefined plate coating, which could vary from batch to batch or comprise other contaminants. Thus, the method disclosed herein is readily transferable to a GMP process, providing another advantage of the method as disclosed herein.

In a still further aspect, an isolated population of cardiac stromal cells is provided, wherein the cardiac stromal cells have been obtained by differentiation of pluripotent stem cells and wherein at least about 80 % of the cells of the population of cardiac stromal cells express CD90, CD73, and CD44. As mentioned above, the expression of said markers is an indicator for the presence of cardiac stromal cells, as determined by flow cytometry.

In an embodiment, at least 80% of the population of cardiac stromal cells express CD44, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD44;
at least 80% of the population of cardiac stromal cells express CD90, preferably at least 81%, more preferably at least 82%, even more preferably at least 83%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD90; and/or
at least 80% of the population of cardiac stromal cells express CD73, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD73, as determined by flow cytometry. Exemplary data for the expression of CD44, CD73, and CD90 in the cardiac stromal cells is provided in **Figure 3A****, C** and **D.**

In a more preferred embodiment, at least 80% of the population of cardiac stromal cells further expresses vimentin, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, even more preferably at least 90% vimentin, even more preferably at least 91%, even more at least 92%, even more preferably at least 93%, even more preferably at least 94%, even more preferably at least 95%, even more preferably at least 96%, even more preferably at least 97%, even more preferably at least 98% vimentin, as determined by flow cytometry.

It is also contemplated that at least 80% of the population of cardiac stromal cells further expresses collagen 1, preferably at least 81%, more preferably at least 82%, even more preferably at least 84%, even more preferably at least 86%, even more preferably at least 88%, even more preferably at least 90%, even more preferably at least 91%, even more preferably at least 92%, even more preferably at least 93%, even more preferably at least 94%, even more preferably at least 95%, even more preferably at least 96%, and most preferably at least 97% collagen 1, as determined by flow cytometry.

The exemplary cells shown in **Figure 3** demonstrate that the cardiac stromal cells can express all five markers CD90, vimentin, collagen 1, CD44 and CD73 at the same time. In an especially preferred embodiment, about 95% or more of the population of cardiac stromal cells are CD90 positive; about 99% or more are CD44 positive; about 99% or more are CD73 positive; about 97% or more are collagen 1 positive; and about 98% or more of are vimentin positive.

In a preferred embodiment, the population of cardiac stromal cells is obtained by the method as disclosed herein. Experimental data of an isolated population of cardiac stromal cells is provided in **Figure 3****.** It also preferred that the population is obtainable by the method as defined by the method disclosed herein. Furthermore, it is contemplated that the population is obtained by the method as defined herein.

In another aspect, a pharmaceutical composition comprising an isolated population of cardiac stromal cells as defined herein, is provided. It is preferred that said pharmaceutical composition further comprises a pharmaceutically acceptable excipient.

According to a still another aspect, an engineered organ tissue is described, said organ tissue comprising a population of cardiac stromal cells as defined herein or as obtained by the method as disclosed herein. Preferably said engineered organ may be an engineered human organ. Generally, the cardiac stromal cells as disclosed herein can support the generation of any organ of the human body. Preferred is an *in vitro* production of human tissue, such as human myocardium or human connective tissue. Connective tissue is one of the four basic types of animal tissue, along with epithelial tissue, muscle tissue, and nervous tissue. Connective tissue is found in between other tissues everywhere in the human body, including the nervous system. For example, the cardiac stromal cells as obtained herein can be used in an *in vitro* production of an engineered connective tissue (ECT; as supported by experimental data in **Figure 6B****)** following protocols as previously demonstrated with primary fibroblasts by the inventors (Zimmermann et al. 2006, Dworatzek et al. 2019, Santos et al. 2019, Schlick et al. 2019), for clinical and research applications. Alternatively, **Figure 6A** provides experimental data that the cardiac stromal cells as produced herein support the formation of engineered heart muscle (human myocardium).

In a still further aspect, a use of the population of cardiac stromal cells (cStC) obtainable and/or obtained by the method as disclosed herein in an *in vitro* model for drug screening. For example, the toxicity and/or efficacy of drugs may be tested *ex vivo.* For example, substances/drugs may alter ECM quality and quantity, such as TGFb1- and BMP-signalling modulators, modulators of the renin-angiotensin-aldosterone-system (RAAS; such as inhibitors of the angiotensin converting enzyme or angiotensin type 1 receptor blockers), and corticoids. Alteration in cStC derived ECM in engineered tissue (such as but not restricted to ECT) results in changes in the respective engineered tissue viscoelastic properties, such as in an increase or decrease of the Young's modulus. The modulus of engineered tissue can be measured as demonstrated by the inventors using for example plate-cone shear rheometry (Schlick et al. 2019) or stress-strain analyses (Santos et al. 2019). The ability to screen drugs for their efficacy and/or toxicity before using said drugs in *in vivo* models is a clear advantage, as several drugs can be screened in parallel and several dosing regimens can be assessed easily. Thus, the present disclosure also relates to the use of the cardiac stromal cells in an *in vitro* model for drug efficacy screening. The results of such drug efficacy screens can also inform drug development before said experimental drug is tested in an *in vivo* system. Furthermore, the use of cardiac stromal cells in an *in vitro* model for drug toxicity screening is also disclosed herein. For example, high doses of a drug can lead to increased toxicity, which can be easily tested *in vitro* by using the cardiac stromal cells in an *in vitro* model. Toxicity can be easily assessed by the rate of death cells in culture. In particular, anti-fibrotic drug development can be guided by using the cardiac stromal cells as used herein.

Furthermore, the use of the population of cardiac stromal cells as defined herein or as obtained by the method disclosed herein in an *in vitro* production of an engineered organ tissue is described herein. In a preferred embodiment, the engineered organ tissue is human engineered organ tissue, preferably wherein the engineered human tissue is engineered human myocardium. In another embodiment, the engineered organ tissue is human engineered organ tissue, preferably wherein the engineered organ human tissue is engineered human connective tissue. It is also contemplated that the population of cardiac stromal cells as defined herein or as obtained by the method according to the method disclosed herein may be used as a research tool.

Generally, the population of cardiac stromal cells as defined herein or as obtained by the method disclosed herein are also suitable for use in medicine. The population of cardiac stromal cells as defined herein or obtained by the method as disclosed herein may be used in organ repair, preferably heart repair or soft tissue repair, more preferably heart repair. Merely as an example, it is contemplated that said cardiac stromal can be advantageously used in heart repair. For example, the cardiac stromal cells can be used to produce engineered heart muscle, which could be used in a patient with heart failure. As an example, **Figure 6A** shows the use of cardiac stromal cells in engineered human myocardium. Another application for the use of cardiac stromal cells can be soft tissue repair. Soft tissue is all the tissue in the body that is not hardened by the processes of ossification or calcification such as bones and teeth. Soft tissue connects, surrounds or supports internal organs and bones, and includes muscle, tendons, ligaments, fat, fibrous tissue, skin, lymph and blood vessels, fasciae, and synovial membranes. In the process of soft tissue repair, the skilled person is aware of several approaches in order to support soft tissue repair. For example, Wehrhan et al. (2010) described the suitability of a bovine collagenous membrane as a dermal substitute after reconstructive surgical procedures. StC containing engineered connective tissue (ECT) can be applied analogously to support soft tissue defects, either by implant site stabilization or augmentation. This is exemplified by data from the inventors showing that non-contractile primary stromal cell comprising ECT could be applied to delay disease progression in a rat model of heart failure (Zimmermann et al. 2006). The application of ECT is not restricted to the heart, but may also be applied to substitute for a soft tissue loss in non-cardiac tissues, similar as suggested by the inventors in (Wehrhan et al. 2010) or in conditions such as after trauma or irradiation induced tissue damage. Analogously to the generation of engineered connective tissue, as e.g. shown in **Figure 6B** herein, it is expected that the cardiac stromal cells as defined herein or obtained by a method disclosed herein, also support the repair of soft tissues *in vivo.* The advantage of such a cellularized tissue compared to collagenous membrane is that the StC containing engineered soft tissue represents a viable tissue comprised of StC with the capability to integrate and produce additional trophic and ECM factors after implantation to facilitate wound healing/repair and thereby supports soft tissue repair. Applications for such soft tissue repair may be dermatology, surgery, dentistry, e.g. supporting the gingival structure for open necks of teeth.

In a further aspect, a serum-free cell culture medium suitable for amplification of a cardiac stromal cells is described, said medium comprising (a) a serum-free basal medium, (b) 10-200 ng/ml FGF2, (c) 5-100 ng/ml VEGF, (d) 0.2-20 mM glutamine, and (e) a ECCM supplement comprising 1.5-180 µg/ml ascorbic acid, 2-50 µg/ml insulin, 1.1-27.5 µg/ml transferrin, 1660-41500 µg/ml albumin, and 11-145 nM selenium. As exemplary shown in step (ii) of the method described herein, said medium can be used to amplify cardiac stromal cells indefinitely as long as the cardiac stromal cells maintain the expression of CD44, CD73, and CD90 in at least 80% of the cardiac stromal cells. In a preferred embodiment, the basal medium is KO DMEM, DMEM, DMEM/F12, RPMI, IMDM, alphaMEM, medium 199, Hams F-10, or Hams F-12, preferably wherein the basal medium is KO DMEM.

In another preferred embodiment, the cell culture medium comprises a final concentration of 15-100 ng/ml FGF2, (preferably 20-80 ng/ml, even more preferably 30-70 ng/ml, most preferably 40-60 ng/ml, and most preferably about 50 ng/ml);
7-50 ng/ml VEGF, (preferably 10-40 ng/ml, even more preferably 15-35 ng/ml, most preferably 20-30 ng/ml, and most preferably about 25 ng/ml); and/or
0.2-20 mM glutamine, preferably, 0.5-10 mM glutamine, more preferably 0.75-5 mM glutamine, even more preferably 1-3 mM glutamine, even more preferably 1.5-2.5 mM glutamine, and most preferably about 2 mM glutamine. For example, glutamine may be comprised in the form of a bioavailable form such as a L-alanyl-L-glutamine dipeptide.

In another preferred embodiment, the cell culture medium comprises the ECCM supplement, wherein the ECCM is formulated to provide a final concentration in the basal medium of 10-80 µg/ml ascorbic acid, 4-40 µg/ml insulin, 2-20 µg/ml transferrin, 3200-30000 µg/ml albumin, and 11-145 nM selenium.

It is especially preferred that the ECCM supplement is formulated to provide a final concentration in the basal medium of 15-60 µg/ml ascorbic acid, 5-30 µg/ml insulin, 3-12.5 µg/ml transferrin, 4800-20000 µg/ml albumin and 20-105 nM selenium. It is further preferred that the ECCM supplement is formulated to provide a final concentration in the basal medium of 27-40 µg/ml ascorbic acid, 7-15 µg/ml insulin, 4.5-7 µg/ml transferrin, 7500-9200 µg/ml albumin and 30-50 nM selenium. It is even more preferred that the ECCM supplement is formulated to provide a final concentration in the basal medium of 30-36 µg/ml ascorbic acid, 9-11 µg/ml insulin, 5-6 µg/ml transferrin, 8000-8600 µg/ml albumin and 35-45 nM selenium. It is even more preferred that the ECCM supplement is formulated to provide a final concentration in the basal medium of about 33 µg/ml ascorbic acid, about 10 µg/ml insulin, about 5.5 µg/ml transferrin, about 8300 µg/ml albumin and about 40.5 nM selenium.

It is also contemplated that the the ECCM supplement further comprises a suitable concentration glutathione, and trace elements. For example, the ECCM comprises the substances as listed in **Table 2.** It is further envisaged that the culture medium comprises 5-20% (v/v) ECCM as specified in **Table 2,** more preferably 6-17.5% (v/v), more preferably 7-15% (v/v), more preferably 8%-12% (v/v), more preferably 9%-11% (v/v), and most preferably about 10% (v/v) ECCM. In a preferred embodiment, the medium comprises the ECCM as defined in any embodiments above or below.

Moreover, a serum free basal medium is provided, wherein the medium is Knockout DMEM medium comprising about 2 mM glutamine, about 50 ng/ml FGF, about 25 ng/ml VEGF and about 1 µM CHIR, and a ECCM supplement comprising 6.6-165 µg/ml ascorbic acid, 2-50 µg/ml insulin, 1.1-27.5 µg/ml transferrin, 1660-41500 µg/ml albumin, and 11-145 nM elenium. In a preferred embodiment, the medium comprises the ECCM as defined in any embodiments above or below.

Finally, a serum free basal medium is provided, wherein the serum free basal medium is Knockout DMEM medium comprising about 2 mM glutamine, about 50 ng/ml FGF, about 25 ng/ml VEGF, and a ECCM supplement comprising 6.6-165 µg/ml ascorbic acid, 2-50 µg/ml insulin, 1.1-27.5 µg/ml transferrin, 1660-41500 µg/ml albumin, and 11-145 nM selenium. In a preferred embodiment, the medium comprises the ECCM as defined in any embodiment above or below.

The invention is further described by the following embodiments:
1. A method for producing a population of cardiac stromal cells from pluripotent stem cells, the method comprising the steps of:
   i. Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises a1) culturing said epicardial cells under suitable conditions in the presence of a first extracellular matrix protein in a serum-free basal medium;
      followed by a2) culturing the cells of step (i) a1) under suitable conditions in the presence of a second extracellular matrix protein in a serum-free basal medium; wherein at least about 80 % of the cells of the obtained population of cardiac stromal cells express CD90, CD73, and CD44; and
   ii. Amplifying the number of said cardiac stromal cells by culturing said population of cardiac stromal cells of step (i) in the presence of at least one third extracellular matrix protein in a serum-free basal medium, wherein at least 80 % of the cells of the cardiac stromal cell population maintain the expression of CD90, CD73, and CD44.
2. The method of embodiment 1, wherein step (ii) stably amplifies the population cardiac stromal cells as determined by the maintained expression of at least 80% of CD90, CD73 and CD44 using flow cytometry.
3. The method of embodiment 1 or 2, wherein the first extracellular matrix protein in step (i) a1) is provided in immobilized form on a substrate and/or wherein the second extracellular matrix protein in step (i) a2) is provided in immobilized form on a substrate.
4. The method of any of the preceding embodiments, wherein the at least one third extracellular matrix protein in step ii) is provided in immobilized form on a substrate.
5. The method of embodiments 1 or 2, wherein the cultivation of the cells in step (i) a1), step (i) a2), and/or the cultivation of said population of said cardiac stromal cells in step (ii) is carried out in suspension culture.
6. The method of embodiment 5, wherein the first extracellular matrix protein in step (i) a1) is provided to the suspension culture in soluble form and and/or wherein the second extracellular matrix protein is provided to the suspension culture in step (i) a2) in soluble form, preferably wherein the first and the second extracellular matrix protein are provided in steps (i) a1) and (i) a2) in soluble form.
7. The method of embodiments 5 or 6, wherein the at least one third extracellular matrix protein in step (ii) is provided to the suspension culture in soluble form.
8. The method of any of the preceding embodiments, wherein the culturing step (i) a1) is carried out for up to 6 days, preferably up to 5 days or up to 4 days.
9. The method of any of embodiments 1 to 7, wherein the culturing step (i) a1) is carried out for 2-8 days, preferably 2.5-7 days, more preferably 3-6 days, even more preferably 3.5-5 days and most preferably around 4 days.
10. The method of any of embodiments 1 to 9, wherein the culturing step (i) a2) is carried out for a period of 12 to 19 days, preferably for a period of 13 to 17 days.
11. The method of any of embodiments 1 to 9, wherein the culturing step (i) a2) is carried out 10-20 days, preferably 11-19 days, more preferably 12-18 days, more preferably 13-17 days, even more preferably 14-16 days and most preferably around 15 days.
12. The method of any of the preceding embodiments, wherein at least 50% of the cells produced by step (i) a1) express of CD90, preferably wherein said expression is determined by flow cytometry.
13. The method of any of embodiments 1 to 12, wherein the serum-free basal medium in step (i) a1) comprises effective amounts of (a) FGF2, (b) vascular endothelial growth factor (VEGF), (c) glutamine and (d) a GSK-3 inhibitor, wherein said amounts result in the expression of CD90 in at least 50% of the cells obtained by step (i) a1), the expression of CD73 in at most 50% of the cells obtained by step (i) a1), and the expression of CD44 in at most 30% of the cells obtained by step (i) a1).
14. The method of any of the preceding embodiments, wherein the serum-free basal medium in step (i) a2) comprises effective amounts of (a) FGF2, (b) vascular endothelial growth factor (VEGF), and (c) glutamine, wherein said amounts result in the expression of CD90, CD73, and CD44 in at least 80 % of said population of cardiac stromal cells obtained by step (i) a2).
15. The method of any of the preceding embodiments, wherein the serum-free basal medium in step ii) comprises effective amounts of (a) FGF2, (b) VEGF, and (c) glutamine, wherein said amounts in step (ii) are effective to amplify the cardiac stromal cell number.
16. The method of any of the preceding embodiments, wherein the method comprises the steps of:
   i. Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises
      a1) culturing said epicardial cells under suitable conditions in the presence of an first extracellular matrix protein in a serum-free basal medium comprising effective amounts of (a) FGF2, (b) vascular endothelial growth factor (VEGF), (c) glutamine and (d) a GSK-3 inhibitor, wherein said amounts result in the expression of CD90 in at least 50% of the cells obtained by step (i) a1), the expression of CD73 in at most 50% of the cells obtained by step (i) a1), and the expression of CD44 in at most 30% of the cells obtained by step (i) a1); followed by
      a2) culturing the cells of step (i) a1) under suitable conditions in the presence of a second extracellular matrix protein in a serum-free basal medium comprising effective amounts of (a) FGF2, (b) VEGF, and (c) glutamine; wherein said amounts result in the expression of CD90, CD73, and CD44 in at least 80 % of said population of cardiac stromal cells obtained by step (i) a2); and
   ii. Amplifying the number of said cardiac stromal cells by culturing said population of cardiac stromal cells of step (i) in the presence of at least one third extracellular matrix protein in a serum-free basal medium comprising effective amounts of (a) FGF2, (b) VEGF, and (c) glutamine, wherein said amounts result in the maintained expression of CD90, CD73, and CD44 in at least 80 % of said cardiac stromal cell population;
17. The method of any of the preceding embodiments, wherein the first, second, and at least one third extracellular matrix protein are provided in immobilised form on a substrate or to the suspension culture in soluble form, preferably wherein the first, second, and at least one third extracellular matrix protein are provided in immobilised form on a substrate.
18. The method of any of the preceding embodiments, wherein the pluripotent stem cells are pluripotent stem cells of primate origin, preferably human pluripotent stem cells.
19. The method of any of the preceding embodiments, wherein the pluripotent stem cells are selected from induced pluripotent stem cells and parthenogenetic stem cells, preferably wherein the pluripotent stem cells are induced pluripotent stem cells.
20. The method of any of embodiment 13 to 19, wherein the serum-free basal medium in step (i) a1) comprises a final concentration of 10-200 ng/ml FGF2, preferably 15-100 ng/ml, more preferably 20-80 ng/ml, even more preferably 30-70 ng/ml, most preferably 40-60 ng/ml, and most preferably about 50 ng/ml.
21. The method of any of embodiment 13 to 20, wherein serum-free basal medium in step (i) a1) comprises a final concentration of 5-100 ng/ml VEGF, preferably 7-50 ng/ml, more preferably 10-40 ng/ml, even more preferably 15-35 ng/ml, most preferably 20-30 ng/ml, and most preferably about 25 ng/ml VEGF.
22. The method of any of embodiment 13 to 21, wherein serum-free basal medium in step (i) a1) comprises a final concentration of 0.2-20 mM glutamine, preferably, 0.5-10 mM glutamine, more preferably 0.75-5 mM glutamine, even more preferably 1-3 mM glutamine, even more preferably 1.5-2.5 mM glutamine, and most preferably about 2 mM glutamine.
23. The method of any of embodiment 13 to 22, wherein the GSK-3 inhibitor of step (i) a1) is selected from a group consisting of CHIR99021, CHIR98014, SB216763, TWS119, Tideglusib, SB415286, 6-bromoindurubin-3-oxime and a valproate salt, preferably wherein the GSK-3 inhibitor is CHIR99021.
24. The method of embodiment 23, wherein the basal medium in step (i) a1) comprises a final concentration of 0.1-10 µM CHIR99021, preferably 0.2-9 µM, more preferably 0.3-8 µM, even more preferably 0.4-7 µM, still more preferably 0.5-6 µM, more preferably 0.6-5 µM, more preferably 0.7-4 µM, more preferably 0.8-3 µM, most preferably 0.9-2 µM, and even most preferably about 1 µM CHIR99021.
25. The method of any of embodiments 13 to 24, wherein the serum-free basal medium in step (i) a1) comprises about 50 ng/ml FGF, about 25 ng/ml VEGF, about 2 mM glutamine and about 1 µM of the GSK-inhibitor, wherein the GSK-inhibitor is preferably selected from the group consisting of CHIR99021, CHIR98014, SB216763, TWS119, Tideglusib, SB415286, 6-bromoindurubin-3-oxime and a valproate salt.
26. The method of any of embodiments 13 to 25, wherein the serum-free basal medium in step (i) a1) further comprises (e) a serum-free ECCM supplement comprising a suitable concentration of ascorbic acid, insulin, transferrin, albumin, and selen preferably wherein the ECCM supplement is formulated to provide a final concentration in the basal medium of 1.5-180 µg/ml ascorbic acid, 2-50 µg/ml insulin, 1.1-27.5 µg/ml transferrin, 1660-41500 µg/ml albumin, and 11-145 nM selenium, even more preferably wherein the ECCM supplement is formulated to provide a final concentration in the basal medium of about 33 µg/ml ascorbic acid, about 10 µg/ml insulin, about 5.5 µg/ml transferrin, about 8300 µg/ml albumin and about 40.5 nM selenium.
27. The method of embodiment 26, wherein the selenium in the ECCM supplement in step (i) a1) is provided as sodium selenite, preferably wherein the ECCM supplement is formulated to provide a final concentration in the basal medium of 0.002-0.025 µg/ml sodium selenite, more preferably wherein the ECCM supplement is formulated to provide a final concentration in the basal medium of about 0.007 µg/ml sodium selenite, even more preferably wherein the ECCM further comprises a suitable amount of glutathione, and trace elements.
28. The method of embodiments any of embodiments 13-27, wherein the serum-free basal medium in step (i) a1) further comprises (e) a serum-free ECCM supplement comprising albumin, thiamine, transferrin, insulin, one or more antioxidants selected from the group consisting of reduced glutathione, ascorbic acid and ascorbic acid-2-phosphate, 10 or more amino acids selected from the group consisting of L-histidine, L-isoleucine, L-methionine, L-Phenylalanine, L-Proline, L-Hydroxyproline, L-Serine, L-Threonine, L-Tryptophan, L-Tyrosine, L-Valine, and 15 or more trace elements selected from the group consisting of Ag⁺, Al³⁺, Ba²⁺, Cd²⁺, Co²⁺, Cr³⁺, Ge⁴⁺, Se⁴⁺, Br⁻, I⁻, Mn²⁺, F⁻, Si⁺, V⁵⁺, Mo⁶⁺, Ni²⁺, Rb⁺, Sn²⁺ and Zr⁴⁺.
29. The method of embodiments 26-28, wherein the ECCM supplement in step (i) a1) is provided by 5-20% (v/v) ECCM supplement as specified in **Table 2,** preferably 6-17.5% (v/v), more preferably 7-15% (v/v), more preferably 8%-12% (v/v), more preferably 9%-11% (v/v), and most preferably about 10% (v/v) ECCM supplement.
30. The method of any of embodiments 13 to 29, wherein the serum-free basal medium in step (i) a2) comprises a final concentration of 10-200 ng/ml FGF2, preferably 15-100 ng/ml, more preferably 20-80 ng/ml, even more preferably 30-70 ng/ml, most preferably 40-60 ng/ml, and most preferably about 50 ng/ml.
31. The method of any of embodiments 13 to 30, wherein the serum-free basal medium in step (i) a2) comprises a final concentration of 5-100 ng/ml VEGF, preferably 7-50 ng/ml, more preferably 10-40 ng/ml, even more preferably 15-35 ng/ml, most preferably 20-30 ng/ml, and most preferably about 25 ng/ml VEGF.
32. The method of any of embodiments 13 to 31, wherein the serum-free basal medium in step (i) a2) comprises a final concentration of 0.2-20 mM glutamine, preferably, 0.5-10 mM glutamine, more preferably 0.75-5 mM glutamine, even more preferably 1-3 mM glutamine, even more preferably 1.5-2.5 mM glutamine, and most preferably about 2 mM glutamine.
33. The method of any of embodiment 13 to 32, wherein serum-free basal medium in step (i) a2) comprises about 50 ng/ml FGF, about 25 ng/ml VEGF, and about 2 mM glutamine.
34. The method of any of embodiments 13 to 33, wherein the serum-free basal medium in step (i) a2) further comprises (d) a serum-free ECCM supplement comprising an suitable concentration of ascorbic acid, insulin, transferrin and albumin, preferably wherein the ECCM supplement is formulated to provide a final concentration in the basal medium of 1.5-180 µg/ml ascorbic acid, 2-50 µg/ml insulin, 1.1-27.5 µg/ml transferrin, 1660-41500 µg/ml albumin, and 11-145 nM selenium, even more preferably wherein the ECCM supplement is formulated to provide a final concentration in the basal medium of about 33 µg/ml ascorbic acid, about 10 µg/ml insulin, about 5.5 µg/ml transferrin, about 8300 µg/ml albumin and about 40.5 nM selenium.
35. The method of embodiment 34, wherein the ECCM supplement in step (i) a2) is provided as sodium selenite, preferably wherein the ECCM supplement is formulated to provide a final concentration in the basal medium of 0.002-0.025 µg/ml sodium selenite, more preferably wherein the ECCM supplement is formulated to provide a final concentration in the basal medium of about 0.007 µg/ml sodium selenite, even more preferably wherein the ECCM further comprises a suitable amount of glutathione, and trace elements.
36. The method of any of embodiments 13-35, wherein the serum-free basal medium in step (i) a2) further comprises (d) a serum-free, ECCM supplement comprising albumin, thiamine, transferrin, insulin, one or more antioxidants selected from the group consisting of reduced glutathione, ascorbic acid and ascorbic acid-2-phosphate, 10 or more amino acids selected from the group consisting of L-histidine, L-isoleucine, L-methionine, L-Phenylalanine, L-Proline, L-Hydroxyproline, L-Serine, L-Threonine, L-Tryptophan, L-Tyrosine, L-Valine, and 15 or more trace elements selected from the group consisting of Ag⁺, Al³⁺, Ba²⁺, Cd²⁺, Co²⁺, Cr³⁺, Ge⁴⁺, Se⁴⁺, Br⁻, I⁻, Mn²⁺, F⁻, Si⁺, V⁵⁺, Mo⁶⁺, Ni²⁺, Rb⁺ Sn²⁺ and Zr⁴⁺.
37. The method of any of embodiments 34 to 36 , wherein the ECCM supplement in step (i) a2) is provided by 5-20% (v/v) ECCM supplement as specified in **Table 2,** preferably 6-17.5% (v/v), more preferably 7-15% (v/v), more preferably 8%-12% (v/v), more preferably 9%-11% (v/v), and most preferably about 10% (v/v) ECCM supplement.
38. The method any of embodiments 13 to 37, wherein the serum-free basal medium in step (ii) comprises a final concentration of 10-200 ng/ml FGF2, preferably 15-100 ng/ml, more preferably 20-80 ng/ml, even more preferably 30-70 ng/ml, most preferably 40-60 ng/ml, and most preferably about 50 ng/ml.
39. The method of any of embodiments 13 to 38, wherein the serum-free basal medium in step (ii) comprises a final concentration of 5-100 ng/ml VEGF, preferably 7-50 ng/ml, more preferably 10-40 ng/ml, even more preferably 15-35 ng/ml, most preferably 20-30 ng/ml, and most preferably about 25 ng/ml VEGF.
40. The method of any of embodiments 13 to 39, wherein the serum-free basal medium in step (ii) comprises a final concentration of 0.2-20 mM glutamine, preferably, 0.5-10 mM glutamine, more preferably 0.75-5 mM glutamine, even more preferably 1-3 mM glutamine, even more preferably 1.5-2.5 mM glutamine, and most preferably about 2 mM glutamine.
41. The method of any of embodiments 13 to 40, wherein the serum-free basal medium in step (ii) comprises about 50 ng/ml FGF, about 25 ng/ml VEGF, and about 2 mM glutamine.
42. The method any of embodiments 13 to 41, wherein the serum-free basal medium in step (ii) further comprises a serum-free ECCM supplement comprising a suitable concentration of ascorbic acid, insulin, transferrin and albumin, preferably wherein the ECCM supplement is formulated to provide a final concentration in the basal medium of 6.6-165 µg/ml ascorbic acid, 2-50 µg/ml insulin, 1.1-27.5 µg/ml transferrin, 1660-41500 µg/ml albumin, and 11-145 nM selenium, even more preferably wherein the ECCM supplement is formulated to provide a final concentration in the basal medium of about 33 µg/ml ascorbic acid, about 10 µg/ml insulin, about 5.5 µg/ml transferrin, about 8300 µg/ml albumin and about 40.5 nM selenium.
43. The method of embodiment 42, wherein the selenium in the ECCM supplement in step (i) a1) is provided as sodium selenite, preferably wherein the ECCM supplement is formulated to provide a final concentration in the basal medium of 0.002-0.025 µg/ml sodium selenite, more preferably wherein the ECCM supplement is formulated to provide a final concentration in the basal medium of about 0.007 µg/ml sodium selenite, even more preferably wherein the ECCM further comprises a suitable amount of, glutathione, and trace elements.
44. The method of any of the embodiments of 13 to 43, wherein the serum-free basal medium in step (ii) further comprises (d) a serum-free ECCM supplement comprising albumin, thiamine, transferrin, insulin, one or more antioxidants selected from the group consisting of reduced glutathione, ascorbic acid and ascorbic acid-2-phosphate 10 or more amino acids selected from the group consisting of L-histidine, L-isoleucine, L-methionine, L-Phenylalanine, L-Proline, L-Hydroxyproline, L-Serine, L-Threonine, L-Tryptophan, L-Tyrosine, L-Valine, and 15 or more trace elements selected from the group consisting of Ag⁺, Al³⁺, Ba²⁺, Cd²⁺, Co²⁺, Cr³⁺, Ge⁴⁺, Se⁴⁺, Br⁻, I⁻, Mn²⁺, F⁻, Si⁺, V⁵⁺, Mo⁶⁺, Ni²⁺, Rb⁺, Sn²⁺ and Zr⁴⁺.
45. The method of any of embodiments 42 to 44, wherein the ECCM supplement in step (ii) is provided by 5-20% (v/v) ECCM supplement as specified in **Table 2,** preferably 6-17.5% (v/v), more preferably 7-15% (v/v), more preferably 8%-12% (v/v), more preferably 9%-11% (v/v), and most preferably about 10% (v/v) ECCM supplement.
46. The method of any of the preceding embodiments, wherein the method comprises the steps of:
   i. Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises
      a1) culturing said epicardial cells under suitable conditions in the presence of an first extracellular matrix protein in a serum-free basal medium comprising (a) 10-200 ng/ml FGF2, (b) 5-100 ng/ml vascular endothelial growth factor (VEGF), (c) 0.2-20 mM glutamine, (d) 0.1-10 µM GSK-3 inhibitor, and (e) the ECCM supplement, wherein the ECCM supplement is formulated to provide a final concentration in the basal medium of 6.6-165 µg/ml ascorbic acid, 2-50 µg/ml insulin, 1.1-27.5 µg/ml transferrin, 1660-41500 µg/ml albumin and 11-145 nM selenium, wherein at least 50% of said cells express CD90, at most 50% of said cells express CD73, and at most 30% of said cells express CD44; followed by
      a2) culturing the cells of step (i) a1) under suitable conditions in the presence of a second extracellular matrix protein in a serum-free basal medium comprising (a) 10-200 ng/ml FGF2, (b) 5-100 ng/ml VEGF, and (c) 0.2-20 mM glutamine, and (d) the ECCM supplement as in step (i) a1); wherein at least about 80 % of the cells of the obtained population of cardiac stromal cells express CD90, CD73, and CD44; and
   ii. Amplifying the number of said cardiac stromal cells by culturing said population of cardiac stromal cells of step (i) in the presence of at least one third extracellular matrix protein in a serum-free basal medium comprising (a) 10-200 ng/ml FGF2, (b) 5-100 ng/ml VEGF, (c) 0.2-20 mM glutamine, and (d) the ECCM supplement as in step (i) a1), wherein at least 80 % of said cardiac stromal cell population maintain the expression of CD90, CD73, and CD44;
47. The method of embodiment 46, wherein the first, second, and at least one third extracellular matrix protein are provided in immobilised form on a substrate or to the suspension culture in soluble form, preferably wherein the first, second, and at least one third extracellular matrix protein are provided in immobilised form on a substrate.
48. The method of embodiments 46 or 47, wherein the serum-free basal medium in step (i) a1), step (i) a2) and/or step (ii) the comprises ECCM supplement, which is provided by 5-20% (v/v) ECCM supplement as specified in Table 2, preferably 6-17.5% (v/v), more preferably 7-15% (v/v), more preferably 8%-12% (v/v), more preferably 9%-11% (v/v), and most preferably about 10% (v/v) ECCM supplement,
49. The method of any of the preceding embodiments, wherein the first extracellular matrix protein comprises laminin, vitronectin, collagen, in particular gelatine, fibronectin, elastin, preferably wherein the first extracellular matrix protein comprises laminin, more preferably wherein the extracellular matrix protein is laminin.
50. The method of embodiment 49, wherein laminin is laminin-521.
51. The method of any of the preceding embodiments, wherein the second extracellular matrix protein is the same or different from the first extracellular matrix protein.
52. The method of embodiment 51, wherein the second extracellular matrix protein is different from the first extracellular matrix protein and comprises vitronectin, laminin, collagen, in particular gelatine, fibronectin, elastin, preferably wherein the second extracellular matrix protein is vitronectin.
53. The method of any of the foregoing embodiments, wherein the at least one third extracellular matrix protein is selected from the group consisting of vitronectin, laminin, collagen, in particular gelatine, fibronectin, elastin, Matrigel, a peptide containing the amino acid sequence RGD, a protein containing the amino acid sequence RGD and combinations thereof, preferably wherein the at least one third extracellular matrix protein is selected from the group consisting of vitronectin, laminin, collagen, in particular gelatine, fibronectin, and elastin, more preferably wherein the at least one third extracellular matrix protein is vitronectin.
54. The method of any of the preceding embodiments, wherein the at least one third extracellular matrix protein is provided in immobilised form on a substrate.
55. The method of embodiment 54, wherein the at least one third extracellular matrix protein is coating the substrate in step (ii) with an extracellular matrix protein selected from the group consisting of vitronectin, laminin, collagen, in particular gelatine, Matrigel, and fibronectin,
   preferably wherein at least one third extracellular matrix protein coated substrate in step (ii) is coated with vitronectin in one or more passage(s) and with vitronectin, laminin, collagen, in particular gelatine, Matrigel, or fibronectin in one or more subsequent passage(s),
   more preferably wherein at least one third extracellular matrix protein coated substrate in step (ii) is coated for one passage with vitronectin and for the subsequent passage or passages with vitronectin, laminin, collagen, in particular gelatine, Matrigel, or fibronectin, even more preferably wherein the extracellular matrix protein in step (ii) is vitronectin.
56. The method of embodiment 54, wherein the at least one third extracellular matrix protein is selected from vitronectin, laminin, collagen, in particular gelatine, Matrigel, and fibronectin, preferably wherein the extracellular matrix protein is vitronectin.
57. The method of any of embodiments 54 to 56, wherein the at least one third extracellular matrix protein is vitronectin and is provided in immobilised form on the substrate with a final concentration of 0.1125 µg/cm² - 7.2 µg/cm² vitronectin, preferably 0.225 µg/cm² - 3.6 µg/cm² vitronectin, more preferably 0.45 µg/cm² - 1.8 µg/cm² vitronectin, even more preferably 0.6 µg/cm² - 1.2 µg/cm² vitronectin, even more preferably 0.7 µg/cm² - 1.1 µg/cm² vitronectin, even more preferably 0.8 µg/cm² - 1 µg/cm² vitronectin, most preferably about 0.9 µg/cm² vitronectin.
58. The method of any of embodiments 54 to 57, wherein the at least one third extracellular matrix protein is laminin and is provided in immobilised form on the substrate with a final concentration of 0.1125 µg/cm² - 7.2 µg/cm² laminin, preferably 0.225 µg/cm² - 3.6 µg/cm² laminin, more preferably 0.45 µg/cm² - 1.8 µg/cm² laminin, even more preferably 0.6 µg/cm² - 1.2 µg/cm² laminin, even more preferably 0.7 µg/cm² - 1.1 µg/cm² laminin, even more preferably 0.8 µg/cm² - 1 µg/cm² laminin, most preferably about 0.9 µg/cm² laminin.
59. The method of any of embodiments 54 to 58, wherein the at least one third extracellular matrix protein is collagen and is provided in immobilised form on the substrate with a final concentration of 8 µg/cm² - 800 µg/cm² collagen, preferably 16 µg/cm² - 400 µg/cm² collagen, more preferably 35 µg/cm² - 250 µg/cm² collagen, even more preferably 50 µg/cm² - 180 µg/cm² collagen, even more preferably 60 µg/cm² - 100 µg/cm² collagen, even more preferably 70 µg/cm² 90 µg/cm² collagen, even more preferably about 80 µg/cm², and most preferably wherein collagen is provided in the form of gelatine.
60. The method of any of embodiments 54 to 59, wherein the at least one third extracellular matrix protein is fibronectin and is provided in immobilised form on a substrate with a final concentration of 0.125 µg/cm² - 8 µg/cm² fibronectin, preferably 0.25 µg/cm² - 4 µg/cm² fibronectin, more preferably 0.5 µg/cm² - 2 µg/cm² fibronectin, even more preferably 0.7 µg/cm² - 1.3 µg/cm² fibronectin, even more preferably 0.8 µg/cm² - 1.2 µg/cm² fibronectin, even more preferably 0.9 µg/cm² - 1.1 µg/cm² fibronectin, most preferably about 1 µg/cm² fibronectin.
61. The method of any of embodiments 54 to 60, wherein the substrate is a plate or beads, preferably wherein the substrate is a plate.
62. The method of any of embodiments 26 to 61, wherein the ECCM used in (i) a1, (i) a2 and/or (ii) essentially contains the components in the concentrations as recited in **Table 2.**
63. The method of embodiment 62, wherein the ECCM supplement is knockout serum replacement (KSR^{™}) supplement.
64. The method of any of the foregoing embodiments, wherein the serum free basal medium used in (i) a1, (i) a2 and/or (ii) comprises KO DMEM, DMEM, DMEM/F12, RPMI, IMDM, alphaMEM, medium 199, Hams F-10, Hams F-12, wherein the basal medium is preferably KO DMEM.
65. The method of any of embodiments 13 to 64, wherein the serum free basal medium used in step (i) a1) is Knockout DMEM medium comprising about 2 mM glutamine, about 10% KSR^{™} supplement, about 50 ng/ml FGF, about 25 ng/ml VEGF and about 1 µM CHIR.
66. The method of any of embodiments 13 to 65, wherein the serum free basal medium used in step (i) a2) is Knockout DMEM medium comprising about 2 mM glutamine, about 10% KSR^{™} supplement, about 50 ng/ml FGF and about 25 ng/ml VEGF.
67. The method of any of embodiments 13 to 66, wherein the serum free basal medium used in step ii) is Knockout DMEM medium comprising about 2 mM glutamine, about 10% KSR^{™} supplement, about 50 ng/ml FGF and about 25 ng/ml VEGF.
68. The method of any of the preceding embodiments, wherein the epicardial cells express Wilms tumor antigen 1 (WT-1), as determined by fluorescent microscopy.
69. The method of any of the preceding embodiments, wherein the epicardial cells express Wilms tumor antigen 1 (WT-1) RNA at least 2-fold more than TBP (TATA-binding protein), preferably 3-fold, more preferably 4-fold, even more preferably at least 5-fold, most preferably at least 6-fold; and at most 15-fold, as determined by qRT-PCR.
70. The method of any of the preceding embodiments, wherein the epicardial cells are obtained as described in Schlick (2018), Doctoral Thesis, November 2018, University of Gottingen, Witty et al. Nat Biotechnology 32, 1026-1035 (2014) or any other suitable method for providing epicardial cells.
71. The method of any of the preceding embodiments, wherein at least 50% of the cell population produced by step (i) a1) express CD90 as determined by flow cytometry, preferably wherein at least 50% of the cell population produced by step (i) a1) express CD90, less than 50% of the cell population produced by step (i) a1) express CD73, and less than 30% of the cell population produced by step (i) a1) express CD44, as determined by flow cytometry.
72. The method of any of the foregoing embodiments, wherein the amplification step (ii) provides a cell population, wherein at least about 81 %, about 82 %, about 83 %, about 84 %, about 85 %, about 86 %, about 87 %, about 88 %, about 89, about 90 %, about 91% about 92 %, about 93%, about 94, about 95%, about 96 %, about 97 %, about 98%, or about 99 % of the cells of the population of the cardiac stromal cells express CD90.
73. The method of any of the foregoing embodiments, wherein at least 80% of the population of cardiac stromal cells obtained by step (ii) express CD90, preferably at least 81%, more preferably at least 82%, even more preferably at least 83%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD90, as determined by flow cytometry.
74. The method of any of the foregoing embodiments, wherein amplification step (ii) provides a cell population, wherein at least about 81 %, about 82 %, about 83 %, about 84 %, about 85 %, about 86 %, about 87 %, about 88 %, about 89, about 90 %, 91%, about 92 %, about 93%, about 94, about 95%, about 96 %, about 97 %, about 98%, or about 99 % of the cells of the population of the cardiac stromal cells express CD73.
75. The method of any of the foregoing embodiments, wherein at least 80% of the population of cardiac stromal cells obtained by step (ii) express CD73, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD73, as determined by flow cytometry.
76. The method of any of the preceding embodiments, wherein amplification step (ii) provides a cell population, wherein about 81 %, about 82 %, about 83 %, about 84 %, about 85 %, about 86 %, about 87 %, about 88 %, about 89, about 90 %, about 91%, about 92 %, about 93%, about 94, about 95%, about 96 %, about 97 %, about 98%, or about 99 % of the cells of the population of the cardiac stromal cells express CD44.
77. The method of any of the preceding embodiments, wherein at least 80% of the population of cardiac stromal cells obtained by step (ii) express CD44, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD44, as determined by flow cytometry.
78. The method of any of the foregoing embodiments, wherein after step (i) a2) at least about 90 % of the cells of said population of cardiac stromal cells express vimentin.
79. The method of any of the foregoing embodiments, wherein at least 80% of the population of cardiac stromal cells obtained by step (i) a2) express vimentin, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, even more preferably at least 90%, even more preferably at least 91%, even more preferably at least 92%, even more preferably at least 93%, even more preferably at least 94%, even more preferably at least 95%, even more preferably at least 96%, even more preferably at least 97%, even more preferably at least 98% vimentin, as determined by flow cytometry.
80. The method of any of the foregoing embodiments, wherein the amplification step (ii) provides a cell population, wherein at least about 90 % of the cells of said population of cardiac stromal cells express vimentin.
81. The method of any of the foregoing embodiments, wherein after step (i) a2) at least about 90 % of the cells of said population of cardiac stromal cells express collagen 1.
82. The method of any of the foregoing embodiments, wherein at least 80% of the population of cardiac stromal cells obtained by step (i) a2) express collagen 1, preferably at least 82%, more preferably at least 82%, even more preferably at least 84%, even more preferably at least 86%, even more preferably at least 88%, even more preferably at least 90%, even more preferably at least 91%, even more preferably at least 92%, even more preferably at least 93%, even more preferably at least 94%, even more preferably at least 95%, even more preferably at least 96%, and most preferably at least 97% collagen 1, as determined by flow cytometry
83. The method of any of the foregoing embodiments, wherein amplification step (ii) provides a cell population, wherein at least about 90 % of the cells of said population of cardiac stromal cells express collagen 1.
84. The method of embodiment 83, wherein amplification step (ii) provides a cell population, wherein at least about 91%, about 92 %, about 93%, about 94, about 95%, about 96 %, about 97 %, about 98%, or about 99 of the cells of the population of the cardiac stromal cells express collagen 1.
85. The method of any of the preceding embodiments, wherein at least about 90 % of the cells produced by step (i) a2) of the population of cardiac stromal cells express vimentin, CD90, collagen-1, and CD73, and at least about 80 % of the cells of the population of cardiac stromal cells express CD44.
86. The method of any of the preceding embodiments, wherein at least 80% of the population of cardiac stromal cells obtained by step (i) a2) express CD44, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90%, as determined by flow cytometry.
87. The method of embodiment 86, wherein at least 90% of the population of cardiac stromal cells obtained by step (i) a2) express CD44, preferably at least 91%, more preferably at least 92%, even more preferably at least 93%, even more preferably at least 94%, even more preferably at least 95%, even more preferably at least 96%, even more preferably at least 97%, even more preferably at least 98%, and most preferably at least 99%, as determined by flow cytometry.
88. The method of any of the foregoing embodiments, wherein the amplification step (ii) provides a cell population, wherein at least about 90 % of the cells of the population of cardiac stromal cells express CD90, CD73 and CD44, preferably wherein the at least 90% of the cells in addition express vimentin and collagen 1.
89. The method of any of the foregoing embodiments, wherein the epicardial cells are obtained by differentiation of pluripotent stem cells by inducing in a step (i*) mesodermal differentiation of the pluripotent stem cells, followed by inducing in a step (i**) epicardial differentiation.
90. The method of embodiment 89, wherein mesodermal differentiation in step (i*) comprises culturing said pluripotent stem cells under suitable conditions on a laminin coated substrate in a serum-free basal medium.
91. The method of embodiment 90, wherein the serum-free basal medium used in step (i*) comprises effective amounts of (a) bone morphogenetic protein 4 (BMP4), (b) Activin A (ActA), (c) a GSK-3 inhibitor, (d) basic fibroblast growth factor (FGF2), (e) glutamine, and (f) a serum-free supplement comprising albumin, transferrin, ethanol amine, selenium or a bioavailable salt thereof, L-carnitine, fatty acid supplement, and triodo-L-thyronine (T3), wherein the amounts are effective to induce mesodermal differentiation of the pluripotent stem cells.
92. The method of any of embodiments 89 to 91, wherein the serum-free basal medium used in step (i*) further comprises a suitable amount of ascorbic acid.
93. The method of any of embodiments 89 to 92, wherein inducing mesodermal differentiation in step (i*) comprises culturing the pluripotent stem cells under suitable conditions on a laminin coated substrate.
94. The method of any of embodiments 89 to 93, wherein the cells obtained by mesodermal differentiation in step (i*) express CD309 and/or CD140a.
95. The method of any of embodiments 89 to 94, wherein the serum-free basal medium used in step (i**) comprises effective amounts of (a) BMP4, (b) retinoic acid (RA), (c) a GSK-3 inhibitor, (d) insulin, (e) glutamine and (f) the serum-free supplement as in step (i*), wherein the amounts are effective to induce epicardial differentiation of the cells obtained by step (i*).
96. The method of any of embodiments 89 to 95, wherein the serum-free basal medium used in step (i**) further comprises a suitable amount of ascorbic acid.
97. The method of any of embodiments 89 to 96, wherein inducing epicardial differentiation in step (i**) comprises culturing the cells of step (i*) under suitable conditions on a laminin coated substrate.
98. The method of embodiment 97, wherein the epicardial cells obtained in step (i**) express Wilms tumor antigen 1 (WT-1).
99. The method of any of the preceding embodiments, wherein the method comprises the steps of:
   i*. Inducing mesodermal differentiation by culturing said pluripotent stem cells under suitable conditions on laminin coated substrate in a serum-free basal medium, wherein at least 90% of the obtained cells express CD90, at most 10% of the obtained cells express CD73 and at most 10% of the obtained cells express CD44, as determined by flow cytometry;
   i**. Inducing epicardial differentiation by culturing the cells of step (i*) under suitable conditions on laminin coated substrate in a serum-free basal medium, wherein the obtained cells express Wilms tumor antigen 1 (WT-1), as determined by fluorescent microscopy,
      i. Inducing epithelial-mesenchymal transition by culturing the cells of step (i**) under suitable conditions in the presence of a first extracellular matrix protein in a serum-free basal medium, wherein at least 50% of said cells express CD90 at most 50% of said cells express CD73 and at most 30% of said cells express CD44; followed by a2) culturing the cells of step (i) a1) under suitable conditions in the presence of a second extracellular matrix protein substrate in a serum-free basal medium; wherein at least about 80 % of the cells of the obtained population of cardiac stromal cells express CD90, CD73, and CD44; and
      ii. Amplifying the number of said cardiac stromal cells by culturing said population of cardiac stromal cells of step (i) in the presence of at least one third extracellular matrix protein substrate in a serum-free basal medium, wherein at least 80 % of said cardiac stromal cell population maintain the expression of CD90, CD73, and CD44.
100. The method of any of the preceding embodiments, wherein the method comprises the steps of:
   i*. Inducing mesodermal differentiation by culturing said pluripotent stem cells under suitable conditions on laminin coated substrate in a serum-free basal medium comprising effective amounts of (a) bone morphogenetic protein 4 (BMP4), (b) Activin A (ActA), (c) a GSK-3 inhibitor, (d) basic fibroblast growth factor (FGF2), (e) glutamine, and (f) a serum-free supplement comprising albumin, transferrin, ethanol amine, selenium or a bioavailable salt thereof, L-carnitine, fatty acid supplement, and triodo-L-thyronine (T3), wherein said amounts result in the expression of CD90 in at least 90% of cells obtained by step (i*), the expression of CD73 in at most 10% of cells obtained by step (i*), and the expression of CD44 in at most 10% of the cells obtained by step (i*) determined by flow cytometry;
   i**. Inducing epicardial differentiation by culturing the cells of step (i*) under suitable conditions on laminin coated substrates in a serum-free basal medium comprising effective amounts of (a) BMP4, (b) retinoic acid (RA), (c) a GSK-3 inhibitor, (d) insulin, (e) glutamine and (f) the serum-free supplement as in (i); wherein said amounts result in the expression of Wilms tumor antigen 1 (WT-1), as determined by fluorescent microscopy;
      i. Inducing epithelial-mesenchymal transition by culturing the cells of step (i**) under suitable conditions in the presence of an first extracellular matrix protein in a serum-free basal medium comprising effective amounts of (a) FGF2, (b) vascular endothelial growth factor (VEGF), (c) glutamine and (d) a GSK-3 inhibitor, wherein said amounts result in the expression of CD90 in at least of the cells obtained by step (i) a1), the expression of CD73 in at most 50% of the cells obtained by step (i) a1), and the expression of CD44 in at most 30% of the cells obtained by step (i) a1); followed by
         a2) culturing the cells under suitable conditions in the presence of a second extracellular matrix protein in a serum-free basal medium comprising effective amounts of (a) FGF2, (b) VEGF, and (c) glutamine; wherein said amounts result in the expression of CD90, CD73, and CD44 in at least 80 % of the obtained population of cardiac stromal cells; and
      ii. Amplifying the number of said cardiac stromal cells by culturing said population of cardiac stromal cells of step (i) in the presence of at least one third extracellular matrix protein in a serum-free basal medium comprising effective amounts of (a) FGF2, (b) VEGF, and (c) glutamine, wherein said amounts result in the maintained expression of CD90, CD73, and CD44 in at least 80 % of said cardiac stromal cell population;
101. The method of any of the preceding embodiments 89 to 100, wherein at least 50% of the obtained cells after step (i*) express CD309 as determined by flow cytometry, preferably wherein at least 55% the cells after step (i) of embodiment 1 express CD309, more preferably wherein at least 60% of the cells after step (i) of embodiment 1 express CD309, even more preferably wherein at least 65% of the cells after step (i) of embodiment 1 express CD309, as determined by flow cytometry.
102. The method of any of embodiments 89 to 101, wherein at least 50% of the obtained cells after step (i*) express CD140a as determined by flow cytometry, preferably wherein at least 55% the cells after step (i*) express CD140a, more preferably wherein at least 60% of the cells after step (i*) express CD140a, even more preferably wherein at least 65% of the cells after step (i*) express CD140a.
103. The method of any of the preceding embodiments 89 to 102, wherein the obtained cells after step (i**) express Wilms tumor antigen 1 (WT-1) RNA at least 2-fold more than TBP (TATA-binding protein), preferably 3-fold, more preferably 4-fold, even more preferably at least 5-fold, most preferably at least 6-fold; and at most 15-fold, as determined by qRT-PCR.
104. The method of any of embodiments 89 to 103, wherein the first extracellular matrix protein is laminin and wherein the laminin coated substrates of step (i*), (i**), and/or (i a1) are coated with a final concentration of 0.1125 µg/cm² - 7.2 µg/cm² laminin, preferably 0.225 µg/cm² - 3.6 µg/cm² laminin, more preferably 0.45 µg/cm² - 1.8 µg/cm² laminin, even more preferably 0.6 µg/cm² - 1.2 µg/cm² laminin, even more preferably 0.7 µg/cm² - 1.1 µg/cm² laminin, even more preferably 0.8 µg/cm² - 1 µg/cm² laminin, most preferably 0.9 µg/cm² laminin.
105. The method of embodiment 104, wherein the substrate is a plate.
106. The method of any of embodiments 89 to 105, wherein the laminin is a combination of an alpha-chain, a beta-chain, and a gamma-chain.
107. The method of embodiment 106, wherein the alpha-chain, the beta-chain, and the gamma-chain are independently selected, preferably wherein the alpha-chain is selected from alpha-1, alpha-2, alpha-3, alpha-4, alpha-5, the beta-chain is selected from beta-1, beta-2, and beta-3, and the gamma chain is selected from gamma-1, gamma-2, and gamma-3, most preferably wherein the laminin is a combination of the alpha-5 chain, the beta-2 chain and the gamma-1 chain.
108. The method of any of embodiments 89 to 107, wherein the laminin is dissolved in Dulbecco's phosphate-buffered saline (DPBS).
109. The method of any of embodiments 89 to 108, wherein step (i*) is carried out for 3-9 days, preferably wherein step (i*) is carried out for 4-8 days, more preferably wherein step (i*) is carried out for 5-7 days, most preferably wherein step (i*) is carried out for around 6 days.
110. The method of any of embodiments 89 to 109, wherein the basal medium of step (i*) further comprises 10-1000 µM, preferably 50-400 µM, more preferably 100-300 µM, even more preferably 150-250 µM, and most preferably about 200 µM of ascorbic acid or a derivative thereof.
111. The method of embodiment 110, wherein the derivative of ascorbic acid is ascorbate-2-phosphate.
112. The method of any of embodiments 89 to 111, wherein the basal medium used in step (i*) is RPMI, DMEM, aMEM, DMEM/F12, StemPro, and Iscove's medium, most preferably wherein the basal medium is RPMI.
113. The method of embodiment 112, wherein the basal medium used in step (i*) is RPMI comprising pyruvate.
114. The method of embodiment 112, wherein the basal medium used in step (i*) is RPMI comprising 0.1-10 mM pyruvate, preferably 0.2-5 mM pyruvate, more preferably 0.4-2.5 mM pyruvate, more preferably 0.8-1.5 mM pyruvate, more preferably 0.9-1.2 mM pyruvate, most preferably about 1 mM pyruvate.
115. The method of any of embodiments 89 to 114, wherein the basal medium in step (i*) comprises a final concentration of 1-100 ng/ml BMP4, preferably 2-50 ng/ml, more preferably 4-25 ng/ml, even more preferably 7-15 ng/ml, even more preferably 8-12.5 ng/ml, even more preferably 9-11 ng/ml, and most preferably about 10 ng/ml.
116. The method of any of embodiments 89 to 115, wherein the basal medium in step (i*) comprises a final concentration of 0.3-30 ng/ml Activin A, preferably 0.5-15 ng/ml, more preferably 0.8-7 ng/ml, even more preferably 1-5 ng/ml, still more preferably 2-4 ng/ml, most preferably 2.5-3.5 ng/ml, and most preferably about 3 ng/ml.
117. The method of any of embodiments 89 to 116, wherein the GSK-3 inhibitor of step (i*) is selected from a group consisting of CHIR99021, CHIR98014, SB216763, TWS119, Tideglusib, SB415286, 6-bromoindurubin-3-oxime and a valproate salt, preferably wherein the GSK-3 inhibitor is CHIR99021.
118. The method of embodiment 117, wherein the GSK3-inhibitor in the basal medium of step (i*) is CHIR99021.
119. The method of embodiment 118, wherein the basal medium in step (i*) comprises a final concentration of 0.1-10 µM CHIR99021, preferably 0.2-9 µM, more preferably 0.3-8 µM, even more preferably 0.4-7 µM, still more preferably 0.5-6 µM, more preferably 0.6-5 µM, more preferably 0.7-4 µM, more preferably 0.8-3 µM, most preferably 0.9-2 µM, and even most preferably about 1 µM CHIR99021.
120. The method of any of embodiments 89 to 119, wherein the basal medium in step (i*) comprises a final concentration in the basal medium of 0.1-10 ng/ml FGF2, preferably 1-9 ng/ml, more preferably 2-8 ng/ml, even more preferably 3-7 ng/ml, most preferably 4-6 ng/ml, and even most preferably about 5 ng/ml.
121. The method of any of embodiments 89 to 120, wherein the basal medium used in step (i*) comprises 0.2-20 mM, more preferably 0.4-10 mM glutamine, more preferably 0.5-5 mM glutamine, even more preferably 1-3 mM glutamine, even more preferably 1.8-2.2 mM glutamine, even preferably about 2 mM glutamine, most preferably wherein the glutamine is present as a L-alanyl-L-glutamine dipeptide.
122. The method of any of embodiments 89 to 121, wherein the basal medium of step (i*) further comprises 10-1000 µM, preferably 50-400 µM, more preferably 100-300 µM, even more preferably 150-250 µM, and most preferably about 200 µM of ascorbic acid or a derivative thereof.
123. The method of any of embodiments 89 to 122, wherein the serum-free supplement in step (i*) is formulated to provide a final concentration in the basal medium of 0.25-25 mg/ml albumin, 0.5-50 µg/ml transferrin, 0.05-5 µg/ml ethanolamine, 7.2-723 nM selenium or a bioavailable salt thereof, 0.2-20 µg/ml L-carnitine, 0.25-25 µg/ml fatty acid supplement, and 0.00005-0.05 µg/ml triodo-L-thyronine (T3).
124. The method of any of embodiments 89 to 123, wherein the serum-free supplement in the basal medium of step (i*) is provided by 0.1-10 % (v/v) B27 minus insulin as specified in **Table 1a**, preferably 0.5-8 % (v/v), more preferably 1-6 % (v/v), even more preferably 1.5-4% (v/v), and even more preferably about 2% (v/v) B27 minus insulin.
125. The method of any of embodiments 89 to 124, wherein step (i**) is carried out for 5-9 days, preferably wherein step (i**) is carried out for 6-8 days, more preferably wherein step (i**) is carried out for 6.5-7.5 days, most preferably wherein step (i**) is carried out for around 7 days.
126. The method of any of embodiments 89 to 125, wherein the basal medium of step (i**) further comprises 10-1000 µM, preferably 50-400 µM, more preferably 100-300 µM, even more preferably 150-250 µM, even more preferably about 200 µM of ascorbic acid or a derivative thereof, and most preferably wherein the derivative of ascorbic acid is ascorbate-2-phosphate.
127. The method of any of embodiments 89 to 126, wherein the basal medium used in step (i**) is RPMI, DMEM, aMEM, DMEM/F12, StemPro, and Iscove's medium, most preferably wherein the basal medium is RPMI.
128. The method of embodiment 127, wherein the basal medium used in step (i**) is RPMI comprising pyruvate.
129. The method of embodiment 128, wherein the basal medium used in step (i**) is RPMI comprising 0.1-10 mM pyruvate, preferably 0.2-5 mM pyruvate, more preferably 0.4-2.5 mM pyruvate, more preferably 0.8-1.5 mM pyruvate, more preferably 0.9-1.2 mM pyruvate, most preferably about 1 mM pyruvate.
130. The method of any of embodiments 89 to 129, wherein the basal medium in step (i**) comprises a final concentration of 5-500 ng/ml BMP4, preferably 10-250 ng/ml BMP4, more preferably 20-125 ng/ml BMP4, even more preferably 35-70 ng/ml BMP4, even more preferably 40-60 ng/ml BMP4, even more preferably 45-55 ng/ml BMP4, and most preferably about 50 ng/ml BMP4.
131. The method of any of embodiments 89 to 130, wherein the basal medium in step (i**) comprises a final concentration of 0.4-40 µM retinoic acid (RA), preferably 0.8-20 µM RA, more preferably 1.6-10 µM RA, even more preferably 2-8 µM RA, still more preferably 2.5-7 µM RA, even more preferably 2.8-6 µM RA, even more preferably 3-5 µM RA, most preferably 3.5-4.5 µM RA, and even most preferably about 4 µM RA.
132. The method of any of embodiments 89 to 131, wherein the GSK-3 inhibitor of step (i**) is selected from a group consisting of CHIR99021, CHIR98014, SB216763, TWS119, Tideglusib, SB415286, 6-bromoindurubin-3-oxime and a valproate salt, preferably wherein the GSK-3 inhibitor is CHIR99021.
133. The method of embodiment 132, wherein the GSK3-inhibitor in the basal medium of step (i**) is CHIR99021.
134. The method of embodiment 133, wherein the basal medium in step (i**) comprises a final concentration in the basal medium of 0.1-10 µM CHIR99021, preferably 0.2-9 µM, more preferably 0.3-8 µM, even more preferably 0.4-7 µM, still more preferably 0.5-6 µM, more preferably 0.6-5 µM, more preferably 0.7-4 µM, more preferably 0.8-3 µM, most preferably 0.9-2 µM, and most preferably about 1 µM CHIR99021.
135. The method of any of embodiments 89 to 134, wherein the basal medium in step (i**) comprises a final concentration of 0.3-30 µg/ml insulin, preferably 0.5-20 µg/ml, more preferably 1-15 µg/ml, even more preferably 1.5-10 µg/ml, even more preferred 2-5 µg/ml, even more preferably 2.5-3.5 µg/ml, most preferably about 3 µg/ml insulin.
136. The method of any of embodiments 89 to 135, wherein the basal medium used in step (i**) comprises 0.2-20 mM glutamine, more preferably 0.4-10 mM, more preferably 0.5-5 mM, more preferably 1-3 mM, more preferably 1.8-2.2 mM, even preferably about 2 mM glutamine, most preferably wherein the glutamine is present as a L-alanyl-L-glutamine dipeptide.
137. The method of any of embodiments 89 to 136, wherein the serum-free supplement in step (i**) is formulated to provide a final concentration in the basal medium of 0.25-25 mg/ml albumin, 0.5-50 µg/ml transferrin, 0.05-5 µg/ml ethanolamine, 7.2-723 nM selenium or a bioavailable salt thereof, in particular sodium selenite, 0.2-20 µg/ml L-carnitine, 0.25-25 µg/ml fatty acid supplement, and 0.00005-0.05 µg/ml triodo-L-thyronine (T3).
138. The method of any of embodiments 89 to 137, wherein the serum-free supplement and the insulin in the basal of step (i**) are provided by B27 as specified in **Table 1b**, preferably wherein the serum-free supplement and the insulin are provided by 0.1-10% (v/v) B27 medium, more preferably 0.5-8% (v/v) B27, more preferably 1-6% B27 (v/v), even more preferably 1.5-4% (v/v) B27, even more preferably about 2% (v/v) B27.
139. The method of any of embodiments 89 to 138, wherein in step (i a1), (i a2) and (ii) the basal medium comprises a final concentration of 0.2-20 mM glutamine, preferably, 0.5-10 mM glutamine, more preferably 0.75-5 mM glutamine, more preferably 1-3 mM glutamine, more preferably 1.5-2.5 mM glutamine, and most preferably about 2 mM glutamine, most preferably wherein the glutamine is present as a L-alanyl-L-glutamine dipeptide.
140. The method of any of embodiments 91 to 139, wherein step (i a1 and i a2) is carried out for 14-28 days, preferably wherein step (i a1 and i a2) is carried out for 15-25 days, more preferably wherein step (i a1 and i a2) is carried out for 16-23 days, most preferably wherein step (i a1 and i a2) is carried out for 17-21 days.
141. The method of any of embodiments 89 to 140, wherein the substrate is a plate or beads, preferably wherein the substrate is a plate.
142. The method of any of the preceding embodiments, comprising prior to step (i*) a seeding step, wherein said pluripotent stem cells are seeded onto laminin coated substrate.
143. The method of embodiment 142, wherein the pluripotent stem cells are seeded onto laminin coated plates.
144. The method of embodiments 142 or 143, wherein the medium used in the seeding step further comprises a ROCK-inhibitor.
145. The method of embodiment 144, wherein the ROCK inhibitor is selected from Y27632, H-1152P, Thiazovivin, Fasudil, Hydroxyfasudil, GSK429286A, and RKI-1447, preferably selected from Y27632, H-1152P, Thiazovivin, Fasudil, Hydroxyfasudil, and more preferably the ROCK inhibitor is Y27632 or H-1152P,
146. The method of embodiment 145, wherein the ROCK inhibitor is Y27632.
147. The method of embodiment 148, wherein the medium used in the seeding step comprises 1-50 µM, preferably 2.5-40 µM, more preferably 5-30 µM, even more preferably 7.5-20 µM, most preferably 8-12 µM, and most preferably about 10 µM Y27632.
148. The method of any of embodiment 142-147, wherein the seeding step is carried out 24-120 hours, more preferably 48-108 hours, most preferably 96 hours prior to step (i*).
149. An isolated population of cardiac stromal cells, wherein the cardiac stromal cells have been obtained by differentiation of pluripotent stem cells and wherein at least about 80 % of the cells of the population of cardiac stromal cells express CD90, CD73, and CD44.
150. The isolated population of cardiac stromal cells of embodiment 149, wherein at least 80% of the population of cardiac stromal cells obtained by step (ii) express CD44, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD44, as determined by flow cytometry.
151. The isolated population of cardiac stromal cells of embodiments 149 or 150, wherein at least about 90 % of the cells of the population of cardiac stromal cells express CD44.
152. The isolated population of cardiac stromal cells of any of embodiments 149 to 151, wherein at least 80% of the population of cardiac stromal cells obtained by step (ii) express CD90, preferably at least 81%, more preferably at least 82%, even more preferably at least 83%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD90, as determined by flow cytometry.
153. The isolated population of cardiac stromal cells of any of embodiments 149 to 152, wherein at least about 91%, about 92 %, about 93%, about 94 %, about 95%, about 96 %, about 97 %, about 98%, or about 99 % of the cells of the population of the cardiac stromal cells express CD90.
154. The isolated population of cardiac stromal cells of any of embodiments 149 to 153, wherein at least 80% of the population of cardiac stromal cells obtained by step (ii) express CD73, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% CD73, as determined by flow cytometry.
155. The isolated population of cardiac stromal cells of any of embodiments 149 to 154, wherein at least about 91%, about 92 %, about 93%, about 94 %, about 95%, about 96 %, about 97 %, about 98%, or about 99 %of the cells of the population of the cardiac stromal cells express CD73.
156. The isolated population of cardiac stromal cells of any of embodiments 149 to 155, wherein about 91%, about 92 %, about 93%, about 94 %, about 95%, about 96 %, about 97 %, about 98%, or about 99 % of the cells of the population of the cardiac stromal cells express CD44.
157. The isolated population of cardiac stromal cells of any of embodiments 149 to 156, wherein at least about 90 %, about 91 %, about 92 %, about 93%, about 94 %, about 95%, about 96 %, about 97 %, about 98%, or about 99% of the cells of said population of cardiac stromal cells express vimentin.
158. The isolated population of cardiac stromal cells of any of embodiments 149 to 157, wherein at least 80% of the population of cardiac stromal cells obtained by step (i) a2) express vimentin, preferably at least 81%, more preferably at least 82%, even more preferably at least 83%, even more preferably at least 84%, even more preferably at least 85%, even more preferably at least 86%, even more preferably at least 87%, even more preferably at least 88%, even more preferably at least 89%, and most preferably at least 90% vimentin, as determined by flow cytometry.
159. The isolated population of cardiac stromal cells of any of embodiments 149 to 158, wherein at least 80% of the population of cardiac stromal cells obtained by step (i) a2) express vimentin, preferably at least 91%, more preferably at least 92%, even more preferably at least 93%, even more preferably at least 94%, even more preferably at least 95%, even more preferably at least 96%, even more preferably at least 97%, even more preferably at least 98% vimentin, as determined by flow cytometry.
160. The isolated population of cardiac stromal cells of any of embodiments 149 to 159, wherein at least about 90 %, about 91 %, about 92 %, about 93%, about 94 %, about 95%, about 96 %, about 97 %, about 98%, or about 99% of the cells of said population of cardiac stromal cells express collagen-1.
161. The isolated population of cardiac stromal cells of any of embodiments 149 to 160, wherein at least 80% of the population of cardiac stromal cells obtained by step (i) a2) express collagen 1, preferably at least 82%, more preferably at least 82%, even more preferably at least 84%, even more preferably at least 86%, even more preferably at least 88%, even more preferably at least 90%, even more preferably at least 91%, even more preferably at least 92%, even more preferably at least 93%, even more preferably at least 94%, even more preferably at least 95%, even more preferably at least 96%, and most preferably at least 97% collagen 1, as determined by flow cytometry.
162. The isolated population of cardiac stromal cells of any of embodiments 149 to 161, wherein about 95% or more of the cells of the population are CD90 positive; about 99% or more of the cells are CD44 positive; about 99% or more of the cells are CD73 positive; about 97% or more of the cells are collagen 1 positive; and about 98% or more of the cells are vimentin positive.
163. The isolated population of cardiac stromal cells of any of embodiments 149 to 162, wherein the population is obtainable by the method as defined in any of embodiments 1 to 151.
164. The isolated population of cardiac stromal cells of any of embodiments 149 to 163, wherein the population is obtained by the method as defined in any of embodiments 1 to 151.
165. A pharmaceutical composition comprising an isolated population of cardiac stromal cells as defined in any of embodiments 149 to 164.
166. The pharmaceutical composition of embodiment 165, further comprising a pharmaceutically acceptable excipient.
167. An engineered organ tissue comprising a population of cardiac stromal cells as defined in any of embodiments 149 to 164 or as obtained by a method according to embodiment 1-148.
168. The engineered organ tissue of embodiment 167, wherein the tissue is human engineered organ tissue, preferably wherein the engineered human organ tissue is engineered human myocardium.
169. Use of the population of cardiac stromal cells (cStC) obtained by the method according to any of embodiments 1-148 or as defined in any of embodiments 149-164 in an *in vitro* model for drug screening.
170. Use of the population of cardiac stromal cells (cStC) obtained by the method according to any of embodiments 1-148 or as defined in any of embodiments 149-164 in an *in vitro* model for drug efficacy screening.
171. Use of the population of cardiac stromal cells (cStC) obtained by the method according to any of embodiments 1-148 or as defined in any of embodiments 149-164 in an *in vitro* model for drug toxicity screening.
172. Use of the population of cardiac stromal cells (cStC) obtained by the method according to any of embodiments 1-148 or as defined in any of embodiments 149-164 in an *in vitro* production of an engineered organ tissue.
173. The use of embodiment 172, wherein the engineered organ tissue is human engineered organ tissue, preferably wherein the engineered human tissue is engineered human myocardium.
174. The use of embodiment 172, wherein the engineered organ tissue is human engineered organ tissue, preferably wherein the engineered organ human tissue is engineered human connective tissue.
175. Use of the population of cardiac stromal cells (cStC) obtained by the method according to any of embodiments 1-151 or as defined in any of embodiments 152-167 as a research tool.
176. The population of cardiac stromal cells (cStC) obtained by the method according to any of embodiments 1-148 or as defined in any of embodiments 149-164 for use in medicine.
177. The population of cardiac stromal cells (cStC) obtained by the method according to any of embodiment 1-148 or as defined in any of embodiments 149-164 for use in organ repair, preferably heart repair or soft tissue repair, more preferably heart repair.
178. Cardiac stromal cells (cStC) produced by the method according to any of embodiment 1-148.
179. The cardiac stromal cells (cStC) of embodiment 178, wherein at least 80% of the cStC express CD90, CD44, CD73, Collagen 1 and/or Vimentin, as determined by flow cytometry, preferably 85%, more preferably 90%, even more preferably 92%, even more preferably 94%, most preferably 95%.
180. A serum-free cell culture medium suitable for amplification of a cardiac stromal cell comprising (a) a serum-free basal medium, (b) 10-200 ng/ml FGF2, (c) 5-100 ng/ml VEGF, (d) 0.2-20 mM glutamine, and (e) a ECCM supplement comprising 1.5-180 µg/ml ascorbic acid, 2-50 µg/ml insulin, 1.1-27.5 µg/ml transferrin, 1660-41500 µg/ml albumin, and 11-145 nM selenium.
181. The cell culture medium of embodiment 180, wherein the basal medium is KO DMEM, DMEM, DMEM/F12, RPMI, IMDM, alphaMEM, medium 199, Hams F-10, or Hams F-12, preferably wherein the basal medium is KO DMEM.
182. The cell culture medium of embodiments 180 or 181, wherein the cell culture medium comprises a final concentration of 15-100 ng/ml FGF2, preferably 20-80 ng/ml, even more preferably 30-70 ng/ml, most preferably 40-60 ng/ml, and most preferably about 50 ng/ml.
183. The cell culture medium of any of embodiments 180 to 182, wherein the culture medium comprises a final concentration of 7-50 ng/ml VEGF, preferably 10-40 ng/ml, even more preferably 15-35 ng/ml, most preferably 20-30 ng/ml, and most preferably about 25 ng/ml VEGF.
184. The cell culture medium of any of embodiments 180 to 183, wherein the ECCM supplement is formulated to provide a final concentration in the basal medium of 10-80 µg/ml ascorbic acid, 4-40 µg/ml insulin, 2-20 µg/ml transferrin, 3200-30000 µg/ml albumin, and 11-145 nM selenium.
185. The cell culture medium of any of embodiments 180 to 184, wherein the ECCM supplement is formulated to provide a final concentration in the basal medium of 15-60 µg/ml ascorbic acid, 5-30 µg/ml insulin, 3-12.5 µg/ml transferrin, 4800-20000 µg/ml albumin, and 20-105 nM selenium.
186. The cell culture medium of any of embodiments 180 to 185, wherein the ECCM supplement is formulated to provide a final concentration in the basal medium of 27-40 µg/ml ascorbic acid, 7-15 µg/ml insulin, 4.5-7µg/ml transferrin, 7500-9200 µg/ml albumin, and 30-50 nM selenium.
187. The cell culture medium of any of embodiments 180 to 186, wherein the ECCM supplement is formulated to provide a final concentration in the basal medium of 30-36 µg/ml ascorbic acid, 9-11 µg/ml insulin, 5-6 µg/ml transferrin, 8000-8600 µg/ml albumin, and 35-45 nM selenium.
188. The cell culture medium of any of embodiments 180 to 187, wherein the ECCM supplement is formulated to provide a final concentration in the basal medium of about 33 µg/ml ascorbic acid, about 10 µg/ml insulin, about 5.5 µg/ml transferrin, about 8300 µg/ml albumin, and about 40.5 nM selenium.
189. The cell culture medium of any of embodiments 180 to 188, wherein the ECCM supplement further comprises a suitable concentration of selenium or a bioavailable salt thereof, glutathione, and trace elements.
190. The cell culture medium of any of embodiments 180 to 189, wherein the ECCM comprises all substances listed in **Table 2,** preferably wherein the culture medium comprises 5-20% (v/v) ECCM in step (i) a2) and/or (ii) as specified in **Table 2**, more preferably 6-17.5% (v/v), more preferably 7-15% (v/v), more preferably 8%-12% (v/v), more preferably 9%-11% (v/v), and most preferably about 10% (v/v) ECCM.
191. The serum-free culture medium of any of embodiments 180 to 190, wherein the ECCM is provided as defined in any of embodiments 26 to 29.
192. The cell culture medium of any of embodiments 180 to 191, wherein the culture medium comprises a final concentration of 0.2-20 mM glutamine, preferably, 0.5-10 mM glutamine, more preferably 0.75-5 mM glutamine, more preferably 1-3 mM glutamine, more preferably 1.5-2.5 mM glutamine, and most preferably about 2 mM glutamine, most preferably wherein the glutamine is present as a L-alanyl-L-glutamine dipeptide.
193. A serum free basal medium, wherein the medium is Knockout DMEM medium comprising about 2 mM glutamine, about 50 ng/ml FGF, about 25 ng/ml VEGF and about 1 µM CHIR, and a ECCM supplement comprising 6.6-165 µg/ml ascorbic acid, 2-50 µg/ml insulin, 1.1-27.5 µg/ml transferrin, 1660-41500 µg/ml albumin, and 11-145 nM selenium.
194. The serum free basal medium of embodiment 193, wherein the ECCM is provided as defined in any of embodiments 26 to 29.
195. A serum free basal medium, wherein the serum free basal medium is Knockout DMEM medium comprising about 2 mM glutamine, about 50 ng/ml FGF, about 25 ng/ml VEGF, and a ECCM supplement comprising 6.6-165 µg/ml ascorbic acid, 2-50 µg/ml insulin, 1.1-27.5 µg/ml transferrin, 1660-41500 µg/ml albumin, and 11-145 nM selenium.
196. The serum free basal medium of embodiment 195, wherein the ECCM is provided as defined in any of embodiments 26 to 29.

### Description of the Figures

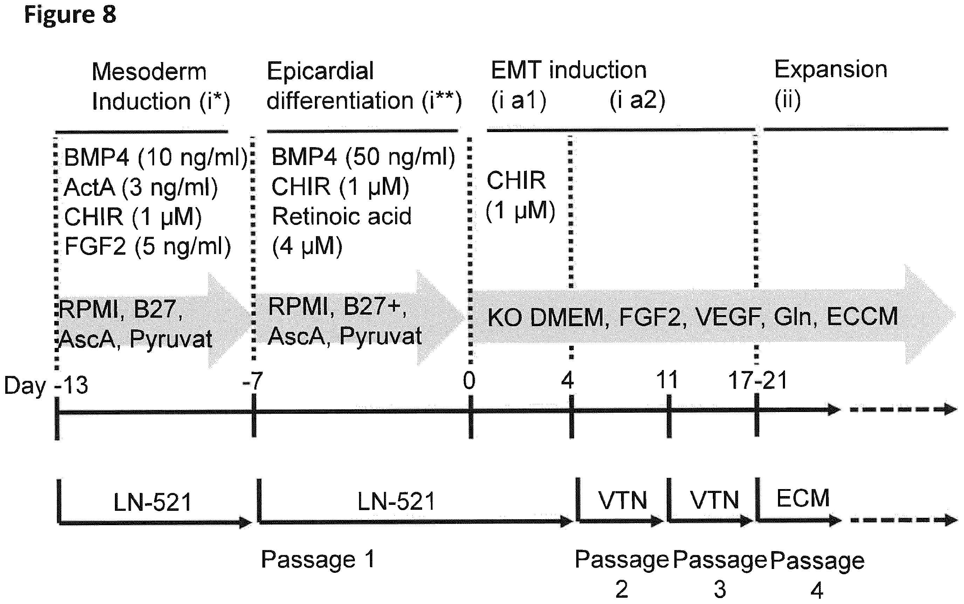

**Figure 1****:**
   **(A) Scalable stromal cell production with stable phenotype.** Overview and chronological sequence of the differentiation/amplification method starting with epicardial cells and resulting in cardiac stromal cells. In step (i) a1), the epithelial-mesenchymal transition (EMT) was induced by culturing the epicardial cells in the presence of a first extracellular matrix protein (for example laminin) in a serum-free basal medium (for example KO DMEM) comprising (a) FGF, (b) VEGF, (c) Glutamin (Gln) and a GSK-3 inhibitor (for example 1µM CHIR). This step took between 2-8 days, preferably 4 days. After completion of said step at least 50% of the cells expressed CD90, at most 50% of the cells expressed CD73 and at most 30% of the cells expressed CD44. Said step was followed by step (i) a2), wherein the cells were passaged and further cultivated in the presence of a second extracellular matrix protein (such as vitronectin) in a basal medium (such as KO DMEM) comprising (a) FGF, (b) VEGF, (c) Glutamin (Gln). Said step took between 10-20 days, preferably 13 days, and the cells were usually passaged once after the completion of 5-10 days. After completion of step (i) a2), the cells were differentiated into cardiac stromal cells so that at least 80% of the cells expressed CD90, CD73 and CD44. Step (ii) amplified the cardiac stromal cells under serum-free conditions. Specifically, the number of cardiac stromal cells was amplified by culturing the cells in the presence of at least a third extracellular matrix protein (such as vitronectin) in a serum-free basal medium (KO DMEM) comprising (a) FGF, (b) VEGF, (c) Glutamin (Gln). Cardiac stromal cells can be amplified in said medium repeatedly and the cardiac stromal cells show a maintained expression of CD90, CD73 and CD44 in at least 80% of the cardiac stromal cell.
   **(B)** Transillumination of human undifferentiated induced pluripotent stem cells on day -14 before the differentiation of human pluripotent stem cells into epicardial cells; day 0 of the method (stage of epicardial cells); and day 17 of the method (stage of cardiac stromal cells); scale 100 µm (day -14) and 20 µm (day 0 and day 17), respectively.
   **(C)** Immunofluorescence images of the differentiation and amplification protocol on day 0 (before step (i) a1) of the method). The left panel showed the expression of F-Actin, the middle panel showed the expression of Wilms tumor antigen 1 (WT1), and in the right panel the nuclei were stained.
**Figure 2****: Scaling of the differentiation protocol and stromal cell amplification.**
   **(A)** Exemplary procedure (refer to Figure 8 for additional details) of iPSC differentiation to cardiac stromal cells (obtained at passage 4 by completion of protocol steps (i*,i**, and i) and the amplification of cardiac stromal cells from passage 4 to passage 9 (i.e., 5 passages) following method step (ii). For example, the method start was with the seeding of ∼1x10E6 iPSC per one T75 culture flask (growth surface area: 75 cm²) 96 h before the start of mesoderm induction (step (i*) for 6 days). After passaging of obtained cells onto six T225 culture flasks (growth surface area: 225 cm² per T225 flask) on culture day -7, formation of (pro)epicardium-like cells was induced for 7 days until culture day 0 (step (i**)), followed by induction of EMT (step (i)). Step (i) comprised of two sub-steps: step (i) a1) until culture day 4 followed by step (i) a2) with a 1:2 passage of cells obtained from step (i)a1) onto twelve T225 culture flasks on culture day 4 and subsequent 1:1 passage onto another twelve T225 culture flasks on culture day 11. Step (i) a2) was completed on culture day 17-21 with a 1:2 passage of the obtained cardiac stromal cells onto twenty-four T225 culture flasks. The following amplification step (ii) comprised a passaging every seven days (weekly), with an average population doubling every 2.1 days, i.e., a 3.3-fold amplification per passage. Average output for cardiac stromal cells was 1x10E5 cells per cm² after passage 4; reseeding was performed with 3x10E4 cells per cm². Note that for practical reasons, i.e., for example limited plate handling capacity and a defined cardiac stromal cell quantity required, fewer than the maximal plate numbers are typically used in a manual cell amplification process. Surplus cardiac stromal cells can be cryo-preserved using standard protocols at the end of each passage without compromising the cardiac stromal cell phenotype. Depending on the cryo-preservation protocol used, there may be differences in cardiac stromal cell retrieval. A retrieval is typically 60-80% of the cryo-preserved cardiac stromal cell quantity.
   **(B)** Data on population doubling level obtained during stroma cell amplification (step (ii)) under serum-free conditions. At an average population doubling level between passages of 3.3, according to an average doubling time of 2.1±0.4 days and considering an effective transfer of ∼90% of the cells from passage to passage a 3-fold increase of cardiac stromal cell yield per passage can be anticipated and is considered in the calculation of cardiac stromal cell amplification growth area for 5 passages in panel (A); the effective cell doubling over 5 passages was 15-times. 15 passages with a constant cardiac stromal cell phenotype (such as demonstrated in Figure 3D) would e.g. allow a 50-fold increase of cardiac stromal cell number.
**Figure 3****: Monitoring the phenotype during differentiation and amplification of cardiac stromal cells and precursors thereof.**
   **(A)** Characterization of cardiac stromal cells by flow cytometry. Expression of markers (Collagen 1, Vimentin, CD90, CD73, and CD44) after 17 days in culture according to steps (i) a1 and (i) a2) (and optionally steps (i)* and (i**) as set out in Figure 8) of the disclosed method. 95% of the cells were CD90 positive; 99% of the cells were CD44 positive; 99% of the cells were CD73 positive; 97% of the cells were collagen-1 positive; 98% of the cells were vimentin positive. The negative control showed only 3% background signal.
   **(B)** Characterization of cardiac stromal cells by immunofluorescence. Expression of Vimentin, anti-human fibroblast antibody (clone TE-7, Millipore), and collagen-1 (Abcam) in the top panel. The corresponding nuclei labelling is presented in the bottom panel. Bars: 50 µm.
   **(C)** Expression of surface markers CD90, CD73, and CD44 as well as the intermediate filament VIM (vimentin) at indicated steps of the differentiation protocol.
      After completion of step (i*) (culture day -7; please refer to Figure 8) more than 90% of the cells expressed CD90, less than 10% of the cells expressed CD73, less than 10% expressed CD44 and more than 40% expressed vimentin.
      After completion of sub-step (i a1 - culture day 4) more than 50% of the cells expressed CD90; less than 50% of the cells expressed CD73; less than 30% of the cells expressed CD44, and more than 80% of the cells expressed vimentin.
      On culture day 17 (during step (i) a2)) the markers CD90, CD73, and vimentin were expressed in more than 90% of the cells, the marker CD44 was expressed in more than 80% of the cells.
      After completion of step (ia2 - culture day 17) the markers CD90, CD73, CD44, and vimentin were expressed in more than 90% of the cells.
   **(D)** Expression of surface markers CD90, CD73, and CD44 were stable in >90% of the cells during step (ii), i.e., amplification. Data was obtained at passage 9, i.e., 5 passages under the amplification step (ii), demonstrating the stability of the cardiac stromal cell phenotype after extensive passaging manifested in maintained expression (for comparison, data shown at passage 4 in Figure 3A and 3C; Data for Figure 3A was obtained from independent experiments to the data obtained in Figures 3C and 3D.
**Figure 4****: Expression profiles of different cell populations.**
   **(A)** Gene expression assessed by RNA sequencing with transcript abundance displayed as Reads Per Kilobase per Million mapped reads (RPKM) of CD140a, vimentin (VIM) and collagen-1 (COL1A1) in undifferentiated iPSC, iPSC derived cardiomyocytes (CM), iPSC-derived stromal cells, and heart derived primary cardiac fibroblasts (as control). Average RPKM values are indicated in the panels. N=2-5 per group.
   **(B)** Gene expression assessed by RNA sequencing with transcript abundance displayed as Reads Per Kilobase per Million mapped reads (RPKM) of CD90, CD73 and CD44 in undifferentiated iPSC, iPSC derived cardiomyocytes (CM), iPSC-derived stromal cells (StC), and heart derived primary cardiac fibroblasts (as control). Average RPKM values are indicated in the panels. N=2-5 per group.
**Figure 5****: Characterization of cardiac stromal cells by transcriptome analysis.**
   **(A)** The transcriptomes of cardiac stromal cells (cStC, n=2), primary cardiac fibroblasts (n=3), neonatal skin fibroblasts (n=2), and gingival fibroblasts (n=3) were compared. (A) Principal component analysis indicated that 46% of total variance could be attributed to principal component 1.
   **(B)** Detailed analysis of the top 100 variance-causing genes of PC1 as well as characteristic genes from literature indicated that the expression patterns of stromal cells were more comparable to primary cardiac fibroblasts than skin and gingiva fibroblasts. This analysis shows that the expression pattern of primary cardiac fibroblasts and cardiac stromal cells according to the differentiation/amplification method were remarkably similar.
   **(C)** Upper panel: Induction of pro-collagen protein and smooth muscle actin and CTGF by TGFβ31 and/or Angiotensin II. Lower panel: Western Blot analysis showing that profibrotic stimuli induce myofibroblast markers (smooth muscle acting and CTGF) in cardiac stromal cells. Expression of myofibroblast proteins in cardiac stromal cells after incubation with Angiotensin II (Ang II) and/or TGFβ1 at the indicated concentrations for 24 hrs. Stable expression of periostin (POSTN) demonstrates a cardiac fibroblast specific phenotype. Tubulin was used as loading control. Furthermore, cardiac stroma cells under non-stimulated conditions serve as controls (see first and last lane of the Western Blot).
**Figure 6****: Use of cardiac stromal cells in engineered heart myocardium and engineered connective tissue.**
   **(A)** Cardiac stromal cells support generation of functional engineered human myocardium (EHM). Generated (iPSC derived) cardiac stromal cells (iPSC-cStC) and primary cardiac fibroblasts (cardiac fibs; acquired from Lonza) were compared as supplements to EHM preparation according to Tiburcy et al. 2017, i.e., EHM comprised of 70% cardiomyocytes and 30% cardiac fibroblasts or cardiac stromal cells (n=4/group). Force of contraction (FOC) of EHM was assessed under isometric conditions, at extracellular calcium concentration of 2 mmol/L, and under electrical field stimulation at 1.5 Hz using standard protocols (Tiburcy et al. 2017). Mean ± Standard Error of the mean is indicated in the columns.
   **(B)** Stiffness of engineered connective tissue (ECT) made from primary cardiac fibroblasts or cardiac stromal cells. ECT were treated with 5 ng/ml TGFb1 for 5 days. n=3 per group, *p>0,05 by 1-way ANOVA and Tukey's post-hoc test. Stiffness was measured by destructive tensile stress test using an RSA-G2 rheometer (TA Instruments) and calculated from the slope of the stress-strain curve as Young's modulus (E; Santos et al. 2019). Mean ± Standard Error of the mean is indicated in the columns.
**Figure 7****: Amplification step (ii) requires an extracellular matrix protein.**
   The cells were cultivated for passage 5 and passage 6 (during the amplification step (ii)) on either vitronectin, laminin (LN-521) gelatin, fibronectin, Matrigel or on uncoated plates. Growth was detected for all coatings, but no growth was detected on uncoated plates. The comparison shows that coating with an extracellular matrix protein is essential for cardiac stromal cell amplification. The coating should preferably be by making use of a defined substance, such as but not limited to vitronectin, Laminin-521, gelatin, fibronectin; but also undefined coatings such as Matrigel may be applied. The images were obtained by bright field microscopy. Bars: 50 µm
**Figure 8****: Scalable stromal cell production with stable phenotype.**
   Overview and chronological sequence of an example for the derivation of cardiac stromal cells from a human pluripotent stem cell (hPSC) source. For inducing mesodermal differentiation, step (i*), the cells were cultured on laminin (LN-521) coated plates in RPMI medium, supplemented with B27 without insulin, ascorbic acid and pyruvate. The medium further comprised 10 ng/ml BMP4, 3ng/ml ActA, 1µM CHIR and 5 ng/ml FGF2. This step (i*) took 6 days followed by passaging to the next step (i**). For inducing the epicardial differentiation, step (i**), the cells were cultured on laminin (LN-521) coated plates in RPMI medium, supplemented with B27 with insulin, ascorbic acid, and pyruvate. The medium further comprised 50 ng/ml BMP4, 1 µM CHIR and 4 µM retinoic acid. This step (i**) takes 7 days (days 6-13), without additional passaging into the next step (i a1). For inducing epithelial-mesenchymal transition (EMT) (step i a1), the culture medium was switched to KO DMEM medium comprising FGF2, VEGF, Glutamine and Eukaryotic Cell Culture Medium (ECCM) supplement comprising Ascorbic Acid, Insulin, Transferrin, Albumin, and Selen. An especially preferred ECCM comprises the ingredients as listed in Table 2. This step took in total 17 days, but may have been extended to up to 21 days (days 0-17 or up to 0-21 of the method) and could be subdivided into two sub-steps, taking 4 days (days 0-4 of the method; step i a1) and 13-17 days (days 4-17 or up to 4-21; step i a2), respectively:
   During the first step (i a1), the medium is further comprising 1 µM CHIR. After 4 days (completion of step (i) a1), cells were passaged to continue the now passage 2 culture on vitronectin (VTN)-coated plates without further 1 µM CHIR supplementation to complete the EMT process (step (i) a2). The cells were passaged again onto vitronectin (VTN)-coated plates (passage 3) on day 24 of the method. For amplifying the cell number (amplification step ii), the cells were cultured on vitronectin (VTN)-coated plates in KO DMEM medium comprising ECCM, FGF2, VEGF, and glutamine (Gln); from passage 4 onwards.

### Examples

The following examples are intended to illustrate the invention further, but are not limited to it. The examples describe technical features, and the invention also relates to combinations of the technical features presented in this section.

### Example 1: Generation of cardiac stromal cells in high quantity, high quality and under serum-free conditions

It has been demonstrated that cardiac stromal cells are essential for the function of engineered heart tissue/engineered myocardium. Protocols to successfully obtain engineered heart tissue are known in the art from e.g. Zimmermann et al. (2006) and Tiburcy et al. (2017). However, the generation of engineered heart tissues for clinical use is dependent on the availability of cardiac stromal cells and myocytes, which are mixed during the generation engineered heart muscle. The bottle neck for these protocols is often the provision of a large quantity of cardiac stromal cells. The inventors optimised the cardiac differentiation/amplification method in order to be able to provide not only a large quantity of cardiac stromal cells but also a high quality of cardiac stromal cells. For clinical use, reproducibility of the method is essential. Reproducibility is best achieved if the method is serum-free so that all media components are known. Furthermore, serum-free conditions allow clinical use, as contaminations from undefined sources, such as serum or matrigel, can be excluded. Such a method was sought for a long time. Especially the amplification of cardiac stromal cells posed a particular difficulty to scientists in the field, as it has been unclear how serum supplementation and matrigel coating could be replaced with defined component. Thus, the inventors developed the first serum-free differentiation and amplification method, wherein cardiac stromal cells can be generated from human pluripotent stem cells in high quantity, quality and under serum-free conditions. In this procedure, a specific temporal sequence of active substances (small molecules as well as inhibitors and stimulators) was used, which induce the differentiation of epicardial cells (optionally starting from human pluripotent stem cells) into cardiac stromal cells and allow mass amplification of these cardiac stromal cells in order to obtain large quantities.
The serum-free differentiation/amplification method is depicted in **Figures 1A** **and** **8**. A detailed step-by-step method is provided in the following. Details on stock preparations, medium preparations, storage conditions, and suppliers are listed in the Materials section at the end of Example 1 below. It is also generally noted for all steps that the skilled person is able to determine the amount of medium required for a given size of tissue culture flask. When culturing cells, the cells were always covered with the given medium.
Epicardial cells can be obtained as also disclosed herein or by any other method known in the art. Exemplary protocols in the art to obtain epicardial cells can be found for example in Witty et al. (2014) or as described in Schlick (2018) Doctoral Thesis, November 2018, University of Gottingen. For further assistance, a detailed protocol in order to obtain epicardial cells is also described herein.

When starting from epicardial cells (day 0) the epithelial-mesenchymal transition is induced comprising two sub-steps: (i) a1) and (i) a2). In step (i) a1), the medium was StC-EM+C medium and the cells were on cultivated in laminin coated plates. The medium was replaced with fresh StC-EM+C on day 1 and the cells were incubated with said medium until day 4 and thereby the first sub-step (i) a1) was completed. The second sub-step started on day 4 with passaging the cells onto vitronectin coated plates. Specifically, accutase was warmed to room temperature and versene was warmed to at 37°C. The cells were washed once with PBS (6 ml for a T75 flask), and then incubated with accutase (6 ml for a T75 flask) for 10-20 minutes. Then, versene (6 ml for a T75 flask) was added to the accutase and incubated for 5 min. StC-EM medium (12 ml for a T75 flask) was added to the pool cells and the cells were centrifuged at 300xg for 5 min at room temperature. The cell number was determined and the cells were diluted with StC-EM medium in order to obtain a density of 3x10E6 cells in 15 ml so that the cells can be plated. Media concentrations are described in detail in the materials section below. On days 7 and 9, the medium was replaced by fresh StC-EM medium. Due to cell division, the cells were passaged again on day 11 on vitronectin plates, as just described. On days 14 and 16 the medium was again replaced by fresh StC-EM medium. On day 17, step (i) a2) of the protocol was completed and cardiac stromal cells were generated. These cardiac stromal cells can then be harvested and used for tissue engineering such an engineered human myocardium (EHM) and engineered connective tissue (ECT) or can be frozen. However, in order to obtain a large quantity of cardiac stromal cells, the cells can be amplified further.

For amplifying the cell number of cardiac stromal cells, step (ii), the cells were passaged using accutase and versene as described for step (i)a2) of the protocol on day 17 and cultured in StC-EM medium. The medium was exchanged every second day and further passaging was performed every week during step (ii). At least six passages for cardiac stromal cell amplification can be performed but step (ii) can be principally performed indefinitely. Cardiac stromal cells can also be harvested and used for tissue engineering after every passage and were characterized by the maintained expression of CD90, CD44 and CD73 in at least 90% of the cells.

In order to obtain epicardial cells from pluripotent stem cells, the following protocol can be used: The human pluripotent stem cells were seeded on day -17 on Laminin (LN-521) coated plates and cultivated in the presence of iPS Brew supplemented 10 µM Rock Inhibitor. The cells were plated in order to obtain a confluent layer of cells on day -13. The optimal cell count for seeding must be determined individually for each cell line. The medium was exchanged on day -14 with iPS-Brew medium without Rock inhibitor.

For inducing mesodermal differentiation of the pluripotent stem cells (step (i*)), the cells were washed once with RPMI medium and then further cultivated in StC-IM medium (as described in detail in the materials section below). On days -12, -11, and -10, the medium was replaced by fresh StC-IM medium.

For inducing epicardial differentiation (step (i**)), the cells were passaged on day -7 onto Laminin LMN-521 coated plates. In order to do so, TrypLE was warmed to 37°C, the medium was removed, cells were washed with TrypLE (4ml for a T75 flask), and then incubated in fresh TrypLE (4ml for a T75 flask) for 3-5 minutes. Then, StC-SM medium was added (e.g. 9ml) and the cells were centrifuged at 300xg for 5 min at room temperature. The cell number was determined and the cells were diluted with StC-SM medium in order to obtain a density of 2x10E6 cells in 15 ml so that the cells can be plated. Media concentrations are described in detail in the materials section below. On days -5 and -3, the medium was replaced by fresh StC-SM medium. The completion of step (i**) (epicardial differentiation) has been experimentally assessed by the expression of Wilms Tumor antigen 1 (WT1) using immunofluorescence (**Figure 1C**). A clear expression of WT-1 was detected in the nuclei of the cells.

One example providing exact media volumes for a small scale and large scale generation of cardiac stromal cells is provided in the following table:

| Day | Medium | Small scale | Large scale |
|---|---|---|---|
| -17 | Split cells on Laminin-coated plate in iPS Brew+10 µM Rock Inhibitor; | 1xT75 with LMN coating in 24 ml | 1xT75 |
| -14 | Medium change iPS-Brew | 1xT75 with 12 ml | 1xT75 with 12 ml |
| -13 | Wash once with 15 ml RPMI, then add 21 ml StC-IM | 1xT75 with 21 ml | 1xT75 with 21 ml |
| -12 | StC-IM | 1xT75 with 21 ml | 1xT75 with 21 ml |
| -11 | StC-IM | 1xT75 with 21 ml | 1xT75 with 21 ml |
| -10 | StC-IM double-feed | 1xT75 with 36 ml | 1xT75 with 36 ml |
| -7 | Passage 1: TypLE protocol, see below | 1xT75 with LMN coating 2x10E6 cells in 15 ml StC-SM | 6xT225 with 6x10E6 per T225 in 45 ml StC-SM |
| -5 | StC-SM | 1xT75 with 15 ml | 6xT225 with 45 ml |
| -3 | StC-SM | 1xT75 with 15 ml | 6xT225 with 45 ml |
| 0 | Switch to StC-EM+C | 1xT75 with 15 ml | 6xT225 with 45 ml |
| 2 | StC-EM+C | 1xT75 with 15 ml | 6xT225 with 45 ml |
| 4 | Passage 2: Accutase/Versene protocol, see below | 1xT75 with VTN coating; 3x10E6 cells in 15 ml StC-EM | 12xT225 with 9x10E6 in 45 ml StC-EM |
| 7 | StC-EM | 1xT75 with 15 ml | 12xT225 with 45 ml |
| 9 | StC-EM | 1xT75 with 15 ml | 12xT225 with 45 ml |
| 11 | Passage 3: Accutase/Versene protocol, see below | 1xT75 with VTN coating; 3x10E6 cells in 15 ml | 12xT225 with 9x10E6 in 45 ml |
| 14 | StC-EM | 1xT75 with 15 ml | 12xT225 with 45 ml |
| 16 | StC-EM | 1xT75 with 15 ml | 12xT225 with 45 ml |
| 17 | Passage 4 or harvest for freezing: Accutase/Versene protocol, see below | 3xT75 with 2x10E6 cells each in 15 ml | 36xT225 with 6x10E6 in 45 ml |
| >17 | Expand by weekly passages | | |

### Materials

### Materials, suppliers, and storage conditions:

- iPS Brew Basalmedium, Miltenyi Biotec, Cat No.170-076-317
- iPS Brew Supplement R, Miltenyi Biotec, Cat No.170-076-318
- (CTS) Vitronectin, Thermo Scientific
- Rock Inhibitor (Stemolecule Y27632), Stemgent, Cat No. 04-0012-10, -20°C
- Versene (1x), Gibco, Cat No. 15040-033, 100 ml
- PBS (1x), Gibco, A1285601
- RPMI 1640 with Glutamax, Invitrogen, Cat No. 61870-010, 4°C
- 100X sodium pyruvate, Invitrogen, Cat No. 11360, 4°C
- L-ascorbic acid 2 phosphate sesquimagnesium salt hydrate (ASC), Sigma, Cat No. A8960-5G, -20°C
- Activin A, CellGenix, 1022-050 GMP or Activin A, R&D, -20°C
- BMP4, Peprotech, AF-120-05ET or BMP4, R&D, -20°C
- bFGF, Peprotech, GMP100-18B or bFGF, Peprotech RUO, -20°C
- VEGF165, GMP100-20 or VEGF165, Peprotech, RUO, -20°C
- CHIR, Stemgent, Cat No. 04-0004, -20°C
- (CTS) B27 supplement, Thermo Scientific, -20°C
- Retinoic acid, Sigma, Cat No. R2625
- (CTS) TrypLE select, Thermo Scientific, 4°C
- (CTS) KO DMEM, Thermo Scientific
- (CTS) KO serum replacement, Thermo Scientific
- Accutase, Thermo Scientific
- LMN-521, Biolamina
- Glutamin, Thermo Scientific
- CryoSure DMSO, WAK, WAK-DMSO-10, Room Temperature
- Sterile filters 0.2 µM (Sartorius Minisart RC25, DMSO-resistant)

### Stocks at (-20°C)

- BMP4 stock at 10 ug/ml
- Activin A stock at 10 ug/ml
- bFGF stock at 10 ug/ml
- VEGF stock at 5 ug/ml
- CHIR stock at 10 mM in DMSO
- Rock Inhibitor (Stemolecule Y27632), stock at 10mM in DMSO
- ASC-2-P stock at 200mM in water (ASC)
- Retinoic acid (Sigma: R2625): 8 mM stock: Dissolve 50 mg RA in 20.8 ml DMSO, sterile filter using a DMSO-resistant filter and aliquot with 500 µl in 0.5 ml tubes (store at -80°C for up to 6 months); use at 4 µM > 0.5 µl/ml medium

### Laminin (LMN) coating

LMN-521 solution was thawed (100 µg/ml) slowly at +2°C to +8°C. LMN 521 Laminin- (100 µg/ml) was diluted with 1xDPBSplus (Ca ++ / Mg ++), to obtain a final concentration of 0.9 µg/cm². For example for a T75cm² flask 15.3 ml PBS and 0.706 ml LMN 521 solution was mixed. The coating was allowed to settle overnight at 4°C. The flasks were warmed to room temperature for 1 hour before use. The supernatant was carefully aspirated (without touching the surface) and medium was immediately added to the flask.

### Vitronectin (VTN) coating:

Vitronectin dilutions were made and sterile filtered (0.2 µm) before use. For example:

| | Dilutions | | | |
|---|---|---|---|---|
| Flask | Vitronectin-Stock (0.9 mg/ml) | 1x PBS | Volume per flask | Amount per area |
| T-25 | 25 µl / flask | 4975 µl / flask | 5000 µl / flask | 0,9 µg /cm² |
| T-75 | 75 µl / flask | 14925 µl / flask | 15000 µl / flask | 0,9 µg /cm² |

The coating was performed for 1 hour at room temperature. The flasks were adjusted to room temperature for 1 h before use.

### Media preparation:

### iPS Brew medium:

iPS Brew medium was prepared according to the manufacturer's instructions (Miltenyi Biotec).

### RPMI wash medium:

Cells were washed RPMI 1640 with Glutamax medium (Invitrogen).

### Stromal cell induction medium (StC-IM), used in step (i*) of the differentiation/amplification protocol

The final concentration of the ingredients is provided in **Figure 8**. For example, 100ml of said StC-IM medium can be obtained according to the following recipe:

| Component | Vol. (for 100 ml) | Unit |
|---|---|---|
| RPMI with Glutamax | 97 | ml |
| Pyruvate | 1 | ml |
| Asc A | 100 | µl |
| B27 minus insulin | 2 | ml |
| CHIR | 10 | µl |
| BMP4 | 100 | µl |
| ActA | 30 | µl |
| bFGF | 50 | µl |

### Basal serum free medium, one component during step (i**) of the differentiation/amplification protocol (BSFM; 4°C)

BSFM was prepared and comprised RPMI 1640 with Glutamax, 1% of 100X sodium pyruvate, 2 % B27 supplement, and 200uM ASC.

### Stromal cell specification medium (StC SM), used in step (i**) of the differentiation/amplification protocol

The final concentration of the ingredients is also provided in **Figure 8**. StC-SM was obtained by supplementing BSFM with 50 ng/ml BMP4 (50 µl stock in 10 ml medium), 4 µmol/L retinoic acid (5 µl stock in 10 ml medium), and 1 µM CHIR (1µl stock in 10ml medium).

### Stromal cell amplification medium with CHIR (StC EM+C), used in step (i) a1) of the differentiation/amplification medium

StC-EM+C medium was used during step (i) a1)) of the differentiation/amplification protocol. Said medium was obtained by supplementing KO DMEM medium with 1% Glutamine, 10% (CTS) KO Serum Replacement, 50 ng/ml FGF (50 µl stock in 10 ml medium), 25 ng/ml VEGF and 1 µM CHIR (1µl stock in 10ml medium).

### Stromal cell amplification medium (StC-EM), used in step (i) a2) and (ii) of the differentiation/amplification medium

StC-EM medium was used during the second sub-step of step (i) a2) and during step (ii) of the differentiation/amplification method. Said medium was obtained by supplementing KO DMEM medium with 1% Glutamine, 10% (CTS) KO Serum Replacement, 50 ng/ml FGF (50 µl stock in 10 ml medium), and 25 ng/ml VEGF.

### Example 2: Mass production of cardiac stromal cells; amplification by at least 15 doublings

In order to show that cardiac stromal cells can not only be differentiated from epicardial cell (or optionally pluripotent stem cells), but can also be amplified, the inventors developed an amplification step (step (ii) of the protocol of Figures 1 and 8). During said step, the cardiac stromal cells retain their cellular properties/phenotype but the cell number amplifies to large quantities under serum-free defined conditions. Figure 2A shows the potential to which cardiac stromal cells can be expanded starting with a regular tissue culture flask with 75 cm². On average a culture flask with 75 cm² contains about 20x10E6 iPSC (2,7x10E5). When the cells were amplifying during step (ii) for 5 passages (passages 4-9) after the differentiation step (i) (optionally preceded by step (i*) and (i**)), the cells can amplify to a growth area of 515840 cm² (i.e. around 52 m²). The average output per cm² was around 1x10E5 cardiac stromal cells. Therefore, this protocol has the potential to achieve 5.2x10E10 cardiac stromal cells when starting from a confluent layer of human pluripotent stem cells in a tissue culture flask with 75 cm² (or 2600 cardiac stromal cells per input iPSC).
Figure 2B provides experimental evidence on the cardiac stromal cell population doublings by showing the total number of times the cells have doubled. For each passaging step, 1-1.3x10E4 cells per cm² were seeded. This data shows that cardiac stromal cells can by expanded by 15 doublings when step (ii) of the protocol was performed for 5 passages (passages 4-9). From this data, it can be appreciated that the cardiac stromal cells show an average doubling time of 2.1±0.4 days during the amplification phase (step (ii) of the protocol). This corresponds to about a tenfold increase in the number of cells per week (per passage). Furthermore, the amplification of the cardiac stromal cells does not plateau, which is also underlining that the cardiac stromal cells expand continuously under the amplification step conditions. In summary, this data shows that the serum-free defined protocol is suitable for large scale production of cardiac stromal cells, mainly due to the serum-free amplification step (ii) of the method.

### Example 3: Generation of a highly pure and homogenous population of cardiac stromal cells

To verify the directed differentiation and mass amplification into highly pure and homogenous cardiac stromal cells, the inventors analysed the cardiac stromal cells by several independent methods: Firstly, (1) the expression of extracellular and intracellular markers was analysed by flow cytometry and immunofluorescence (**Figure 3A-C**).

In order to verify the purity and homogeneity of the generated cardiac stromal cells, the cells were analysed by flow cytometry (**Figure 3A**). In flow cytometry, as used here, the expression of cardiac stromal cell markers was measured using immunostaining. In particular, the expression of CD90, CD44, CD73, Collagen 1 and Vimentin after step (i) a2) was assessed in order to characterize the generated cardiac stromal cells. CD90, CD44 and CD73 are extracellular markers, while Collagen 1 and Vimentin are intracellular markers. The proportion of CD90 positive cells was 95%; the proportion of CD44 positive cells was 99%; the proportion of CD73 positive cells was 99% measured against respective isotype controls (IgG1, BD Biosciences). The proportion of Collagen 1 positive cells was 97%; the proportion of Vimentin positive cells was 98%. At the same time the negative control (polyclonal rabbit IgG) only showed a background signal of 3%. This shows that the obtained cardiac stromal cells were highly pure and homogenous. In summary, **Figure 3A** shows that the cardiac stromal cells obtained after step (i) a2) were a homogenous population of cells characterized by the expression of CD90, CD44, CD73, Collagen 1 and Vimentin in at least 90% of the cells.
In order to verify the generation of cardiac stromal cells further, the cells were also analysed by fluorescence microscopy (**Figure 3B**). In this process, vimentin, anti-human fibroblast specific protein and Collagen1 were stained immunologically and DNA of the cell nuclei was visualized with Hoechst 33342. All three markers show a homogenous distribution and a high purity of cardiac stromal cells after step (i) a2). Furthermore, the morphology of the cells is typical for cardiac stromal cells.
In order to not only analyze the expression of CD90, CD73 and CD44 in cardiac stromal cells, said markers were specifically assessed by FACS during the differentiation and amplification protocol (**Figure 3C**). After completion of step (i) a1) (culture day 4) more than 50% of the cells expressed CD90; less than 50% of the cells expressed CD73; less than 30% of the cells expressed CD44, and more than 80% of the cells expressed vimentin. On culture day 17 (during step (i) a2)) the markers CD90, CD73, and vimentin were expressed in more than 90% of the cells, the marker CD44 was expressed in more than 80% of the cells. After completion of step (i) a2) (culture day 17) the markers CD90, CD73, CD44, and vimentin were expressed in more than 90% of the cells. The expression profile after the optional step (i*) was also assessed (culture day -7; please refer to Figure 8) and more than 90% of the cells expressed CD90, less than 10% of the cells expressed CD73, less than 10% of the cells expressed CD44, and more than 40% of the cells expressed vimentin. Furthermore, CD73 was successively more expressed over the course of the differentiation. Similarly, CD44 was also successively more expressed over the course of the differentiation and amplification protocol.

### Example 4: Mass amplification and maintained expression of characteristic markers of cardiac stromal cells

In order to show that the amplification step (ii) leads to a maintained expression of the characteristic markers of cardiac stromal cells, a FACS analyses of the cardiac stromal cells at passage 9 was performed (**Figure 3D**). At passage 9, the cardiac stromal cells were already amplified over 5 passages (see Figure 2). As demonstrated in **Figure 3D**, more than 90% of the amplified cardiac stromal cells expressed CD90, CD44, and CD73. Thus, cardiac stromal cells retained the phenotype after step (i) a2) stably and maintained expression of said characteristic markers. This showed that said amplification step is suitable for mass amplification of cardiac stromal cells under defined serum-free conditions. Furthermore, it is plausible that cardiac stromal cells can be amplified indefinitely under the conditions of step (ii) as long as the characteristic markers CD90, CD44 and CD73 maintain expression.

### Example 5: Genetic analysis and analysis of fibroblast properties of the obtained cardiac stromal cells

As a second and third independent method, the genetic characteristics of the cardiac stromal cells were assessed: Specifically, (2) the gene expression of CD140a, vimentin (VIM), collagen-1 (COL1A1), CD90, CD73 and CD44 in iPSCs, iPSC derived cardiomyocytes (CM), (iPSC-derived) cardiac stromal cells (cStC), and heart derived primary cardiac fibroblasts (as control) were compared (**Figure 4**); and (3) a transcriptome analysis was performed to compare and contrast the obtained cardiac stromal cells with primary cardiac fibroblasts and other fibroblast types (**Figure 5A and B**);

To further demonstrate that the gene expression profile of the cardiac stromal cells also mirrored the identity of cardiac stromal cells, the expression of several marker genes was analyzed and compared in induced pluripotent stem cells (iPSC), iPSC derived cardiomyocytes (iPSC CM), (iPSC derived) cardiac stromal cells as disclosed herein (cStC) and human primary cardiac fibroblasts.

Expression values of **Figure 4A** and **4B****:**

| Expression values in RPKM | iPSCs | iPSC-derived cardiomyocytes | (iPSC-derived) cardiac stromal cells (cStC) | primary cardiac fibroblasts |
|---|---|---|---|---|
| PDGFRA (CD140a) | 1 | 8 | 70 | 204 |
| VIM | 13 | 95 | 1310 | 2421 |
| COL1A1 | 4 | 47 | 1540 | 726 |
| THY1 (CD90) | 34 | 1 | 30 | 57 |
| NT5E (CD73) | 1 | 2 | 227 | 515 |
| CD44 | 1 | 2 | 142 | 97 |

As can be readily seen in **Figures 4A** and **4B**, iPSC and iPSC CM did not express CD140a, vimentin, collagen 1, CD73 or CD44 at a level above background. In contrast, cardiac stromal cells as disclosed herein and primary cardiac fibroblasts expressed CD140a, vimentin, collagen 1, CD73 or CD44. The expression of CD90 could be detected in iPSC, cardiac stromal cells as disclosed herein (cStC) and primary cardiac fibroblasts, while cardiomyocytes lost the expression of CD90 over the course of differentiation. In summary, the gene expression analysis showed by an independent method that also the genetic markers or cardiac stromal cells were expressed in the cells as obtained by the method disclosed herein (**Figure 4**). Furthermore, the expression profile of primary cardiac fibroblasts were mirrored by the cardiac stromal cells as obtained by the disclosed method. Furthermore, the gene expression profile was different in every tested marker compared to cardiomyocytes, which underlined the clear difference in expression profile between cardiomyocytes and non-myocytes, i.e. the cardiac stromal cells.
In order to ensure that specifically cardiac stromal cells were generated, the transcriptome of the obtained cardiac stromal cells was compared to primary cardiac fibroblasts, neonatal skin fibroblasts, and gingival fibroblasts (**Figure 5A and 5B**). RNA was extracted from these cells, sequenced, and the population expression pattern was analysed. When comparing the transcriptomes of the obtained cardiac stromal cells, primary cardiac fibroblasts, neonatal skin fibroblasts, and gingival fibroblasts a principal component analysis was performed (**Figure 5A**), which indicated that 46% of total variance could be attributed to principle component 1 (PC1). A further detailed analysis of the top 100 variance-causing RNAs of PC1 as well as characteristic, fibroblast-enriched genes from the inventors previous analyses (Figure 3B in Tiburcy et al. 2017) indicated that the expression patterns of obtained cardiac stromal cells were to a large extent in agreement with the primary cardiac fibroblasts. The indicated genes in Figure 5B are the following: DDR2, THY1, TIMP2, TBX4, HOXA11, ISL1, MMP1, CD44, NTSE, BMP4, TCF21, SOX17, TBX20, HEY1, HOXAS, TBX1, COL1A1, FN1, DES, HAND1, PECAM1, WT1, TEX, HAND2, GATA4, ALDH2, POSTN.
However, the obtained cardiac stromal cells showed clear differences when compared to the neonatal skin fibroblasts and gingival fibroblasts. Therefore, the obtained cardiac stromal fibroblasts were clearly more comparable to primary cardiac fibroblasts than skin and gingiva fibroblasts. Consequently, the transcriptome analysis shows that the specific type of stromal cells, i.e. cardiac stromal cells, were obtained by the protocol according to **Figure 1** and **8**. To further analyze the fibroblast-properties of the resulting cardiac stromal cells, the inventors also tested whether the cells could be stimulated to enhance collagen secretion and expression of myofibroblast markers. The conversion to myofibroblasts is for example crucial in the context of wound healing processes/scar formation and said conversion can be induced by e.g. TGFb1 and Angiotensin II. **Figure 5C** shows that pro-collagen protein and smooth muscle actin (a-SMA) and CTGF were induced by TGFbeta and or/ Angiotensin II, which are all myofibroblast markers. Tubulin served as a loading control. Consistent with an activated primary cardiac fibroblast phenotype in vitro the cardiac stromal cells showed a robust expression of periostin even under non-stimulated control conditions supporting the RNA expression analyses (**Figure 5B**). Thus, the cardiac stromal cells produced according to the method disclosed herein were also capable of secreting collagen as well as the conversion to myofibroblasts.

### Example 6: Suitability for engineered human tissue

In addition to the structural analysis of the cardiac stromal cells, the functional characteristics of the obtained cardiac stromal cells was also assessed by the inventors. Thus, as a fourth (4) independent method, the suitability for engineered tissue, exemplified by the engineering of heart muscle and connective tissue, was also tested by the inventors. Said fourth independent method was carried out by performing contraction experiments, which compare primary cardiac fibroblasts and the obtained cardiac stromal cells in engineered human myocardium (EHM; **Figure 6A**) and engineered connective tissue (ECT; **Figure 6B**).
For EHM generation, the primary cardiac fibroblasts or the obtained cardiac stromal cells were mixed with cardiomyocytes and extracellular matrix according to the methods disclosed in Tiburcy et al. (2017). Briefly, these contraction experiments were carried out in organ baths and measure the force of contraction (FOC) of the produced engineered heart muscle/engineered myocardium in response to electrical stimulation (see further details in the methods section herein). Specifically, the FOC of engineered human myocardium (EHM) were made with 70% cardiomyocytes and 30% primary cardiac fibroblasts (black bar) or cardiac stromal cells as disclosed herein (gray bar). Extracellular calcium concentration was 2 mmol/L and the EHM was stimulated with an electrical stimulus of 1.5 Hz and the FOC was measured in millinewtons (mN). The FOC of the EHM generated with primary cardiac fibroblasts was 2.0 mN with a standard error or the mean of 0.1 mN, while the FOC of the EHM generated with the cardiac stromal cells as disclosed herein was 1.9 mM with a standard error of the mean of 0.03 mN. Thus, the FOCs were comparable and were nearly identical. Furthermore, the obtained cardiac stromal cells showed an 3-fold smaller standard error, which emphasises the reproducibility being achieved by using serum-free defined cardiac stromal cells obtained by the method as disclosed herein. This very low standard error underlines the high purity and homogeneity of the obtained cardiac stromal cells according to the method of **Figure 1** and **8**.
Similarly, ECT was generated with the primary cardiac fibroblasts and cardiac stromal cells as disclosed herein and the result in stiffness upon treatment with TGFb1 was compared. A key feature of connective tissue is that the stiffness increases upon treatment with a pro-fibrotic stimulus like TGFbeta1 (TGFb1), as previously described in Dworatzek et al. 2019. Briefly, these stiffness experiments measure stiffness in the presence or absence of TGFb1 (see further details in the methods section herein). Specifically, the stiffness of ECT was measured in kilo Pascals (kPa). In the absence of TGFb1, the stiffness of ECT generated with primary cardiac fibroblasts and cardiac stromal cells as described herein was 6 and 9 kPa, respectively (SEM: 1kPa for the primary cardiac fibroblasts and 2kPa for the cardiac stromal cells). In the presence of 5 ng/ml TGFb1, the stiffness of the ECT generated with cardiac stromal cells was 46 kPa (SEM: 1 kPa), while the stiffness of the ECT generated with primary cardiac fibroblasts was only 21 kPa (SEM: 1 kPa). Thus, the stiffness was significantly larger upon induction with TGFb1 indicative of a fibrotic response. Furthermore, the stiffness was significantly larger in ECT generated with cardiac stromal cells as disclosed herein compared to ECT generated with primary cardiac fibroblasts. This again supports underlines the sensitivity of the cardiac stromal cells to pro-fibrotic stimuli and underlines the suitability of the cardiac stromal cells as disclosed herein for fibrosis modeling in engineered human tissue.

### Example 7: Amplification of cardiac stromal cells can only be performed in the presence of an extracellular matrix protein and does not work on uncoated culture surfaces under strictly serum-free conditions

The method as depicted in **Figure 8** shows that the amplification step (ii) was carried out on vitronectin coated plates. The inventors also tested whether other ECM proteins would also support the amplification step and whether an amplification without coating the plates would also work in serum-free conditions. To test this idea, the cardiac stromal cells were cultivated on vitronectin, laminin LN-521, collagen in the form of gelatine, fibronectin, matrigel coated and on uncoated plates for passage 5 and 6 and the results are shown in **Figure 7****.** All ECM protein coated plates (vitronectin, LN-521, gelatine, fibronectin) and the matrigel coated plates supported further amplification of the cardiac fibroblasts, while the cardiac stromal cells on the uncoated plates died in passage 5 on the uncoated plates. Thus, the cardiac stromal cells according to Figures 1 or 8 cannot be expanded on uncoated plates. For example, US 2018/0094245 A1 discloses in paragraph [0081], that the cardiac fibroblasts were plated on uncoated plates for amplification. At first sight, the data of **Figure 7** seems contradicting to US 2018/0094245 A1. However, paragraph [0081] of said disclosure also states that the cardiac fibroblasts were plated in the presence of 2% fetal bovine serum. Therefore, the plating step is not serum-free in US 2018/0094245 A1. In fact, it is known that FBS contains variable amounts glycoproteins, which may coat the culture substrate and thus may create an undefined surface coating. Thus it is plausible that the serum during the plating step in US 2018/0094245 A1 supported the further growth of the cardiac fibroblasts on initially uncoated plates. The loss of CD90 suggests a transformation of the fibroblasts and thus the fibroblasts according to US 2018/0094245 A1 have a different phenotype compared to the disclosed cardiac stromal cells. As the present differentiation/amplification protocol is fully serum-free, the defined extracellular matrix protein coating of the plates was demonstrated to be essential. The extracellular matrix protein can be selected from vitronectin, LN-521, gelatine, fibronectin or even matrigel, according to **Figure 7****.** Furthermore, it is plausible that any other extracellular matrix protein will support the amplification step in the same way as vitronectin, LN-521, gelatine, fibronectin supported the amplification step. Of course, a defined extracellular matrix protein such as vitronectin, LN-521, gelatine or fibronectin is preferred.

### Methods

### Bright field microscopy

Cells were imaged using a Zeiss Axiovert200 microscope with Zeiss Axiocam MRm camera. Photos were acquired using Zeiss Axiovision software.

### Flow cytometry

Single live cell suspensions were analyzed after staining with CD44-APC H7 (BD Biosciences), CD90-PE, and CD73-APC (all BD Biosciences) for 10 min at 4°C. After washing, cells were incubated for 10 min in Sytox Blue Dead Cell Stain (Life Technologies, 5 nmol/L) to exclude dead cells. Vimentin (abcam) and Collagen 1 (abcam) were stained in 4% formalin fixed cell suspensions for 45 min at 4°C followed by staining with appropriate secondary antibodies and Hoechst-33342 (Life Technologies, 10 µg/ml) to detect nuclear DNA for 30 min at 4°C. Cells were run on either a LSRII SORP Cytometer (BD Biosciences) or CytoFlex Cytometer (Beckman Coulter). At least 10,000 events were analyzed per sample.

### Immunofluorescence staining.

Cells were fixed in 4% formalin (Histofix, Roth). After 3 washes with PBS, cells were incubated with primary antibodies in PBS, 5% fetal bovine serum (Thermo Scientific), 1% bovine serum albumin, 0.5% Triton-X (both Sigma-Aldrich) for 2 hours at room temperature or overnight at 4°C. The antibodies used were Vimentin (abcam), Anti-human fibroblast (clone TE-7, Millipore), collagen 1 (abcam), and Wilms Tumor Antigen 1 (WT1, abcam). After several washes, appropriate secondary antibodies and/or phalloidin (to label f-actin) and Hoechst-33342 (Life Technologies, 10 µg/ml) to detect nuclear DNA were added for 1 hour at room temperature. Immunostainings were imaged using a Zeiss LSM710 confocal microscope.

### RNA sequencing (transcriptome analysis)

RNA was extracted using the TRIZOL method (Thermo Scientific). Quality and integrity of RNA was assessed with the Fragment Analyzer from Advanced Analytical by using the standard sensitivity RNA Analysis Kit (DNF-471). RNA-seq libraries were prepared using a modified strand-specific, massively-parallel cDNA sequencing (RNA-Seq) protocol from Illumina, the TruSeq Stranded Total RNA (Cat.No. RS-122-2301). Libraries were sequenced on a HiSeq 4000 platform (Illumina) generating 50 bp single-end reads (30-40 Mio reads/sample). Sequence images were transformed with Illumina software BaseCaller to BCL files, which was demultiplexed to fastq files with bcl2fastq v2.17.1.14. The quality check was done using FastQC (version0.11.5, Babraham Bioinformatics). Sequence reads were aligned to the human genome reference assembly (UCSC version hg38) using Star. For each gene, the number of mapped reads was counted using FeatureCounts. Raw counts were normalized and transformed to log2CPM values. Using a Z-scale normalized matrix a heatmap was generated using the heatmap function in R applying a euclidean distance clustering algorithm. Reads Per Kilobase per Million mapped reads (RPKM) were calculated based on Ensembl transcript length using biomaRT (v2.24).

### EHM generation and analysis

EHM generation has been described in Tiburcy et al. (2017) in detail. Briefly, for EHM generation single cells suspension of iPSC-derived cardiomyocytes, primary cardiac fibroblasts, and cardiac stromal cells, as obtained by the method described herein, were prepared. Equal volumes of collagen (0.9 mg/ml) and concentrated serum-free medium (2x RPMI, 8% B27 without insulin, 200 U/ml penicillin, and 200 mg/ml streptomycin) were mixed on ice. After pH neutralization by dropwise addition of 0.1 N NaOH either 1x10E6 cardiomyocytes with 0,5x10E6 primary cardiac fibroblasts (Lonza) or 1x10E6 cardiomyocytes with 0,5x10E6 cardiac stromal cells, as obtained by the method described herein, were added to the collagen mix. After 60 min of hydrogel formation at 37°C EHM culture medium was added: Iscove's medium with 4% B27 without insulin, 1% non-essential amino acids, 2 mmol/l glutamine, 300 µmol/l ascorbic acid, 100 ng/ml IGF1 (AF-100-11), 10 ng/ml, FGF-2 (AF-100-18B), 5 ng/ml VEGF165 (AF-100-20), 5 ng/ml TGF-β1 (AF-100-21C), and P/S (all growth factors were obtained from PeproTech).
After 4 weeks of EHM maturation on flexible posts EHM were isometrically suspended in organ baths (Föhr Medical Instruments) filled with Tyrode's solution (in mmol/L: 120 NaCl, 1 MgCl2, 0.2 CaCl2, 5.4 KCI, 22.6 NaHCO3, 4.2 NaH2PO4, 5.6 glucose, and 0.56 ascorbate) at 37°C and constant bubbling with 5% CO2 and 95% 02. Force of contraction measurements were performed at 1.5 Hz electrical field stimulation with 5 ms square pulses (150 mA) and 2 mM extracellular calcium concentration.

### ECT generation and analysis

In general, the ECT was generated as previously described in Santos et al. 2019 and is described in detail therein. Briefly, to generate hECT, 0.3 mg bovine collagen type I (Collagen solutions LLC) was neutralized with 0.1 N NaOH, buffered with 2x DMEM and then mixed with 0.75x10⁶ cardiac stromal cells as described herein per tissue (Santos et al. 2019). The mixture was cast into a polystyrene cell culture plate with 48 wells containing two flexible poles each (produced by TPK-Kunststofftechnik GmbH according to the inventor's patent 2016060314484000DE) and allowed to gel for 1 h in a humidified incubator at 37 °C and 5% CO₂. During culture ECT condense around the flexible poles and remain suspended between them. ECTs were treated without (control) or with 5 ng/ml TGFb1 for 5 days. Stiffness was measured by destructive tensile stress test using an RSA-G2 rheometer (TA Instruments) and calculated from the slope of the stress-strain curve as Young's modulus (reported as E in kPa; Santos et al. 2019). Briefly, the engineered tissues were transferred from the flexible poles into an organ bath containing PBS at 37 °C and fixed between two custom-made hooks. Tissues were then stretched at a constant linear rate of 0.03 mm/s for human tissues until the point of rupture.

### Western Blot analysis

For protein extraction, cells lysis was performed in CytoBuster (Merck Millipore) containing cOmplete protease inhibitor cocktail and PhosStop phosphatase inhibitor cocktail (Merck) for 10 min on ice. After a centrifugation step for 30 min at 12,000 g at 4°C, the supernatant was supplemented with 4x SDS-containing Laemmli buffer. The samples were denatured and subjected to SDS-PAGE on 10% polyacrylamide gels, followed by transfer onto Amersham Protran nitrocellulose membranes (GE Healthcare). The membranes were cut according to the molecular weight of the specific proteins and blocked with 1x Rotiblock (Carl Roth) for 1 h at room temperature, followed by a washing step with TBST (10 mM Tris pH 7.4, 150 mM NaCl, 0.1% Tween 20) before probing the membranes at 4°C overnight with primary antibodies. The primary antibodies were Pro-collagen-1 (Santa Cruz Biotechnologies), alpha-tubulin (Sigma-Aldrich), smooth muscle actin (Sigma-Aldrich), Periostin (Santa Cruz Biotechnologies), and CTGF (Santa Cruz Biotechnologies). Incubation with appropriate secondary peroxidase-coupled antibodies (Sigma-Aldrich) for 1 h at room temperature was preceded and followed by three washing steps with TBST. Finally, secondary antibodies were detected using SuperSignal West Femto Maximum Sensitivity Substrate (Thermo Scientific) and a ChemiDoc MP Imaging System (Bio-Rad).

### Tables

**Table 1a: Composition of the serum-free supplement 'B27 minus insulin' (50x concentration, liquid)**

| 10 ml of 'B27 minus insulin' per 500 ml medium corresponds to 2% B27 minus insulin (v/v); 'B27 minus insulin' contains the same ingredients as 'B27' but is lacking human insulin. | | |
|---|---|---|
| **ingredients** | **concentration in B27** | **final concentration in basal medium** |
| | **µg/ml** | **µg/ml** |
| Bovine serum albumin, fraction V IgG free, fatty acid poor | 125000 | 2500 |
| Catalase | 125 | 2,5 |
| Glutathion reduced | 50 | 1 |
| Superoxide Dismutase | 125 | 2,5 |
| Humanes Holo-Transferrin | 250 | 5 |
| T3 (triodo-I-thyronine) | 0,1 | 0,002 |
| L-carnitine-HCl | 100 | 2 |
| Ethanolamine | 50 | 1 |
| D+-galactose | 750 | 15 |
| Putrescine | 805 | 16,1 |
| sodium-Selenite | 0,625 | 0,0125 |
| Corticosterone | 1 | 0,02 |
| linoleic acid | 50 | 1 |
| linolenic acid | 50 | 1 |
| Progesterone | 0,315 | 0,0063 |
| Reti nylacetate | 5 | 0,1 |
| DL -alpha tocopherole (Vit E) | 50 | 1 |
| DL-alpha tocopherol Acetate | 50 | 1 |
| Biotin | 125 | 2,5 |

**Table 1b: Composition of the serum-free supplement B27 (50% concentration, liquid)**

| 10 ml of (50%-)B27 per 500 ml medium corresponds to 2% B27 (v/v); | | |
|---|---|---|
| **ingredients** | **concentration in 50% B27** | **final concentration in basal medium at 2% B27** |
| | **µg/ml** | **µg/ml** |
| Bovine serum albumin, fraction V IgG free, fatty acid poor | 125000 | 2500 |
| Catalase | 125 | 2,5 |
| Glutathion reduced | 50 | 1 |
| Human Insulin | 156,25 | 3,125 |
| Superoxide Dismutase | 125 | 2,5 |
| Humanes Holo-Transferrin | 250 | 5 |
| T3 (triodo-I-thyronine) | 0,1 | 0,002 |
| L-carnitine-HCI | 100 | 2 |
| Ethanolamine | 50 | 1 |
| D+-galactose | 750 | 15 |
| Putrescine | 805 | 16,1 |
| sod i u m-Selen ite | 0,625 | 0,0125 |
| Corticosterone | 1 | 0,02 |
| linoleic acid | 50 | 1 |
| linolenic acid | 50 | 1 |
| Progesterone | 0,315 | 0,0063 |
| Reti nylacetate | 5 | 0,1 |
| DL -alpha tocopherole (Vit E) | 50 | 1 |
| DL-alpha tocopherol Acetate | 50 | 1 |
| Biotin | 125 | 2,5 |

**Table 2: Composition of ECCM supplement (100%)**

| **Substance** | **concentration (µg/ml)** | **Substance** | **concentration (µg/ml)** |
|---|---|---|---|
| Glycine | 150 | Ag⁺ | 0.0006 |
| L-Histidine | 940 | Al³⁺ | 0.0007 |
| L-Isoleucine | 3400 | Ba²⁺ | 0.008 |
| L-Methionine | 90 | Cd²⁺ | 0.03 |
| L-Phenylalanine | 1800 | Cr³⁺ | 0.003 |
| L-Proline | 4000 | Ge⁴⁺ | 0.003 |
| L-Hydroxyproline | 100 | Se⁴⁺ | 0.02 |
| L-Serine | 800 | Br⁻ | 0.004 |
| L-Threonine | 2200 | I⁻ | 0.0007 |
| L-Tryptophane | 440 | Mn²⁺ | 0.0004 |
| L-Tyrosine | 77 | F⁻ | 0.010 |
| L-Valine | 2400 | Si⁴⁺ | 0.01 |
| Thiamine | 33 | V⁵⁺ | 0.003 |
| Glutathione reduced | 10 | Mp⁶⁺ | 0.006 |
| Ascorbic acid -2-PO₄ (Mg-salt) | 330 | Ni²⁺ | 0.0002 |
| Transferrin | 55 | Rb⁺ | 0.005 |
| Insulin | 100 | Sn²⁺ | 0.0002 |
| sod i u m-Selen ite | 0.07 | Zr⁴⁺ | 0.01 |
| AlbuMAX | 83 000 | | |
| AgNO₃ | 0.0009 | KBr | 0.0006 |
| AlCl₃ 6H₂O | 0.006 | KI | 0.0009 |
| Ba(C2H₃O₂)₂ | 0.01 | MnCl₂ 4H₂O | 0.002 |
| CdSO₄ 8H₂O | 0.08 | NaF | 0.02 |
| COCl₂ 6H₂O | 0.01 | Na2SiO₃ 9H₂O | 1 |
| Cr₂(SO4)₃ 1H₂O | 0.003 | NaVO₃ | 0.006 |
| GeO₂ | 0.003 | (NH₄)₆MO₇O₂₄ 4H₂O | 0.06 |
| Na2SeP₃ | 0.007 | RbCl | 0.007 |
| H₂SeO₃ | 0.02 | SnCl₂ | 0.0003 |
| | | ZrOCl₂ 8H₂O | 0.02 |

### List of references

US 2018/0094245 A1
WO 98/30679
WO2015/040142
Bao X, Lian X, Hacker TA, Schmuck EG, Qian T, Bhute VJ, Han T, Shi M, Drowley L, Plowright A, Wang OD, Goumans MJ, Palecek SP. Long-term self-renewing human epicardial cells generated from pluripotent stem cells under defined xeno-free conditions. Nat Biomed Eng. 2016;1. pii: 0003. doi: 10.1038/s41551-016-0003
Bao X, Lian X, Qian T, Bhute VJ, Han T, Palecek SP. Directed differentiation and long-term maintenance of epicardial cells derived from human pluripotent stem cells under fully defined conditions. Nat Protoc. 2017 Sep;12(9):1890-1900. doi: 10.1038/nprot.2017.080
Brewer, G. J., Torricelli, J. R., Evege, E. K. and Price, P. J. (1993), Optimized survival of hippocampal neurons in B27-supplemented neurobasal™, a new serum-free medium combination. J. Neurosci. Res., 35: 567-576.
Chen VC, Couture SM, Ye J, et al. Scalable GMP compliant suspension culture system for human ES cells. Stem Cell Res. 2012;8(3):388-402.
Chen VC, Ye J, Shukla P, et al. Development of a scalable suspension culture for cardiac differentiation from human pluripotent stem cells. Stem Cell Res. 2015;15(2):365-375.
Didié M, Christalla P, Rubart M, Muppala V, Döker S, Unsöld B, El-Armouche A, Rau T, Eschenhagen T, Schwoerer AP, Ehmke H, Schumacher U, Fuchs S, Lange C, Becker A, Tao W, Scherschel JA, Soonpaa MH, Yang T, Lin Q, Zenke M, Han DW, Schöler HR, Rudolph C, Steinemann D, Schlegelberger B, Kattman S, Witty A, Keller G, Field LJ, Zimmermann WH (2013) Parthenogenetic Stem Cells for Tissue Engineered Heart Repair. J Clin Invest 123: 1285-1298.
Dworatzek E, Mahmoodzadeh S, Schriever C, Kusumoto K, Kramer L, Santos G, Fliegner D, Leung YK, Ho SM, Zimmermann WH, Lutz S, Regitz-Zagrosek V (2019) Sex-specific regulation of collagen I and III expression by 17β-Estradiol in cardiac fibroblasts: role of estrogen receptors. Cardiovasc Res. 115:315-327.
Frank ND, Jones ME, Vang B, Coeshott C (2019) Evaluation of reagents used to coat the hollow-fiber bioreactor membrane of the Quantum® Cell Expansion System for the culture of human mesenchymal stem cells. Materials Science and Engineering 96, 77-85
Hynes RO, Naba A. Overview of the matrisome-an inventory of extracellular matrix constituents and functions. Cold Spring Harb Perspect Biol. 2012;4(1):a004903.
Ieda M, Tsuchihashi T, Ivey KN, Ross RS, Hong TT, Shaw RM, Srivastava D. Cardiac fibroblasts regulate myocardial proliferation through beta1 integrin signaling. Dev Cell. 2009 Feb;16(2):233-44
Ivey MJ, Tallquist MD (2016) Defining the Cardiac Fibroblast: A New Hope. Circ J 80(11):2269-2276
Iyer D, Gambardella L, Bernard WG, Serrano F, Mascetti VL, Pedersen RA, Talasila A, Sinha S. Robust derivation of epicardium and its differentiated smooth muscle cell progeny from human pluripotent stem cells. Development. 2015 Apr 15;142(8): 1528-41.
Kattman SJ, Alec D. Witty, Mark Gagliardi, Nicole C. Dubois, Maryam Niapour, Akitsu Hotta, James Ellis, Gordon Keller (2011) Stage-Specific Optimization of Activin/Nodal and BMP Signaling Promotes Cardiac Differentiation of Mouse and Human Pluripotent Stem Cell Lines, Cell Stem Cell, Volume 8, Issue 2, 228 - 240
Li Y, Song D, Mao L, Abraham DM, Bursac N, Lack of Thy1 defines a pathogenic fraction of cardiac fibroblasts in heart failure. Biomaterials 2020; 236
Naito, H. et al. Optimizing engineered heart tissue for therapeutic applications as surrogate heart muscle. Circulation 114, 172-78 (2006).
Pinto AR, Ilinykh A, Ivey MJ, Kuwabara JT, D'Antoni ML, Debuque R, Chandran A, Wang L, Arora K, Rosenthal NA, Tallquist MD. Revisiting Cardiac Cellular Composition. Circ Res. 2016 Feb 5;118(3):400-9.
Santos G, Hartmann S, Zimmermann WH, Ridley A, Lutz S (2019) Inhibition of Rho-associated kinases suppresses cardiac myofibroblast function in engineered connective and heart muscle tissues. J Mol Cell Cardiol. 134:13-28.
Schlick, Susanne Franziska (2018) Fibroblast-Cardiomyocyte Cross-Talk in Heart Muscle Formation and Function, Doctoral Thesis, in partial fulfillment of the requirements for the degree, "Doctor rerum naturalium (Dr. rer. nat.)", at the Georg-August University Göttingen, November 2018: https://d-nb.info/1177361744/34
Schlick SF, Spreckelsen F, Tiburcy M, Iyer LM, Meyer T, Zelarayan LC, Luther S, Parlitz U, Zimmermann WH, Rehfeldt F (2019) Agonistic and antagonistic roles of fibroblasts and cardiomyocytes on viscoelastic stiffening of engineered human myocardium. Prog Biophys Mol Biol 144:51-60
Takahashi, Kazutoshi et al. Induction of Pluripotent Stem Cells from Adult Human Fibroblasts by Defined Factors. Cell. 2007; 131(5), 861 - 872
Takahashi K, Yamanaka S. Induction of Pluripotent Stem Cells from Mouse Embryonic and Adult Fibroblast Cultures by Defined Factors. Cell. 2006 Aug 25; 126(4):P663-676
Thomson J, Itskovitz-Eldor, Shapiro et al. Embryonic Stem Cell Lines Derived from Human Blastocysts. Science. 06 Nov 1998. 282(5391), 1145-1147
Tiburcy, M., Hudson, J. E., Balfanz, P., Schlick, S., Meyer, T., Chang Liao, M. L., Levent, E., Raad, F., Zeidler, S., Wingender, E., Riegler, J., Wang, M., Gold, J. D., Kehat, I., Wettwer, E., Ravens, U., Dierickx, P., van Laake, L. W., Goumans, M. J., Khadjeh, S., ... Zimmermann, W. H. (2017). Defined Engineered Human Myocardium With Advanced Maturation for Applications in Heart Failure Modeling and Repair. Circulation, 135(19), 1832-1847.
Wehrhan F, Nkenke E, Melnychenko I, Amann K, Schlegel KA, Goerlach C, Zimmermann WH, Schultze-Mosgau S (2010) Skin repair using a porcine collagen I/III membrane-vascularization and epithelization properties. Dermatol Surg. 36:919-930
Witty AD, Mihic A, Tam RY, Fisher SA, Mikryukov A, Shoichet MS, Li RK, Kattman SJ, Keller G. (2014) Generation of the epicardial lineage from human pluripotent stem cells. Nat Biotechnol. Oct;32(10): 1026-35.
Yanzhen Li, Daniel Song, Lan Mao, Dennis M. Abraham, Nenad Bursac (2020) Lack of Thy1 defines a pathogenic fraction of cardiac fibroblasts in heart failure, Biomaterials, Volume 236, 119824
Zhang H, Tian L, Shen M, Tu C, Wu H, Gu M, Paik DT, Wu JC. Generation of Quiescent Cardiac Fibroblasts From Human Induced Pluripotent Stem Cells for In Vitro Modeling of Cardiac Fibrosis. Circ Res. 2019 Aug 16;125(5):552-566.
Zhou P, Pu WT. Recounting Cardiac Cellular Composition. Circ Res. 2016 Feb 5;118(3):368-70.
Zimmermann WH, Melnychenko I, Wasmeier G, Didie M, Naito H, Nixdorff U, Hess A, Budinsky L, Brune K, Michaelis B, Dhein S, Schwoerer A, Ehmke H, Eschenhagen T (2006) Engineered heart tissue grafts improve systolic and diastolic function in infarcted rat hearts. Nat Med 12:452-458.

## Claims

1. A method for producing a population of cardiac stromal cells from pluripotent stem cells, the method comprising the steps of:
i. Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises a1) culturing said epicardial cells under suitable conditions in the presence of a first extracellular matrix protein in a serum-free basal medium;
followed by a2) culturing the cells of step (i) a1) under suitable conditions in the presence of a second extracellular matrix protein in a serum-free basal medium; wherein at least about 80 % of the cells of the obtained population of cardiac stromal cells express CD90, CD73, and CD44; and
ii. Amplifying the number of said cardiac stromal cells by culturing said population of cardiac stromal cells of step (i) in the presence of at least one third extracellular matrix protein in a serum-free basal medium, wherein at least 80 % of the cells of the cardiac stromal cell population maintain the expression of CD90, CD73, and CD44.

2. The method of claim 1, wherein step (ii) stably amplifies the population of cardiac stromal cells as determined by the maintained expression of at least 80% of CD90, CD73, and CD44, preferably at least 81%, more preferably at least 82%, even more preferably at least 83 %, even more preferably at least 84%, even more preferably at least 85%, even more preferably at least 86%, even more preferably at least 87%, even more preferably at least 88%, even more preferably at least 89%, and most preferably at least 90%, as determined by flow cytometry.

3. The method of claim 1 or 2, wherein the method comprises the steps of:
i. Inducing epithelial-mesenchymal transition of epicardial cells obtained by differentiation of pluripotent stem cells, wherein the epicardial cells express Wilms tumor antigen (WT-1), wherein by inducing epithelial-mesenchymal transition the epicardial cells are differentiated into cardiac stromal cells, wherein inducing epithelial-mesenchymal transition comprises
a1) culturing said epicardial cells under suitable conditions in the presence of an first extracellular matrix protein in a serum-free basal medium comprising effective amounts of (a) FGF2, (b) vascular endothelial growth factor (VEGF), (c) glutamine and (d) a GSK-3 inhibitor, wherein said amounts result in the expression of CD90 in at least 50% of the cells obtained by step (i) a1), the expression of CD73 in at most 50% of the cells obtained by step (i) a1), and the expression of CD44 in at most 30% of the cells obtained by step (i) a1); followed by
a2) culturing the cells of step (i) a1) under suitable conditions in the presence of a second extracellular matrix protein in a serum-free basal medium comprising effective amounts of (a) FGF2, (b) VEGF, and (c) glutamine; wherein said amounts result in the expression of CD90, CD73, and CD44 in at least 80 % of the obtained population cardiac stromal cells; and
ii. Amplifying the number of said cardiac stromal cells by culturing said population of cardiac stromal cells of step (i) in the presence of at least one third extracellular matrix protein in a serum-free basal medium comprising effective amounts of (a) FGF2, (b) VEGF, and (c) glutamine, wherein said amounts result in the maintained expression of CD90, CD73, and CD44 in at least 80 % of said cardiac stromal cell population obtained by step (ii).

4. The method any of the preceding claims, wherein the serum-free basal medium in step (i) a1) comprises a final concentration of 10-200 ng/ml FGF2, preferably 15-100 ng/ml, more preferably 20-80 ng/ml, even more preferably 30-70 ng/ml, most preferably 40-60 ng/ml, and most preferably about 50 ng/ml;
wherein serum-free basal medium in step (i) a1) comprises a final concentration of 5-100 ng/ml VEGF, preferably 7-50 ng/ml, more preferably 10-40 ng/ml, even more preferably 15-35 ng/ml, most preferably 20-30 ng/ml, and most preferably about 25 ng/ml VEGF;
wherein serum-free basal medium in step (i) a1) comprises a final concentration of 0.2-20 mM glutamine, preferably, 0.5-10 mM glutamine, more preferably 0.75-5 mM glutamine, more preferably 1-3 mM glutamine, more preferably 1.5-2.5 mM glutamine, even more preferably about 2 mM glutamine, and most preferably wherein the glutamine is present as a L-alanine-L-glutamine dipeptide; and/or
wherein the basal medium in step (i) a1) comprises a final concentration of 0.1-10 µM CHIR99021, preferably 0.2-9 µM, more preferably 0.3-8 µM, even more preferably 0.4-7 µM, still more preferably 0.5-6 µM, more preferably 0.6-5 µM, more preferably 0.7-4 µM, more preferably 0.8-3 µM, most preferably 0.9-2 µM, and even most preferably about 1 µM CHIR99021.

5. The method of any of the preceding claims, wherein the serum-free basal medium in step (i) a2) comprises a final concentration of 10-200 ng/ml FGF2, preferably 15-100 ng/ml, more preferably 20-80 ng/ml, even more preferably 30-70 ng/ml, most preferably 40-60 ng/ml, and most preferably about 50 ng/ml;
wherein the serum-free basal medium in step (i) a2) comprises a final concentration of 5-100 ng/ml VEGF, preferably 7-50 ng/ml, more preferably 10-40 ng/ml, even more preferably 15-35 ng/ml, most preferably 20-30 ng/ml, and most preferably about 25 ng/ml VEGF; and/or
wherein the serum-free basal medium in step (i) a2) comprises a final concentration of 0.2-20 mM glutamine, preferably, 0.5-10 mM glutamine, more preferably 0.75-5 mM glutamine, more preferably 1-3 mM glutamine, more preferably 1.5-2.5 mM glutamine, even more preferably about 2 mM glutamine, and most preferably wherein the glutamine is present as a L-alanine-L-glutamine dipeptide.

6. The method of any of the preceding claims, wherein the serum-free basal medium in step (ii) comprises a final concentration of 10-200 ng/ml FGF2, preferably 15-100 ng/ml, more preferably 20-80 ng/ml, even more preferably 30-70 ng/ml, most preferably 40-60 ng/ml, and most preferably about 50 ng/ml;
wherein the serum-free basal medium in step (ii) comprises a final concentration of 5-100 ng/ml VEGF, preferably 7-50 ng/ml, more preferably 10-40 ng/ml, even more preferably 15-35 ng/ml, most preferably 20-30 ng/ml, and most preferably about 25 ng/ml VEGF; and/or
wherein the serum-free basal medium in step (ii) comprises a final concentration of 0.2-20 mM glutamine, preferably, 0.5-10 mM glutamine, more preferably 0.75-5 mM glutamine, more preferably 1-3 mM glutamine, more preferably 1.5-2.5 mM glutamine, even more preferably about 2 mM glutamine, and most preferably wherein the glutamine is present as a L-alanine-L-glutamine dipeptide.

7. The method of any of the preceding claims, wherein the first extracellular matrix protein comprises laminin, vitronectin, collagen, in particular gelatine, fibronectin, elastin, preferably wherein the first extracellular matrix protein comprises laminin, more preferably wherein the extracellular matrix protein is laminin, most preferably wherein laminin is laminin-521;
wherein the second extracellular matrix protein is different from the first extracellular matrix protein and comprises vitronectin, laminin, collagen, in particular gelatine, fibronectin, and elastin, preferably wherein the second extracellular matrix protein is vitronectin; and/or
wherein the at least one third extracellular matrix protein is selected from vitronectin, laminin, collagen, in particular gelatine, fibronectin, and elastin, preferably wherein the extracellular matrix protein is vitronectin.

8. The method of any of the preceding claims, wherein the epicardial cells express Wilms tumor antigen 1 (WT-1) as determined by fluorescent microscopy;
wherein the epicardial cells express Wilms tumor antigen 1 (WT-1) RNA at least 2-fold more than TBP (TATA-binding protein), preferably 3-fold, more preferably 4-fold, even more preferably at least 5-fold, most preferably at least 6-fold; and at most 15-fold, as determined by qRT-PCR; and/or
wherein the epicardial cells are obtained as described in Schlick (2018), Doctoral Thesis, November 2018, University of Göttingen, Witty et al. Nat Biotechnology 32, 1026-1035 (2014), or any other suitable method for providing epicardial cells.

9. The method of any of the preceding claims, wherein the method comprises the steps of:
i*. Inducing mesodermal differentiation by culturing said pluripotent stem cells under suitable conditions on laminin coated substrate in a serum-free basal medium comprising effective amounts of (a) bone morphogenetic protein 4 (BMP4), (b) Activin A (ActA), (c) a GSK-3 inhibitor, (d) basic fibroblast growth factor (FGF2), (e) glutamine, and (f) a serum-free supplement comprising albumin, transferrin, ethanol amine, selenium or a bioavailable salt thereof, L-carnitine, fatty acid supplement, and triodo-L-thyronine (T3), wherein said amounts result in the expression of CD90 in at least 90% of cells obtained by step (i*), the expression of CD73 in at most 10% of cells obtained by step (i*), and the expression of CD44 in at most 10% of the cells obtained by step (i*), as determined by flow cytometry;
i**. Inducing epicardial differentiation by culturing the cells of step (i*) under suitable conditions on laminin coated substrates in a serum-free basal medium comprising effective amounts of (a) BMP4, (b) retinoic acid (RA), (c) a GSK-3 inhibitor, (d) insulin, (e) glutamine and (f) the serum-free supplement as in (i); whereby the obtained cells express Wilms tumor antigen 1 (WT-1), as determined by fluorescent microscopy
i. Inducing epithelial-mesenchymal transition by a1) culturing the cells of step (i**) under suitable conditions in the presence of an first extracellular matrix protein in a serum-free basal medium comprising effective amounts of (a) FGF2, (b) vascular endothelial growth factor (VEGF), (c) glutamine and (d) a GSK-3 inhibitor, wherein said amounts result in the expression of CD90 in at least 50% of the cells obtained by step (i) a1), the expression of CD73 in at most 50% of the cells obtained by step (i) a1), and the expression of CD44 in at most 30% of the cells obtained by step (i) a1); followed by
a2) culturing the cells of step (i) a1) under suitable conditions in the presence of a second extracellular matrix protein in a serum-free basal medium comprising effective amounts of (a) FGF2, (b) VEGF, and (c) glutamine; wherein said amounts result in the expression of CD90, CD73, and CD44 in at least 80 % of the obtained population of cardiac stromal cells; and
ii. Amplifying the number of said cardiac stromal cells by culturing said population of cardiac stromal cells of step (i) in the presence of at least one third extracellular matrix protein in a serum-free basal medium comprising effective amounts of (a) FGF2, (b) VEGF, and (c) glutamine, wherein said amounts result in the maintained expression of CD90, CD73, and CD44 in at least 80 % of said cardiac stromal cell population.

10. An isolated population of cardiac stromal cells, wherein the cardiac stromal cells have been obtained by differentiation of pluripotent stem cells and wherein at least about 80 % of the cells of the population of cardiac stromal cells express CD90, CD73, and CD44.

11. An engineered organ tissue comprising a population of cardiac stromal cells as defined in claim 10.

12. Use of the population of cardiac stromal cells (cStC) obtained by the method according to any of claims 1-9 or as defined in claim 10, or use of the engineered organ tissue as defined in claim 11 in an *in vitro* model for drug screening, preferably *in vitro* model for drug efficacy screening or in an *in vitro* model for drug toxicity screening.

13. Use of the population of cardiac stromal cells (cStC) obtained by the method according to any of claims 1-9 or as defined in claim 10 in an *in vitro* production of an engineered organ tissue, preferably of a human engineered organ tissue, more preferably wherein the engineered human organ tissue is engineered human myocardium or engineered human connective tissue.

14. The population of cardiac stromal cells (cStC) obtained by the method according to any of claim 1-9 or as defined in claim 10 for use in organ repair, preferably heart repair or soft tissue repair.

15. A serum-free cell culture medium suitable for amplification of cardiac stromal cells comprising (a) a serum-free basal medium, (b) 10-200 ng/ml FGF2, (c) 5-100 ng/ml VEGF, (d) 0.2-20 mM glutamine, and (e) an eukaryotic cell culture medium supplement comprising 6.6-165 µg/ml ascorbic acid, 2-50 µg/ml insulin, 1.1-27.5 µg/ml transferrin, 1660-41500 µg/ml albumin, and 11-145 nM selenium.
